# EUROPEAN PATENT APPLICATION

(11) **EP 2 915 818 A2**
(43) Date of publication of application: **09.09.2015**
(21) Application number: 15162064.8
(22) Date of filing: 28.12.2012
(51) Int. Cl.: C07K 16/24, C07K 16/26, C07K 16/22, C07K 16/28, C07K 16/18, A61K 45/06

(54) **DUAL VARIABLE DOMAIN IMMUNOGLOBULINS AND USES THEREOF**

(30) Priority: 30.12.2011 US 201161581966 P
(62) Divisional of application: 12814110.8
(71) Applicant: AbbVie Inc., North Chicago, IL 60064 (US)
(72) Inventor: Ghayur, Tariq, Holliston, MA 01746 (US); Liu, Junjian, Shrewsbury, MA 01545 (US)
(74) Representative: Finnegan Europe LLP

(57) **Abstract**

Multivalent and multispecific binding proteins, methods of making, and their the diagnosis, prevention, and/or treatment diseases are provided.

## Description

### Related Applications

This application claims benefit of U.S. Provisional Application No. 61/581,966, filed December 30, 2011, the complete disclosure of which is hereby incorporated by reference.

### Field

Multivalent and multispecific binding proteins, methods of making, and their uses in the diagnosis, prevention, and/or treatment diseases are provided.

### Background

Engineered proteins, such as multispecific binding proteins capable of binding two or more antigens, are known in the art. Such multispecific binding proteins can be generated using cell fusion, chemical conjugation, or recombinant DNA techniques. There are a variety of multispecific binding protein structures known in the art and many structures and methods have distinct disadvantages.

Bispecific antibodies have been produced using quadroma technology. However, the presence of mis-paired by-products and significantly reduced production yields with this technology means that sophisticated purification procedures are required. Bispecific antibodies can also be produced by chemical conjugation of two different mAbs. However, this approach does not yield homogeneous preparations.

Other approaches used previously include coupling of two parental antibodies with a hetero-bifunctional crosslinker, production of tandem single-chain Fv molecules, diabodies, bispecific diabodies, single-chain diabodies, and di-diabodies. However, each of these approaches have disadvantages. In addition, a multivalent antibody construct comprising two Fab repeats in the heavy chain of an IgG and capable of binding four antigen molecules has been described (see PCT Publication No. WO 0177342 and Miller et al. (2003) J. Immunol. 170(9): 4854-61).

US Patent No. 7,612,181 provides a novel family of binding proteins capable of binding two or more antigens with high affinity, which are called dual variable domain binding proteins (DVD binding protein) or dual variable domain immunoglobulins (DVD-Ig™).

While a variety of structures are provided in the art, some with advantages and disadvantages, specific constructs are required for preparing multivalent binding proteins with specific properties and which bind to specific targets. Additionally, new variable domain sequences can further improve the properties of the binding proteins. Specifically, with certain prior art DVD constructs, some amount of steric hinderance has affected the binding of targets to those prior art DVD constructs, especially to the inner domain (C-terminal domain).

There is a need in the art for improved multivalent binding proteins with reduced steric hinderance, improved affinity, and improved potency, especially for the inner domain (C-terminal domain). Novel binding proteins that have reduced steric hinderance are provided herein.

### Summary

In one embodiment, binding proteins with a VH/VL crossover are provided comprising a polypeptide chain that binds TNF and one of PGE2, SOST, NGF, and LPA, wherein the polypeptide chain comprises VD1-(X1)n-VD2-C-(X2)n, wherein VD1 is a first variable domain, VD2 is a second variable domain, C is a constant domain, X1 represents an amino acid or polypeptide, X2 represents an Fc region and n is 0 or 1, are provided. In an embodiment, the VD1 is a light chain variable domain and VD2 is a heavy chain variable domain. In another embodiment, VD1 and VD2 are capable of binding the same antigen. In another embodiment, VD1 and VD2 are capable of binding different antigens. In still another embodiment, C is a heavy chain constant domain. For example, X1 is a linker with the proviso that X1 is not CH1.

In one embodiment, binding proteins with a VH/VL crossover are provided comprising a heavy chain polypeptide chain that binds TNF and one of PGE2, SOST, NGF, and LPA, wherein the polypeptide chain comprises VD1-(X1)n-VD2-C-(X2)n, wherein VD1 is a light chain variable domain, VD2 is a heavy chain variable domain, C is a heavy chain constant domain, X1 is a linker, and X2 is an Fc region. In an embodiment, X1 is a linker with the proviso that it is not CH1.

In one embodiment, binding proteins with a VH/VL crossover are provided comprising a light chain polypeptide chain that binds TNF and one of PGE2, SOST, NGF, and LPA, wherein the polypeptide chain comprises VD1-(X1)n-VD2-C-(X2)n, wherein VD1 is a heavy chain variable domain, VD2 is a light chain variable domain, C is a light chain constant domain, X1 is a linker, and X2 does not comprise an Fc region. In an embodiment, X1 is a linker with the proviso that it is not CL.

In another embodiment, a binding protein a VH/VL crossover that binds TNF and one of PGE2, SOST, NGF, and LPA, wherein the first polypeptide chain is a heavy chain and comprises VD1-(X1)n-VD2-C-(X2)n, wherein VD1 is a light chain variable domain, VD2 is a heavy chain variable domain, C is a heavy chain constant domain, X1 is a first linker, and X2 is an Fc region; and the second polypeptide chain is a light chain and comprises VD1-(X1)n-VD2-C-(X2)n, wherein VD1 is a heavy chain variable domain, VD2 is a light chain variable domain, C is a light chain constant domain, X1 is a second linker, and X2 does not comprise an Fc region is provided. In some embodiments, the first and second X1 are the same. In other embodiments, the first and second X1 are different. In some embodiments the first X1 is not a CH1 domain and/or the second X1 is not a CL domain. In one embodiment, the first X1 and the second X1 are short (e.g., 6 amino acid) linkers. In another embodiment, the first X1 and the second X1 are long (e.g., greater than 6 amino acid) linkers. In another embodiment, the first X1 is a short linker and the second X1 is a long linker. In another embodiment, the first X1 is a long linker and the second X1 is a short linker.

In an embodiment, the Dual Variable Domain (DVD) binding protein with a VH/VL crossover comprises four polypeptide chains, wherein each of the first two polypeptide chains is a heavy chain and comprises VD1-(X1)n-VD2-C-(X2)n, wherein VD1 is a light chain variable domain, VD2 is a heavy chain variable domain, C is a heavy chain constant domain, X1 is a first linker, and X2 is an Fc region; and each of the second two polypeptide chain is a light chain and comprises VD1-(X1)n-VD2-C-(X2)n, wherein VD1 is a heavy chain variable domain, VD2 is a light chain variable domain, C is a light chain constant domain, X1 is a second linker, and X2 does not comprise an Fc region. Such a DVD binding protein has four antigen binding sites. In some embodiments, the first and second X1 are the same. In other embodiments, the first and second X1 are different. In some embodiments, the first X1 is not a CH1 domain and/or the second X1 is not a CL domain. In another embodiment, the binding proteins disclosed herein are capable of binding TNF and one of PGE2, SOST, NGF, and LPA. Accordingly, in some embodiments, the binding proteins comprise at least two variable domain sequences (e.g., VD1 and VD2) capable of binding TNF and one of PGE2, SOST, NGF, and LPA. In an embodiment, wherein the binding protein is a heavy chain polypeptide chain,
(a) the binding protein binds TNF and one of PGE2, SOST, NGF, and LPA;
(b) VD1 comprises three CDRs from SEQ ID NO: 31, 33, 35, 37, 39, 41, 43, 45, 47 or 49; and
(c) VD2 comprises three CDRs from SEQ ID NO: 30, 32, 34, 36, 38, 40, 42, 44, 46, or 48.

In an embodiment,
(a) VD1 comprises SEQ ID NO: 31, 33, 35, 37, 39, 41, 43, 45, 47 or 49; and
(b) VD2 comprises SEQ ID NO: 30, 32, 34, 36, 38, 40, 42, 44, 46, or 48.

In another embodiment, wherein the binding protein is a light chain polypeptide chain,
(a) the binding protein binds TNF and one of PGE2, SOST, NGF, and LPA;
(b) VD1 comprises three CDRs from SEQ ID NO: 30, 32, 34, 36, 38, 40, 42, 44, 46, or 48; and
(c) VD2 comprises three CDRs from SEQ ID NO: 31, 33, 35, 37, 39, 41, 43, 45, 47 or 49.

In another embodiment,
(a) VD1 comprises SEQ ID NO: 30, 32, 34, 36, 38, 40, 42, 44, 46, or 48; and
(b) VD2 comprises SEQ ID NO: 31, 33, 35, 37, 39, 41, 43, 45, 47 or 49.

In an embodiment, the binding protein comprises first and second polypeptide chains, wherein the first polypeptide chain is a heavy chain and the second polypeptide chain is a light chain,
(a) the binding protein binds TNF and one of PGE2, SOST, NGF, and LPA;
(b) VD1 of the heavy chain is a light chain variable region and comprises three CDRs from SEQ ID NO: 31, 33, 35, 37, 39, 41, 43, 45, 47 or 49; and
(c) VD2 of the heavy chain is a heavy chain variable region and comprises three CDRs from SEQ ID NO: 30, 32, 34, 36, 38, 40, 42, 44, 46, or 48
(d) VD1 of the light chain is a heavy chain variable region and comprises three CDRs from SEQ ID NO: 30, 32, 34, 36, 38, 40, 42, 44, 46, or 48; and
(e) VD2 of the light chain is a light chain variable region and comprises three CDRs from SEQ ID NO: 31, 33, 35, 37, 39, 41, 43, 45, 47 or 49.

In another embodiment,
(a) VD1 of the heavy chain is a light chain variable region and comprises SEQ I D NO: 31, 33, 35, 37, 39, 41, 43, 45, 47 or 49; and
(b) VD2 of the heavy chain is a heavy chain variable region and comprises SEQ ID NO: 30, 32, 34, 36, 38, 40, 42, 44, 46, or 48
(c) VD1 of the light chain is a heavy chain variable region and comprises SEQ ID NO: 30, 32, 34, 36, 38, 40, 42, 44, 46, or 48; and
(d) VD2 of the light chain is a light chain variable region and comprises SEQ ID NO: 31, 33, 35, 37, 39, 41, 43, 45, 47 or 49.

In another embodiment, the binding protein comprises two of said heavy chains and two of said light chains.

In another embodiment, the binding protein comprises a heavy chain and a light chain sequence as shown in the Table 1 herein

A further embodiment, of any of the heavy chain, light chain, two chain, or four chain embodiments, includes at least one X1 linker comprising AKTTPKLEEGEFSEAR (SEQ ID NO: 1); AKTTPKLEEGEFSEARV (SEQ ID NO: 2); AKTTPKLGG (SEQ ID NO: 3); SAKTTPKLGG (SEQ ID NO: 4); SAKTTP (SEQ ID NO: 5); RADAAP (SEQ ID NO: 6); RADAAPTVS (SEQ ID NO: 7); RADAAAAGGPGS (SEQ ID NO: 8); RADAAAA(G₄S)₄ (SEQ ID NO: 9); SAKTTPKLEEGEFSEARV (SEQ ID NO: 10); ADAAP (SEQ ID NO: 11); ADAAPTVSIFPP (SEQ ID NO: 12); TVAAP (SEQ ID NO: 13); TVAAPSVFIFPP (SEQ ID NO: 14); QPKAAP (SEQ ID NO: 15); QPKAAPSVTLFPP (SEQ ID NO: 16); AKTTPP (SEQ ID NO: 17); AKTTPPSVTPLAP (SEQ ID NO: 18); AKTTAP (SEQ ID NO: 19); AKTTAPSVYPLAP (SEQ ID NO: 20); ASTKGP (SEQ ID NO: 21); ASTKGPSVFPLAP (SEQ ID NO: 22), GGGGSGGGGSGGGGS (SEQ ID NO: 23); GENKVEYAPALMALS (SEQ ID NO: 24); GPAKELTPLKEAKVS (SEQ ID NO: 25); or GHEAAAVMQVQYPAS (SEQ ID NO: 26); TVAAPSVFIFPPTVAAPSVFIFPP (SEQ ID NO: 27); ASTKGPSVFPLAPASTKGPSVFPLAP (SEQ ID NO: 28); or G/S based sequences (e.g., G4S (SEQ ID NO: 29) and G4S repeats). In an embodiment, X2 is an Fc region. In another embodiment, X2 is a variant Fc region.

In still another embodiment, the Fc region, if present in the first polypeptide, is a native sequence Fc region or a variant sequence Fc region. In yet another embodiment, the Fc region is an Fc region from an IgG1, an Fc region from an IgG2, an Fc region from an IgG3, an Fc region from an IgG4, an Fc region from an IgA, an Fc region from an IgM, an Fc region from an IgE, or an Fc region from an IgD.

A method of making a binding protein that binds TNF and one of PGE2, SOST, NGF, and LPA is provided. In an embodiment, the method of making a binding protein that binds TNF and one of PGE2, SOST, NGF, and LPA comprises the steps of a) obtaining a first parent antibody, or antigen binding portion thereof, that binds TNF; b) obtaining a second parent antibody, or antigen binding portion thereof, that binds one of PGE2, SOST, NGF, and LPA; c) preparing construct(s) encoding any of the binding proteins described herein; and d) expressing the polypeptide chains, such that a binding protein that binds the first and the second antigen is generated.

In any of the embodiments herein, the VD1 heavy chain variable domain, if present, and light chain variable domain, if present, can be from a first parent antibody or antigen binding portion thereof; the VD2 heavy chain variable domain, if present, and light chain variable domain, if present, can be from a second parent antibody or antigen binding portion thereof. The first and second parent antibodies can be the same or different.

In one embodiment, the first parent antibody or antigen binding portion thereof, binds a first antigen, and the second parent antibody or antigen binding portion thereof, binds a second antigen. In an embodiment, the first and second antigens are the same antigen. In another embodiment, the parent antibodies bind different epitopes on the same antigen. In another embodiment, the first and second antigens are different antigens. In another embodiment, the first parent antibody or antigen binding portion thereof, binds the first antigen with a potency different from the potency with which the second parent antibody or antigen binding portion thereof, binds the second antigen. In yet another embodiment, the first parent antibody or antigen binding portion thereof, binds the first antigen with an affinity different from the affinity with which the second parent antibody or antigen binding portion thereof, binds the second antigen.

In another embodiment, the first parent antibody or antigen binding portion thereof, and the second parent antibody or antigen binding portion thereof, are a human antibody, CDR grafted antibody, humanized antibody, and/or affinity matured antibody.

In another embodiment, the binding protein possesses at least one desired property exhibited by the first parent antibody or antigen binding portion thereof, or the second parent antibody or antigen binding portion thereof. Alternatively, the first parent antibody or antigen binding portion thereof and the second parent antibody or antigen binding portion thereof possess at least one desired property exhibited by the binding protein. In an embodiment, the desired property is one or more antibody parameters. In another embodiment, the antibody parameters are antigen specificity, affinity to antigen, potency, biological function, epitope recognition, stability, solubility, production efficiency, immunogenicity, pharmacokinetics, bioavailability, tissue cross reactivity, or orthologous antigen binding. In an embodiment, the binding protein is multivalent. In another embodiment, the binding protein is multispecific. The multivalent and or multispecific binding proteins described herein have desirable properties particularly from a therapeutic standpoint. For instance, the multivalent and or multispecific binding protein may (1) be internalized (and/or catabolized) faster than a bivalent antibody by a cell expressing an antigen to which the antibodies bind; (2) be an agonist binding protein; and/or (3) induce cell death and/or apoptosis of a cell expressing an antigen to which the multivalent binding protein is capable of binding. The "parent antibody", which provides at least one antigen binding specificity of the multivalent and or multispecific binding protein, may be one that is internalized (and/or catabolized) by a cell expressing an antigen to which the antibody binds; and/or may be an agonist, cell death-inducing, and/or apoptosis-inducing antibody, and the multivalent and or multispecific binding protein as described herein may display improvement(s) in one or more of these properties. Moreover, the parent antibody may lack any one or more of these properties, but may acquire one or more of them when constructed as a multivalent binding protein as described herein.

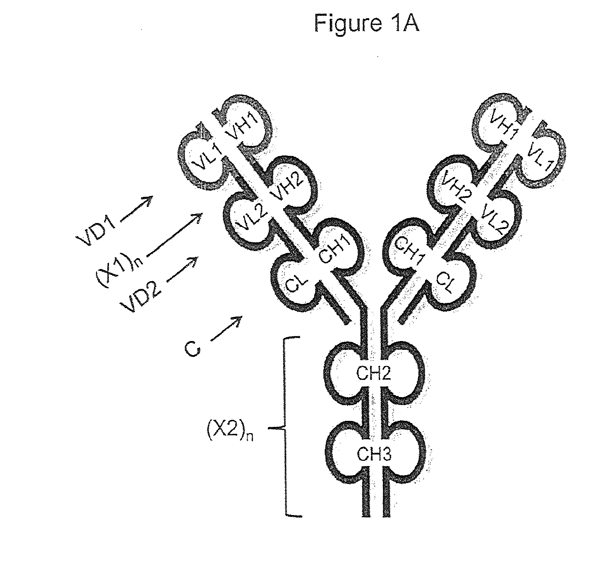

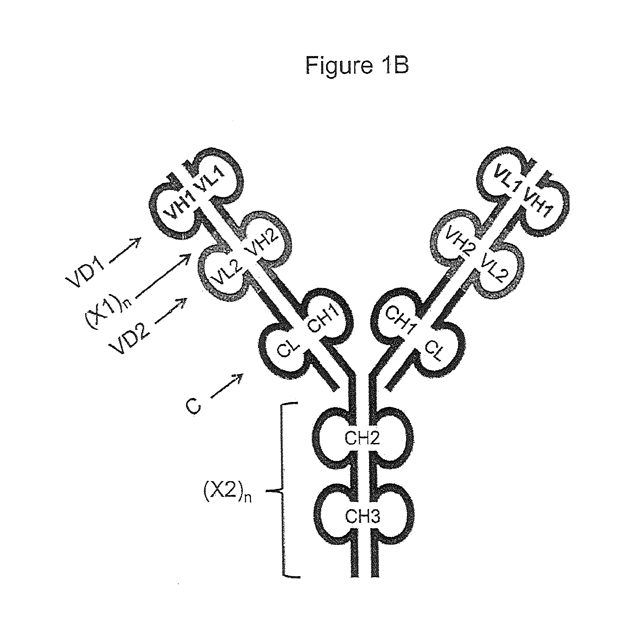

In one embodiment, binding proteins with a VH/VL crossover are provided, wherein the crossover improves at least one desired property exhibited by the binding protein compared to a binding protein comprising the same variable domains without a crossover (i.e., in the orientation shown in Figure 1A.) An embodiment of the invention includes a method for improving at least one desired property exhibited by a binding protein comprising moving a VH sequence for VD1 to the light chain and/or moving a VL sequence for VD1 to the heavy chain (i.e., changing the orientation of the binding protein from that shown in Figure 1A to that shown in Figure 1 B).

In another embodiment, the binding protein has an on rate constant (Kₒₙ) to one or more targets of at least about 10²M⁻¹s⁻¹ ; at least about 10³M⁻¹s⁻¹; at least about 10⁴M⁻¹s⁻¹ ; at least about 10⁵M⁻¹s⁻¹; or at least about 10⁶M⁻¹s⁻¹, as measured by surface plasmon resonance. In an embodiment, the binding protein has an on rate constant (Kₒₙ) to one or more targets from about 10²M⁻¹s⁻¹ to about 10³M⁻¹s⁻¹; from about 10³M⁻¹s⁻¹ to about 10⁴M⁻¹s⁻¹. from about 10⁴M⁻¹s⁻¹ to about 10⁵M⁻¹s⁻¹; or from about 10⁵M⁻¹s⁻¹ to about 10⁶M⁻¹s⁻¹, as measured by surface plasmon resonance.

In another embodiment, the binding protein has an off rate constant (K_{off}) for one or more targets of at most about 10⁻³s⁻¹ ; at most about 10⁻⁴s⁻¹ ; at most about 10⁻⁵s⁻¹; or at most about 10⁻⁶s⁻¹, as measured by surface plasmon resonance. In an embodiment, the binding protein has an off rate constant (K_{off}) to one or more targets of about 10⁻³s⁻¹ to about 10⁻⁴s⁻¹; of about 10⁻⁴s⁻¹ to about 10⁻⁵s⁻¹; or of about 10⁻⁵s⁻¹ to about 10⁻⁶s⁻¹, as measured by surface plasmon resonance.

In another embodiment, the binding protein has a dissociation constant (K_{d}) to one or more targets of at most about 10⁻⁷M; at most about 10⁻⁸M; at most about 10⁻⁹M; at most about 10⁻¹⁰M; at most about 10⁻¹¹M; at most about 10⁻¹²M; or at most 10⁻¹³M. In an embodiment, the binding protein has a dissociation constant (K_{d}) to its targets of about 10⁻⁷M to about 10⁻⁸M; of about 10⁻⁸M to about 10⁻⁹M; of about 10⁻⁹M to about 10⁻¹⁰M; of about 10⁻¹⁰M to about 10⁻¹¹M; of about 10⁻¹¹M to about 10⁻¹²M; or of about 10⁻¹² to M about 10⁻¹³M.

In another embodiment, the binding protein is a conjugate further comprising an agent. In an embodiment, the agent is an immunoadhesion molecule, an imaging agent, a therapeutic agent, or a cytotoxic agent. In an embodiment, the imaging agent is a radiolabel, an enzyme, a fluorescent label, a luminescent label, a bioluminescent label, a magnetic label, or biotin. In another embodiment, the radiolabel is ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm. In yet another embodiment, the therapeutic or cytotoxic agent is an anti-metabolite, an alkylating agent, an antibiotic, a growth factor, a cytokine, an anti-angiogenic agent, an anti-mitotic agent, an anthracycline, toxin, or an apoptotic agent.

In another embodiment, the binding protein is a crystallized binding protein and exists as a crystal. In an embodiment, the crystal is a carrier-free pharmaceutical controlled release crystal. In another embodiment, the crystallized binding protein has a greater half life in vivo than the soluble counterpart of the binding protein. In yet another embodiment, the crystallized binding protein retains biological activity.

In another embodiment, the binding protein described herein is glycosylated. For example, the glycosylation pattern is a human glycosylation pattern.

An isolated nucleic acid encoding any one of the binding proteins disclosed herein is also provided. A further embodiment provides a vector comprising the isolated nucleic acid disclosed herein wherein the vector is pcDNA; pTT (Durocher et al. (2002) Nucleic Acids Res. 30(2); pTT3 (pTT with additional multiple cloning site; pEFBOS (Mizushima and Nagata (1990) Nucleic Acids Res. 18(17); pBV; pJV; pcDNA3.1 TOPO; pEF6 TOPO; pBOS; pHybE; or pBJ. In an embodiment, the vector is a vector disclosed in US Patent Publication No. 20090239259.

In another aspect, a host cell is transformed with the vector disclosed herein. In an embodiment, the host cell is a prokaryotic cell, for example, E.coli. In another embodiment, the host cell is a eukaryotic cell, for example, a protist cell, an animal cell, a plant cell, or a fungal cell. In an embodiment, the host cell is a mammalian cell including, but not limited to, CHO, COS, NS0, SP2, PER.C6, or a fungal cell, such as *Saccharomyces cerevisiae,* or an insect cell, such as Sf9. In an embodiment, two or more binding proteins, e.g., with different specificities, are produced in a single recombinant host cell. For example, the expression of a mixture of antibodies has been called Oligoclonics™ (Merus B.V., The Netherlands) US Patent Nos. 7,262,028 and 7,429,486.

A method of producing a binding protein disclosed herein comprising culturing any one of the host cells disclosed herein in a culture medium under conditions sufficient to produce the binding protein is provided. In an embodiment, 50%-75% of the binding protein produced by this method is a dual specific tetravalent binding protein. In another embodiment, 75%-90% of the binding protein produced by this method is a dual specific tetravalent binding protein. In another embodiment, 90%-95% of the binding protein produced is a dual specific tetravalent binding protein.

One embodiment provides a composition for the release of a binding protein wherein the composition comprises a crystallized binding protein, an ingredient, and at least one polymeric carrier. In an embodiment, the polymeric carrier is poly (acrylic acid), a poly (cyanoacrylate), a poly (amino acid), a poly (anhydride), a poly (depsipeptide), a poly (ester), poly (lactic acid), poly (lactic-co-glycolic acid) or PLGA, poly (b-hydroxybutryate), poly (caprolactone), poly (dioxanone), poly (ethylene glycol), poly ((hydroxypropyl) methacrylamide, poly [(organo)phosphazene], a poly (ortho ester), poly (vinyl alcohol), poly (vinylpyrrolidone), a maleic anhydride- alkyl vinyl ether copolymer, a pluronic polyol, albumin, alginate, cellulose, a cellulose derivative, collagen, fibrin, gelatin, hyaluronic acid, an oligosaccharide, a glycaminoglycan, a sulfated polysaccharide, or blends and copolymers thereof. In an embodiment, the ingredient is albumin, sucrose, trehalose, lactitol, gelatin, hydroxypropyl-β- cyclodextrin, methoxypolyethylene glycol, or polyethylene glycol.

Another embodiment provides a method for treating a mammal comprising the step of administering to the mammal an effective amount of a composition disclosed herein.

A pharmaceutical composition comprising a binding protein disclosed herein and a pharmaceutically acceptable carrier is provided. In a further embodiment, the pharmaceutical composition comprises at least one additional therapeutic agent for treating a disorder. For example, the additional agent may be a therapeutic agent, an imaging agent, a cytotoxic agent, an angiogenesis inhibitor (including but not limited to an anti-VEGF antibody or a VEGF-trap), a kinase inhibitor (including but not limited to a KDR and a TIE-2 inhibitor), a co-stimulation molecule blocker (including but not limited to anti-B7.1, anti-B7.2, CTLA4-Ig, anti-CD20), an adhesion molecule blocker (including but not limited to an anti-LFA-1 antibody, an anti-E/L selectin antibody, a small molecule inhibitor), an anti-cytokine antibody or functional fragment thereof (including but not limited to an anti-IL-18, an anti-TNF, and an anti-IL-6/cytokine receptor antibody), methotrexate, cyclosporin, rapamycin, FK506, a detectable label or reporter, a TNF antagonist, an antirheumatic, a muscle relaxant, a narcotic, a non-steroid anti-inflammatory drug (NSAID), an analgesic, an anesthetic, a sedative, a local anesthetic, a neuromuscular blocker, an antimicrobial, an antipsoriatic, a corticosteriod, an anabolic steroid, an erythropoietin, an immunization, an immunoglobulin, an immunosuppressive, a growth hormone, a hormone replacement drug, a radiopharmaceutical, an antidepressant, an antipsychotic, a stimulant, an asthma medication, a beta agonist, an inhaled steroid, an epinephrine or analog, a cytokine, or a cytokine antagonist.

A method for treating a human subject suffering from a disorder in which the target, or targets, capable of being bound by the binding protein disclosed herein is detrimental, comprising administering to the human subject a binding protein disclosed herein such that the activity of the target, or targets, in the human subject is inhibited and one or more symptoms is alleviated or treatment is achieved is provided.

TNF-α plays a role in the pathology associated with a variety of diseases involving immune and inflammatory elements, such as autoimmune diseases, particularly those assocated with inflammation, including Crohn's disease, psoriasis (including plaque psoriasis), arthritis (including rheumatoid arthritis, psoratic arthritis, osteoarthritis, or juvenile idiopathic arthritis), multiple sclerosis, systemic lupus erythematosus, and ankylosing spondylitis. Therefore, the binding proteins herein may be used to treat these disorders. In another embodiment, the disorder is a respiratory disorder; asthma; allergic and nonallergic asthma; asthma due to infection; asthma due to infection with respiratory syncytial virus (RSV); chronic obstructive pulmonary disease (COPD); a condition involving airway inflammation; eosinophilia; fibrosis and excess mucus production; cystic fibrosis; pulmonary fibrosis; an atopic disorder; atopic dermatitis; urticaria; eczema; allergic rhinitis; allergic enterogastritis; an inflammatory and/or autoimmune condition of the skin; an inflammatory and/or autoimmune condition of gastrointestinal organs; inflammatory bowel diseases (IBD); ulcerative colitis; an inflammatory and/or autoimmune condition of the liver; liver cirrhosis; liver fibrosis; liver fibrosis caused by hepatitis B and/or C virus; scleroderma; tumors or cancers; hepatocellular carcinoma; glioblastoma; lymphoma; Hodgkin's lymphoma; a viral infection; a bacterial infection; a parasitic infection; HTLV-1 infection; suppression of expression of protective type 1 immune responses, suppression of expression of a protective type 1 immune response during vaccination, neurodegenerative diseases, neuronal regeneration, and spinal cord injury.

Another embodiment provides for the use of the binding protein in the treatment of a disease or disorder, wherein said disease or disorder is rheumatoid arthritis, osteoarthritis, juvenile chronic arthritis, septic arthritis, Lyme arthritis, psoriatic arthritis, reactive arthritis, spondyloarthropathy, systemic lupus erythematosus, Crohn's disease, ulcerative colitis, inflammatory bowel disease, insulin dependent diabetes mellitus, thyroiditis, asthma, allergic diseases, psoriasis, dermatitis scleroderma, graft versus host disease, organ transplant rejection, acute or chronic immune disease associated with organ transplantation, sarcoidosis, atherosclerosis, disseminated intravascular coagulation, Kawasaki's disease, Grave's disease, nephrotic syndrome, chronic fatigue syndrome, Wegener's granulomatosis, Henoch-Schoenlein purpurea, microscopic vasculitis of the kidneys, chronic active hepatitis, uveitis, septic shock, toxic shock syndrome, sepsis syndrome, cachexia, infectious diseases, parasitic diseases, acquired immunodeficiency syndrome, acute transverse myelitis, Huntington's chorea, Parkinson's disease, Alzheimer's disease, stroke, primary biliary cirrhosis, hemolytic anemia, malignancies, heart failure, Addison's disease, sporadic, polyglandular deficiency type I and polyglandular deficiency type II, Schmidt's syndrome, adult (acute) respiratory distress syndrome, alopecia, alopecia areata, arthropathy, Reiter's disease, psoriatic arthropathy, ulcerative colitic arthropathy, enteropathic synovitis, chlamydia, yersinia and salmonella associated arthropathy, atheromatous disease/arteriosclerosis, atopic allergy, autoimmune bullous disease, pemphigus vulgaris, pemphigus foliaceus, pemphigoid, linear IgA disease, autoimmune haemolytic anaemia, Coombs positive haemolytic anaemia, acquired pernicious anaemia, juvenile pernicious anaemia, myalgic encephalitis/Royal Free Disease, chronic mucocutaneous candidiasis, giant cell arteritis, primary sclerosing hepatitis, cryptogenic autoimmune hepatitis, acquired immunodeficiency related diseases, hepatitis B, hepatitis C, common varied immunodeficiency (common variable hypogammaglobulinaemia), dilated cardiomyopathy, female infertility, ovarian failure, premature ovarian failure, fibrotic lung disease, cryptogenic fibrosing alveolitis, post-inflammatory interstitial lung disease, interstitial pneumonitis, connective tissue disease associated interstitial lung disease, mixed connective tissue disease associated lung disease, systemic sclerosis associated interstitial lung disease, rheumatoid arthritis associated interstitial lung disease, systemic lupus erythematosus associated lung disease, dermatomyositis/polymyositis associated lung disease, Sjögren's disease associated lung disease, ankylosing spondylitis associated lung disease, vasculitic diffuse lung disease, haemosiderosis associated lung disease, drug-induced interstitial lung disease, fibrosis, radiation fibrosis, bronchiolitis obliterans, chronic eosinophilic pneumonia, lymphocytic infiltrative lung disease, postinfectious interstitial lung disease, gouty arthritis, autoimmune hepatitis, type-1 autoimmune hepatitis (classical autoimmune or lupoid hepatitis), type-2 autoimmune hepatitis (anti-LKM antibody hepatitis), autoimmune mediated hypoglycaemia, type B insulin resistance with acanthosis nigricans, hypoparathyroidism, acute immune disease associated with organ transplantation, chronic immune disease associated with organ transplantation, osteoarthrosis, primary sclerosing cholangitis, psoriasis type 1, psoriasis type 2, idiopathic leucopaenia, autoimmune neutropaenia, renal disease NOS, glomerulonephritides, microscopic vasulitis of the kidneys, lyme disease, discoid lupus erythematosus, male infertility idiopathic or NOS, sperm autoimmunity, multiple sclerosis (all subtypes), sympathetic ophthalmia, pulmonary hypertension secondary to connective tissue disease, Goodpasture's syndrome, pulmonary manifestation of polyarteritis nodosa, acute rheumatic fever, rheumatoid spondylitis, Still's disease, systemic sclerosis, Sjörgren's syndrome, Takayasu's disease/arteritis, autoimmune thrombocytopaenia, idiopathic thrombocytopaenia, autoimmune thyroid disease, hyperthyroidism, goitrous autoimmune hypothyroidism (Hashimoto's disease), atrophic autoimmune hypothyroidism, primary myxoedema, phacogenic uveitis, primary vasculitis, vitiligo acute liver disease, chronic liver diseases, alcoholic cirrhosis, alcohol-induced liver injury, choleosatatis, idiosyncratic liver disease, drug-induced hepatitis, non-alcoholic steatohepatitis, allergy and asthma, group B streptococci (GBS) infection, mental disorders, depression, schizophrenia, Th2 Type and Th1 Type mediated diseases, acute and chronic pain, different forms of pain, cancers, lung cancer, breast cancer, stomach cancer, bladder cancer, colon cancer, pancreatic cancer, ovarian cancer, prostate cancer, rectal cancer, hematopoietic malignancies, leukemia, lymphoma, Abetalipoprotemia, acrocyanosis, acute and chronic parasitic or infectious processes, acute leukemia, acute lymphoblastic leukemia (ALL), acute myeloid leukemia (AML), acute or chronic bacterial infection, acute pancreatitis, acute renal failure, adenocarcinomas, aerial ectopic beats, AIDS dementia complex, alcohol-induced hepatitis, allergic conjunctivitis, allergic contact dermatitis, allergic rhinitis, allograft rejection, alpha-I-antitrypsin deficiency, amyotrophic lateral sclerosis, anemia, angina pectoris, anterior horn cell degeneration, anti cd3 therapy, antiphospholipid syndrome, anti-receptor hypersensitivity reactions, aortic and peripheral aneuryisms, aortic dissection, arterial hypertension, arteriosclerosis, arteriovenous fistula, ataxia, atrial fibrillation (sustained or paroxysmal), atrial flutter, atrioventricular block, B cell lymphoma, bone graft rejection, bone marrow transplant (BMT) rejection, bundle branch block, Burkitt's lymphoma, burns, cardiac arrhythmias, cardiac stun syndrome, cardiac tumors, cardiomyopathy, cardiopulmonary bypass inflammation response, cartilage transplant rejection, cerebellar cortical degenerations, cerebellar disorders, chaotic or multifocal atrial tachycardia, chemotherapy associated disorders, chronic myelocytic leukemia (CML), chronic alcoholism, chronic inflammatory pathologies, chronic lymphocytic leukemia (CLL), chronic obstructive pulmonary disease (COPD), chronic salicylate intoxication, colorectal carcinoma, congestive heart failure, conjunctivitis, contact dermatitis, cor pulmonale, coronary artery disease, Creutzfeldt-Jakob disease, culture negative sepsis, cystic fibrosis, cytokine therapy associated disorders, dementia pugilistica, demyelinating diseases, dengue hemorrhagic fever, dermatitis, dermatologic conditions, diabetes, diabetes mellitus, diabetic ateriosclerotic disease, diffuse Lewy body disease, dilated congestive cardiomyopathy, disorders of the basal ganglia, Down's syndrome in middle age, drug-induced movement disorders induced by drugs which block CNS dopamine receptors, drug sensitivity, eczema, encephalomyelitis, endocarditis, endocrinopathy, epiglottitis, epstein-barr virus infection, erythromelalgia, extrapyramidal and cerebellar disorders, familial hematophagocytic lymphohistiocytosis, fetal thymus implant rejection, Friedreich's ataxia, functional peripheral arterial disorders, fungal sepsis, gas gangrene, gastric ulcer, glomerular nephritis, graft rejection of any organ or tissue, gram negative sepsis, gram positive sepsis, granulomas due to intracellular organisms, hairy cell leukemia, Hallervorden-Spatz disease, Hashimoto's thyroiditis, hay fever, heart transplant rejection, hemachromatosis, hemodialysis, hemolytic uremic syndrome/thrombolytic thrombocytopenic purpura, hemorrhage, hepatitis A, His bundle arrythmias, HIV infection/HIV neuropathy, Hodgkin's disease, hyperkinetic movement disorders, hypersensitity reactions, hypersensitivity pneumonitis, hypertension, hypokinetic movement disorders, hypothalamic-pituitary-adrenal axis evaluation, idiopathic Addison's disease, idiopathic pulmonary fibrosis, antibody mediated cytotoxicity, Asthenia, infantile spinal muscular atrophy, inflammation of the aorta, influenza a, ionizing radiation exposure, iridocyclitis/uveitis/optic neuritis, ischemia- reperfusion injury, ischemic stroke, juvenile rheumatoid arthritis, juvenile spinal muscular atrophy, Kaposi's sarcoma, kidney transplant rejection, legionella, leishmaniasis, leprosy, lesions of the corticospinal system, lipedema, liver transplant rejection, lymphederma, malaria, malignamt lymphoma, malignant histiocytosis, malignant melanoma, meningitis, meningococcemia, metabolic/idiopathic, migraine headache, mitochondrial multi.system disorder, mixed connective tissue disease, monoclonal gammopathy, multiple myeloma, multiple systems degenerations (Mencel Dejerine-Thomas Shi-Drager and Machado-Joseph), mycobacterium avium intracellulare, mycobacterium tuberculosis, myelodyplastic syndrome, myocardial infarction, myocardial ischemic disorders, nasopharyngeal carcinoma, neonatal chronic lung disease, nephritis, nephrosis, neurodegenerative diseases, neurogenic muscular atrophies, neutropenic fever, non-hodgkins lymphoma, occlusion of the abdominal aorta and its branches, occulsive arterial disorders, okt3 therapy, orchitis/epidydimitis, orchitis/vasectomy reversal procedures, organomegaly, osteoporosis, pancreas transplant rejection, pancreatic carcinoma, paraneoplastic syndrome/hypercalcemia of malignancy, parathyroid transplant rejection, pelvic inflammatory disease, perennial rhinitis, pericardial disease, peripheral atherlosclerotic disease, peripheral vascular disorders, peritonitis, pernicious anemia, pneumocystis carinii pneumonia, pneumonia, POEMS syndrome (polyneuropathy, organomegaly, endocrinopathy, monoclonal gammopathy, and skin changes syndrome), post perfusion syndrome, post pump syndrome, post-MI cardiotomy syndrome, preeclampsia, progressive supranucleo palsy, primary pulmonary hypertension, radiation therapy, Raynaud's phenomenon and disease, Raynoud's disease, Refsum's disease, regular narrow QRS tachycardia, renovascular hypertension, reperfusion injury, restrictive cardiomyopathy, sarcomas, scleroderma, senile chorea, senile dementia of Lewy body type, seronegative arthropathies, shock, sickle cell anemia, skin allograft rejection, skin changes syndrome, small bowel transplant rejection, solid tumors, specific arrythmias, spinal ataxia, spinocerebellar degenerations, streptococcal myositis, structural lesions of the cerebellum, subacute sclerosing panencephalitis, syncope, syphilis of the cardiovascular system, systemic anaphalaxis, systemic inflammatory response syndrome, systemic onset juvenile rheumatoid arthritis, T-cell or FAB ALL telangiectasia, thromboangitis obliterans, thrombocytopenia, toxicity, transplants, trauma/hemorrhage, type III hypersensitivity reactions, type IV hypersensitivity, unstable angina, uremia, urosepsis, valvular heart diseases, varicose veins, vasculitis, venous diseases, venous thrombosis, ventricular fibrillation, viral and fungal infections, vital encephalitis/aseptic meningitis, vital-associated hemaphagocytic syndrome, Wernicke-Korsakoff syndrome, Wilson's disease, xenograft rejection of any organ or tissue, acute coronary syndromes, acute idiopathic polyneuritis, acute inflammatory demyelinating polyradiculoneuropathy, acute ischemia, adult Still's disease, anaphylaxis, anti-phospholipid antibody syndrome, aplastic anemia, atopic eczema, atopic dermatitis, autoimmune dermatitis, autoimmune disorder associated with streptococcus infection, autoimmune enteropathy, autoimmune hearing loss, autoimmune lymphoproliferative syndrome (ALPS), autoimmune myocarditis, autoimmune premature ovarian failure, blepharitis, bronchiectasis, bullous pemphigoid, cardiovascular disease, catastrophic antiphospholipid syndrome, celiac disease, cervical spondylosis, chronic ischemia, cicatricial pemphigoid, clinically isolated syndrome (cis) with risk for multiple sclerosis, childhood onset psychiatric disorder, dacryocystitis, dermatomyositis, diabetic retinopathy, disk herniation, disk prolaps, drug induced immune hemolytic anemia, endometriosis, endophthalmitis, episcleritis, erythema multiforme, erythema multiforme major, gestational pemphigoid, Guillain-Barré syndrome (GBS), Hughes syndrome, idiopathic Parkinson's disease, idiopathic interstitial pneumonia, IgE-mediated allergy, immune hemolytic anemia, inclusion body myositis, infectious ocular inflammatory disease, inflammatory demyelinating disease, inflammatory heart disease, inflammatory kidney disease, IPF/UIP, iritis, keratitis, keratojuntivitis sicca, Kussmaul disease or Kussmaul-Meier disease, Landry's paralysis, Langerhan's cell histiocytosis, livedo reticularis, macular degeneration, microscopic polyangiitis, morbus bechterev, motor neuron disorders, mucous membrane pemphigoid, multiple organ failure, myasthenia gravis, myelodysplastic syndrome, myocarditis, nerve root disorders, neuropathy, non-A non-B hepatitis, optic neuritis, osteolysis, pauciarticular JRA, peripheral artery occlusive disease (PAOD), peripheral vascular disease (PVD), peripheral artery, disease (PAD), phlebitis, polyarteritis nodosa (or periarteritis nodosa), polychondritis, poliosis, polyarticular JRA, polyendocrine deficiency syndrome, polymyositis, polymyalgia rheumatica (PMR), primary Parkinsonism, prostatitis, pure red cell aplasia, primary adrenal insufficiency, recurrent neuromyelitis optica, restenosis, rheumatic heart disease, sapho (synovitis, acne, pustulosis, hyperostosis, and osteitis), secondary amyloidosis, shock lung, scleritis, sciatica, secondary adrenal insufficiency, silicone associated connective tissue disease, sneddon-wilkinson dermatosis, spondilitis ankylosans, Stevens-Johnson syndrome (SJS), temporal arteritis, toxoplasmic retinitis, toxic epidermal necrolysis, transverse myelitis, TRAPS (tumor necrosis factor receptor, type 1 allergic reaction, type II diabetes, urticaria, usual interstitial pneumonia (UIP), vasculitis, vernal conjunctivitis, viral retinitis, Vogt-Koyanagi-Harada syndrome (VKH syndrome), wet macular degeneration, or wound healing.

In an embodiment, the binding proteins, or antigen-binding portions thereof, are used to treat cancer or in the prevention or inhibition of metastases from the tumors described herein either when used alone or in combination with radiotherapy and/or chemotherapeutic agents.

In another aspect, methods of treating a patient suffering from a disorder comprising the step of administering any one of the binding proteins disclosed herein before, concurrently, or after the administration of a second agent, are provided. In an embodiment, the second agent is budenoside, epidermal growth factor, a corticosteroid, cyclosporin, sulfasalazine, an aminosalicylate, 6-mercaptopurine, azathioprine, metronidazole, a lipoxygenase inhibitor, mesalamine, olsalazine, balsalazide, an antioxidant, a thromboxane inhibitor, an IL-1 receptor antagonist, an anti-IL-1β mAbs, an anti-IL-6 or IL-6 receptor mAb, a growth factor, an elastase inhibitor, a pyridinyl-imidazole compound, an antibody or agonist of TNF, LT, IL-1, IL-2, IL-6, IL-7, IL-8, IL-12, IL-13, IL-15, IL-16, IL-18, IL-23, EMAP-II, GM-CSF, FGF, or PDGF, an antibody to CD2, CD3, CD4, CD8, CD-19, CD25, CD28, CD30, CD40, CD45, CD69, CD90 or a ligand thereof, methotrexate, cyclosporin, FK506, rapamycin, mycophenolate mofetil, leflunomide, an NSAID, ibuprofen, prednisolone, a phosphodiesterase inhibitor, an adenosine agonist, an antithrombotic agent, a complement inhibitor, an adrenergic agent, IRAK, NIK, IKK, p38, a MAP kinase inhibitor, an IL-1β converting enzyme inhibitor, a TNFα-converting enzyme inhibitor, a T-cell signalling inhibitor, a metalloproteinase inhibitor, sulfasalazine, azathioprine, a 6-mercaptopurine, an angiotensin converting enzyme inhibitor, a soluble cytokine receptor, a soluble p55 TNF receptor, a soluble p75 TNF receptor, sIL-1RI, sIL-1RII, sIL-6R, an antiinflammatory cytokine, IL-4, IL-10, IL-11, IL-13, or TGFβ. In a particular embodiment, the pharmaceutical compositions disclosed herein are administered to a patient by parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, or transdermal administration.

Anti-idiotype antibodies to the binding proteins disclosed herein are also provided. An anti-idiotype antibody includes any protein or peptide-containing molecule that comprises at least a portion of an immunoglobulin molecule such as, but not limited to, at least one complementarily determining region (CDR) of a heavy or light chain or a ligand binding portion thereof, a heavy chain or light chain variable region, a heavy chain or light chain constant region, a framework region, or any portion thereof, that can be incorporated into a binding protein provided herein.

A method of determining the presence, amount or concentration of an antigen, or fragment thereof, in a test sample is provided. The method comprises assaying the test sample for the antigen, or fragment thereof, by an immunoassay. The immunoassay (i) employs at least one binding protein and at least one detectable label and (ii) comprises comparing a signal generated by the detectable label as a direct or indirect indication of the presence, amount or concentration of the antigen, or fragment thereof, in the test sample to a signal generated as a direct or indirect indication of the presence, amount or concentration of the antigen, or fragment thereof, in a control or a calibrator. The calibrator is optionally part of a series of calibrators in which each of the calibrators differs from the other calibrators in the series by the concentration of the antigen, or fragment thereof. The method can comprise (i) contacting the test sample with at least one capture agent, which binds to an epitope on the antigen, or fragment thereof, so as to form a capture agent/antigen, or fragment thereof, complex, (ii) contacting the capture agent/antigen, or fragment thereof, complex with at least one detection agent, which comprises a detectable label and binds to an epitope on the antigen, or fragment thereof, that is not bound by the capture agent, to form a capture agent/antigen, or fragment thereof/detection agent complex, and (iii) determining the presence, amount or concentration of the antigen, or fragment thereof, in the test sample based on the signal generated by the detectable label in the capture agent/antigen, or fragment thereof/detection agent complex formed in (ii), wherein at least one capture agent and/or at least one detection agent is the at least one binding protein.

Alternatively, the method can comprise (i) contacting the test sample with at least one capture agent, which binds to an epitope on the antigen, or fragment thereof, so as to form a capture agent/antigen, or fragment thereof, complex, and simultaneously or sequentially, in either order, contacting the test sample with detectably labeled antigen, or fragment thereof, which can compete with any antigen, or fragment thereof, in the test sample for binding to the at least one capture agent, wherein any antigen, or fragment thereof, present in the test sample and the detectably labeled antigen compete with each other to form a capture agent/antigen, or fragment thereof, complex and a capture agent/detectably labeled antigen, or fragment thereof, complex, respectively, and (ii) determining the presence, amount or concentration of the antigen, or fragment thereof, in the test sample based on the signal generated by the detectable label in the capture agent/detectably labeled antigen, or fragment thereof, complex formed in (ii), wherein at least one capture agent is the at least one binding protein and wherein the signal generated by the detectable label in the capture agent/detectably labeled antigen, or fragment thereof, complex is inversely proportional to the amount or concentration of antigen, or fragment thereof, in the test sample.

The test sample can be from a patient, in which case the method can further comprise diagnosing, prognosticating, or assessing the efficacy of therapeutic/prophylactic treatment of the patient. If the method further comprises assessing the efficacy of therapeutic/prophylactic treatment of the patient, the method optionally further comprises modifying the therapeutic/prophylactic treatment of the patient as needed to improve efficacy. The method can be adapted for use in an automated system or a semi-automated system. Accordingly, the methods described herein also can be used to determine whether or not a subject has or is at risk of developing a given disease, disorder or condition. Specifically, such a method can comprise the steps of:
(a) determining the concentration or amount in a test sample from a subject of analyte, or fragment thereof, (e.g., using the methods described herein, or methods known in the art); and
(b) comparing the concentration or amount of analyte, or fragment thereof, determined in step (a) with a predetermined level, wherein, if the concentration or amount of analyte determined in step (a) is favorable with respect to a predetermined level, then the subject is determined not to have or be at risk for a given disease, disorder or condition. However, if the concentration or amount of analyte determined in step (a) is unfavorable with respect to the predetermined level, then the subject is determined to have or be at risk for a given disease, disorder or condition.

Additionally, provided herein is method of monitoring the progression of disease in a subject. Optimally the method comprising the steps of:
(a) determining the concentration or amount in a test sample from a subject of analyte;
(b) determining the concentration or amount in a later test sample from the subject of analyte; and
(c) comparing the concentration or amount of analyte as determined in step (b) with the concentration or amount of analyte determined in step (a), wherein if the concentration or amount determined in step (b) is unchanged or is unfavorable when compared to the concentration or amount of analyte determined in step (a), then the disease in the subject is determined to have continued, progressed or worsened. By comparison, if the concentration or amount of analyte as determined in step (b) is favorable when compared to the concentration or amount of analyte as determined in step (a), then the disease in the subject is determined to have discontinued, regressed or improved.

Optionally, the method further comprises comparing the concentration or amount of analyte as determined in step (b), for example, with a predetermined level. Further, optionally the method comprises treating the subject with one or more pharmaceutical compositions for a period of time if the comparison shows that the concentration or amount of analyte as determined in step (b), for example, is unfavorably altered with respect to the predetermined level.

Also provided is a kit for assaying a test sample for targets of the binding protein or fragment thereof. The kit comprises at least one component for assaying the test sample for an antigen, or fragment thereof, and instructions for assaying the test sample for an antigen, or fragment thereof, wherein the at least one component includes at least one composition comprising the binding protein disclosed herein, wherein the binding protein is optionally detectably labeled.

### Brief Description of the Drawings

Figure 1A is a schematic representation of Dual Variable Domain (DVD) binding protein constructs generally.
Figure 1B provides a schematic representation of a Dual Variable Domain (DVD) binding protein of the invention with a VH/VL crossover. In this representation, VD1 differs from Figure 1A. Namely, VH1 and VL1 domains were crossed over on both arms of the of the DVD binding protein. VH1 appears on the light chain of both arms, and VL1 appears on the heavy chain of both arms.

### Detailed Description

Multivalent and/or multispecific binding proteins with a VH/VL crossover are provided. The binding proteins bind TNF and one of PGE2, SOST, NGF, and LPA. Dual variable domain binding proteins (DVD binding proteins) or dual variable domain immunoglobulins (DVD-Ig™), and pharmaceutical compositions thereof, as well as nucleic acids, recombinant expression vectors and host cells for making such DVD binding proteins are also provided. Methods of using the DVD binding proteins to detect specific antigens, either in vitro or in vivo are also provided.

Unless otherwise defined herein, scientific and technical terms used herein have the meanings that are commonly understood by those of ordinary skill in the art. In the event of any latent ambiguity, definitions provided herein take precedent over any dictionary or extrinsic definition. Unless otherwise required by context, singular terms shall include pluralities and plural terms shall include the singular. The use of "or" means "and/or" unless stated otherwise. The use of the term "including", as well as other forms, such as "includes" and "included", is not limiting.

Generally, nomenclatures used in connection with cell and tissue culture, molecular biology, immunology, microbiology, genetics and protein and nucleic acid chemistry and hybridization described herein are those well known and commonly used in the art. The methods and techniques provided herein are generally performed according to conventional methods well known in the art and as described in various general and more specific references that are cited and discussed throughout the present specification unless otherwise indicated. Enzymatic reactions and purification techniques are performed according to manufacturer's specifications, as commonly accomplished in the art or as described herein. The nomenclatures used in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

That the disclosure may be more readily understood, select terms are defined below.

The term "antibody" refers to an immunoglobulin (Ig) molecule, which is generally comprised of four polypeptide chains, two heavy (H) chains and two light (L) chains, or a functional fragment, mutant, variant, or derivative thereof, that retains the epitope binding features of an Ig molecule. Such fragment, mutant, variant, or derivative antibody formats are known in the art. In an embodiment of a full-length antibody, each heavy chain is comprised of a heavy chain variable region (VH) and a heavy chain constant region (CH). The CH is comprised of three domains, CH1, CH2 and CH3. Each light chain is comprised of a light chain variable region (VL) and a light chain constant region (CL). The CL is comprised of a single CL domain. The VH and VL can be further subdivided into regions of hypervariability, termed complementarity determining regions (CDRs), interspersed with regions that are more conserved, termed framework regions (FRs). Generally, each VH and VL is composed of three CDRs and four FRs, arranged from amino-terminus to carboxy-terminus in the following order: FR1, CDR1, FR2, CDR2, FR3, CDR3, and FR4. Immunoglobulin molecules can be of any type (e.g., IgG, IgE, IgM, IgD, IgA and IgY), class (e.g., IgG1, IgG2, IgG3, IgG4, IgA1 and IgA2), or subclass.

The term "heavy chain" when referring to the binding proteins herein is used when the VD2 domain is a VH domain and the constant domain is all or part of a heavy chain constant domain. The heavy chain may include a VL at the VD1 position.

The term "light chain" when referring to the binding proteins herein is used when the VD2 domain is a VL domain and the constant domain is all or part of a light chain constant domain. The light chain may include a VH at the VD1 position.

The term "bispecific antibody" refers to an antibody that binds one antigen (or epitope) on one of its two binding arms (one pair of HC/LC), and binds a different antigen (or epitope) on its second binding arm (a different pair of HC/LC). A bispecific antibody has two distinct antigen binding arms (in both specificity and CDR sequences), and is monovalent for each antigen to which it binds. Bispecific antibodies include those generated by quadroma technology (Milstein and Cuello (1983) Nature 305(5934): 537-40), by chemical conjugation of two different monoclonal antibodies (Staerz et al. (1985) Nature 314(6012): 628-31), or by knob-into-hole or similar approaches which introduces mutations in the Fc region (Holliger et al. (1993) Proc. Natl. Acad. Sci. USA 90(14): 6444-6448).

An "affinity matured" antibody is an antibody with one or more alterations in one or more CDRs thereof which result an improvement in the affinity of the antibody for antigen, compared to a parent antibody which does not possess those alteration(s). Exemplary affinity matured antibodies will have nanomolar or even picomolar affinities for the target antigen. Affinity matured antibodies are produced by procedures known in the art. Marks et al. (1992) BioTechnology 10:779-783 describes affinity maturation by VH and VL domain shuffling. Random mutagenesis of CDR and/or framework residues is described by Barbas et al. (1994) Proc. Nat. Acad. Sci. USA 91:3809-3813; Schier et al. (1995) Gene 169:147-155; Yelton et al. (1995) J. Immunol. 155:1994-2004; Jackson et al. (1995) J. Immunol. 154(7):3310-9; Hawkins et al. (1992) J. Mol. Biol. 226:889-896 and mutation at selective mutagenesis positions, contact or hypermutation positions with an activity enhancing amino acid residue as described in US Patent No. 6,914,128.

The term "CDR-grafted antibody" refers to an antibody that comprises heavy and light chain variable region sequences in which the sequences of one or more of the CDR regions of VH and/or VL are replaced with CDR sequences of another antibody. For example, the two antibodies can be from different species, such as antibodies having murine heavy and light chain variable regions in which one or more of the murine CDRs has been replaced with human CDR sequences.

The term "humanized antibody" refers to an antibody from a non-human species that has been altered to be more "human-like", i.e., more similar to human germline sequences. One type of humanized antibody is a CDR-grafted antibody, in which non-human CDR sequences are introduced into human VH and VL sequences to replace the corresponding human CDR sequences. A "humanized antibody" is also an antibody or a variant, derivative, analog or fragment thereof that comprises framework region (FR) sequences having substantially (e.g., at least 80%, at least 85%, at least 90%, at least 95%, at least 98% or at least 99% identity to) the amino acid sequence of a human antibody and at least one CDR having substantially the amino acid sequence of a non-human antibody. A humanized antibody may comprise substantially all of at least one, and typically two, variable domains (Fab, Fab', F(ab') 2, FabC, Fv) in which the sequence of all or substantially all of the CDR regions correspond to those of a non-human immunoglobulin (i.e., donor antibody) and the sequence of all or substantially all of the FR regions are those of a human immunoglobulin. The humanized antibody also may include the CH1, hinge, CH2, CH3, and CH4 regions of the heavy chain. In an embodiment, a humanized antibody also comprises at least a portion of a human immunoglobulin Fc region. In some embodiments, a humanized antibody only contains a humanized light chain. In some embodiments, a humanized antibody only contains a humanized heavy chain. In some embodiments, a humanized antibody only contains a humanized variable domain of a light chain and/or humanized variable domain of a heavy chain. In some embodiments, a humanized antibody contains a light chain as well as at least the variable domain of a heavy chain. In some embodiments, a humanized antibody contains a heavy chain as well as at least the variable domain of a light chain.

The terms "dual variable domain binding protein" and "dual variable domain immunoglobulin" refer to a binding protein that has two variable domains in each of its two binding arms (e.g., a pair of HC/LC) (see PCT Publication No. WO 02/02773), each of which is able to bind to an antigen. In an embodiment, each variable domain binds different antigens or epitopes. In another embodiment, each variable domain binds the same antigen or epitope. In another embodiment, a dual variable domain binding protein has two identical antigen binding arms, with identical specificity and identical CDR sequences, and is bivalent for each antigen to which it binds. In an embodiment, the DVD binding proteins may be monospecific, i.e., capable of binding one antigen or multispecific, i.e., capable of binding two or more antigens. DVD binding proteins comprising two heavy chain DVD polypeptides and two light chain DVD polypeptides are referred to as a DVD-Ig™. In an embodiment, each half of a four chain DVD binding protein comprises a heavy chain DVD polypeptide, and a light chain DVD polypeptide, and two antigen binding sites. In an embodiment, each binding site comprises a heavy chain variable domain and a light chain variable domain with a total of 6 CDRs involved in antigen binding per antigen binding site.

The term "antiidiotypic antibody" refers to an antibody raised against the amino acid sequence of the antigen combining site of another antibody. Antiidiotypic antibodies may be administered to enhance an immune response against an antigen.

The term "biological activity" refers to any one or more biological properties of a molecule (whether present naturally as found in vivo, or provided or enabled by recombinant means). Biological properties include, but are not limited to, binding a receptor, inducing cell proliferation, inhibiting cell growth, inducing other cytokines, inducing apoptosis, and enzymatic activity.

The term "neutralizing" refers to counteracting the biological activity of an antigen when a binding protein specifically binds to the antigen. In an embodiment, the neutralizing binding protein binds to an antigen (e.g., a cytokine) and reduces its biologically activity by at least about 20%, 40%, 60%, 80%, 85% or more.

"Specificity" refers to the ability of a binding protein to selectively bind an antigen.

"Affinity" is the strength of the interaction between a binding protein and an antigen, and is determined by the sequence of the CDRs of the binding protein as well as by the nature of the antigen, such as its size, shape, and/or charge. Binding proteins may be selected for affinities that provide desired therapeutic end-points while minimizing negative side-effects. Affinity may be measured using methods known to one skilled in the art (US 20090311253).

The term "potency" refers to the ability of a binding protein to achieve a desired effect, and is a measurement of its therapeutic efficacy. Potency may be assessed using methods known to one skilled in the art (US 20090311253).

The term "cross-reactivity" refers to the ability of a binding protein to bind a target other than that against which it was raised. Generally, a binding protein will bind its target tissue(s)/antigen(s) with an appropriately high affinity, but will display an appropriately low affinity for non-target normal tissues. Individual binding proteins are generally selected to meet two criteria. (1) Tissue staining appropriate for the known expression of the antibody target. (2) Similar staining pattern between human and tox species (mouse and cynomolgus monkey) tissues from the same organ. These and other methods of assessing cross-reactivity are known to one skilled in the art (US 20090311253).

The term "biological function" refers the specific *in vitro* or *in vivo* actions of a binding protein. Binding proteins may target several classes of antigens and achieve desired therapeutic outcomes through multiple mechanisms of action. Binding proteins may target soluble proteins, cell surface antigens, as well as extracellular protein deposits. Binding proteins may agonize, antagonize, or neutralize the activity of their targets. Binding proteins may assist in the clearance of the targets to which they bind, or may result in cytotoxicity when bound to cells. Portions of two or more antibodies may be incorporated into a multivalent format to achieve distinct functions in a single binding protein molecule. The *in vitro* assays and *in vivo* models used to assess biological function are known to one skilled in the art (US 20090311253).

A "stable" binding protein is one in which the binding protein essentially retains its physical stability, chemical stability and/or biological activity upon storage. A multivalent binding protein that is stable *in vitro* at various temperatures for an extended period of time is desirable. Methods of stabilizing binding proteins and assessing their stability at various temperatures are known to one skilled in the art (US 20090311253).

The term "solubility" refers to the ability of a protein to remain dispersed within an aqueous solution. The solubility of a protein in an aqueous formulation depends upon the proper distribution of hydrophobic and hydrophilic amino acid residues, and therefore, solubility can correlate with the production of correctly folded proteins. A person skilled in the art will be able to detect an increase or decrease in solubility of a binding protein using routine HPLC techniques and methods known to one skilled in the art (US 20090311253).

Binding proteins may be produced using a variety of host cells or may be produced in vitro, and the relative yield per effort determines the "production efficiency." Factors influencing production efficiency include, but are not limited to, host cell type (prokaryotic or eukaryotic), choice of expression vector, choice of nucleotide sequence, and methods employed. The materials and methods used in binding protein production, as well as the measurement of production efficiency, are known to one skilled in the art (US 20090311253).

The term "immunogenicity" means the ability of a substance to induce an immune response. Administration of a therapeutic binding protein may result in a certain incidence of an immune response. Potential elements that might induce immunogenicity in a multivalent format may be analyzed during selection of the parental antibodies, and steps to reduce such risk can be taken to optimize the parental antibodies prior to incorporating their sequences into a multivalent binding protein format. Methods of reducing the immunogenicity of antibodies and binding proteins are known to one skilled in the art (US 20090311253).

The terms "label" and "detectable label" mean a moiety attached to a member of a specific binding pair, such as an antibody or its analyte to render a reaction (e.g., binding) between the members of the specific binding pair, detectable. The labeled member of the specific binding pair is referred to as "detectably labeled." Thus, the term "labeled binding protein" refers to a protein with a label incorporated that provides for the identification of the binding protein. In an embodiment, the label is a detectable marker that can produce a signal that is detectable by visual or instrumental means, e.g., incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). Examples of labels for polypeptides include, but are not limited to, the following: radioisotopes or radionuclides (e.g., ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm); chromogens, fluorescent labels (e.g., FITC, rhodamine, lanthanide phosphors), enzymatic labels (e.g., horseradish peroxidase, luciferase, alkaline phosphatase); chemiluminescent markers; biotinyl groups; predetermined polypeptide epitopes recognized by a secondary reporter (e.g., leucine zipper pair sequences, binding sites for secondary antibodies, metal binding domains, epitope tags); and magnetic agents, such as gadolinium chelates. Representative examples of labels commonly employed for immunoassays include moieties that produce light, e.g., acridinium compounds, and moieties that produce fluorescence, e.g., fluorescein. In this regard, the moiety itself may not be detectably labeled but may become detectable upon reaction with yet another moiety.

The term "conjugate" refers to a binding protein, such as an antibody, that is chemically linked to a second chemical moiety, such as a therapeutic or cytotoxic agent. The term "agent" includes a chemical compound, a mixture of chemical compounds, a biological macromolecule, or an extract made from biological materials. In an embodiment, the therapeutic or cytotoxic agents include, but are not limited to, pertussis toxin, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. When employed in the context of an immunoassay, the conjugate antibody may be a detectably labeled antibody used as the detection antibody.

The terms "crystal" and "crystallized" refer to a binding protein (e.g., an antibody), or antigen binding portion thereof, that exists in the form of a crystal. Crystals are one form of the solid state of matter, which is distinct from other forms such as the amorphous solid state or the liquid crystalline state. Crystals are composed of regular, repeating, three-dimensional arrays of atoms, ions, molecules (e.g., proteins such as antibodies), or molecular assemblies (e.g., antigen/antibody complexes). These three-dimensional arrays are arranged according to specific mathematical relationships that are well-understood in the field. The fundamental unit, or building block, that is repeated in a crystal is called the asymmetric unit. Repetition of the asymmetric unit in an arrangement that conforms to a given, well-defined crystallographic symmetry provides the "unit cell" of the crystal. Repetition of the unit cell by regular translations in all three dimensions provides the crystal. See Giege, R. and Ducruix, A. Barrett, CRYSTALLIZATION OF NUCLEIC ACIDS AND PROTEINS, A PRACTICAL APPROACH, 2nd ea., pp. 20 1-16, Oxford University Press, New York, New York, (1999).

The term "vector" refers to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Other vectors include RNA vectors. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, other forms of expression vectors are also included, such as viral vectors (e.g., replication defective retroviruses, adenoviruses and adeno-associated viruses), which serve equivalent functions. A group of pHybE vectors (US Patent Application Serial No. 61/021,282) were used for parental antibody and DVD-binding protein cloning. V1, derived from pJP183; pHybE-hCg1,z,non-a V2, was used for cloning of antibody and DVD heavy chains with a wildtype constant region. V2, derived from pJP191; pHybE-hCk V3, was used for cloning of antibody and DVD light chains with a kappa constant region. V3, derived from pJP192; pHybE-hCl V2, was used for cloning of antibody and DVDs light chains with a lambda constant region. V4, built with a lambda signal peptide and a kappa constant region, was used for cloning of DVD light chains with a lambda-kappa hybrid V domain. V5, built with a kappa signal peptide and a lambda constant region, was used for cloning of DVD light chains with a kappa-lambda hybrid V domain. V7, derived from pJP183; pHybE-hCg1,z,non-a V2, was used for cloning of antibody and DVD heavy chains with a (234,235 AA) mutant constant region.

The terms "recombinant host cell" or "host cell" refer to a cell into which exogenous DNA has been introduced. Such terms refer not only to the particular subject cell, but to the progeny of such a cell. Because certain modifications may occur in succeeding generations due to either mutation or environmental influences, such progeny may not, in fact, be identical to the parent cell, but are still included within the scope of the term "host cell" as used herein. In an embodiment, host cells include prokaryotic and eukaryotic cells. In an embodiment, eukaryotic cells include protist, fungal, plant and animal cells. In another embodiment, host cells include but are not limited to the prokaryotic cell line *E.Coli;* mammalian cell lines CHO, HEK 293, COS, NS0, SP2 and PER.C6; the insect cell line Sf9; and the fungal cell *Saccharomyces cerevisiae.*

The term "transfection" encompasses a variety of techniques commonly used for the introduction of exogenous nucleic acid (e.g., DNA) into a host cell, e.g., electroporation, calcium-phosphate precipitation, DEAE-dextran transfection and the like.

The term "cytokine" refers to a protein released by one cell population that acts on another cell population as an intercellular mediator. The term "cytokine" includes proteins from natural sources or from recombinant cell culture and biologically active equivalents of the native sequence cytokines.

The term "biological sample" means a quantity of a substance from a living thing or formerly living thing. Such substances include, but are not limited to, blood, (e.g., whole blood), plasma, serum, urine, amniotic fluid, synovial fluid, endothelial cells, leukocytes, monocytes, other cells, organs, tissues, bone marrow, lymph nodes and spleen.

The term "component" refers to an element of a composition. In relation to a diagnostic kit, for example, a component may be a capture antibody, a detection or conjugate antibody, a control, a calibrator, a series of calibrators, a sensitivity panel, a container, a buffer, a diluent, a salt, an enzyme, a co-factor for an enzyme, a detection reagent, a pretreatment reagent/solution, a substrate (e.g., as a solution), a stop solution, and the like that can be included in a kit for assay of a test sample. Thus, a "component" can include a polypeptide or other analyte as above, that is immobilized on a solid support, such as by binding to an anti-analyte (e.g., anti-polypeptide) antibody. Some components can be in solution or lyophilized for reconstitution for use in an assay.

"Control" refers to a composition known to not analyte ("negative control") or to contain analyte ("positive control"). A positive control can comprise a known concentration of analyte. "Control," "positive control," and "calibrator" may be used interchangeably herein to refer to a composition comprising a known concentration of analyte. A "positive control" can be used to establish assay performance characteristics and is a useful indicator of the integrity of reagents (e.g., analytes).

"Predetermined cutoff" and "predetermined level" refer generally to an assay cutoff value that is used to assess diagnostic/prognostic/therapeutic efficacy results by comparing the assay results against the predetermined cutoff/level, where the predetermined cutoff/level already has been linked or associated with various clinical parameters (e.g., severity of disease, progression/nonprogression/improvement, etc.). While the present disclosure may provide exemplary predetermined levels, it is well-known that cutoff values may vary depending on the nature of the immunoassay (e.g., antibodies employed, etc.). It further is well within the ordinary skill of one in the art to adapt the disclosure herein for other immunoassays to obtain immunoassay-specific cutoff values for those other immunoassays based on this disclosure. Whereas the precise value of the predetermined cutoff/level may vary between assays, correlations as described herein (if any) may be generally applicable.

"Pretreatment reagent," e.g., lysis, precipitation and/or solubilization reagent, as used in a diagnostic assay as described herein is one that lyses any cells and/or solubilizes any analyte that is/are present in a test sample. Pretreatment is not necessary for all samples, as described further herein. Among other things, solubilizing the analyte (e.g., polypeptide of interest) may entail release of the analyte from any endogenous binding proteins present in the sample. A pretreatment reagent may be homogeneous (not requiring a separation step) or heterogeneous (requiring a separation step). With use of a heterogeneous pretreatment reagent there is removal of any precipitated analyte binding proteins from the test sample prior to proceeding to the next step of the assay.

"Quality control reagents" in the context of immunoassays and kits described herein, include, but are not limited to, calibrators, controls, and sensitivity panels. A "calibrator" or "standard" typically is used (e.g., one or more, such as a plurality) in order to establish calibration (standard) curves for interpolation of the concentration of an analyte, such as an antibody or an analyte. Alternatively, a single calibrator, which is near a predetermined positive/negative cutoff, can be used. Multiple calibrators (i.e., more than one calibrator or a varying amount of calibrator(s)) can be used in conjunction so as to comprise a "sensitivity panel."

The term "specific binding partner" is a member of a specific binding pair. A specific binding pair comprises two different molecules that specifically bind to each other through chemical or physical means. Therefore, in addition to antigen and antibody specific binding, other specific binding pairs can include biotin and avidin (or streptavidin), carbohydrates and lectins, complementary nucleotide sequences, effector and receptor molecules, cofactors and enzymes, enzyme inhibitors and enzymes, and the like. Furthermore, specific binding pairs can include members that are analogs of the original specific binding members, for example, an analyte-analog. Immunoreactive specific binding members include antigens, antigen fragments, and antibodies, including monoclonal and polyclonal antibodies as well as complexes, fragments, and variants (including fragments of variants) thereof, whether isolated or recombinantly produced.

The term "Fc region" defines the C-terminal region of an immunoglobulin heavy chain, which may be generated by papain digestion of an intact antibody. The Fc region may be a native sequence Fc region or a variant Fc region. The Fc region of an immunoglobulin generally comprises two constant domains, a CH2 domain and a CH3 domain, and optionally comprises a CH4 domain. Replacements of amino acid residues in the Fc portion to alter antibody effector function are known in the art (e.g., US Patent Nos. 5,648,260 and 5,624,821). The Fc region mediates several important effector functions, e.g., cytokine induction, antibody dependent cell mediated cytotoxicity (ADCC), phagocytosis, complement dependent cytotoxicity (CDC), and half-life/ clearance rate of antibody and antigen-antibody complexes. In some cases these effector functions are desirable for a therapeutic immunoglobulin but in other cases might be unnecessary or even deleterious, depending on the therapeutic objectives.

The term "antigen-binding portion" of a binding protein means one or more fragments of a binding protein (e.g., an antibody) that retain the ability to specifically bind to an antigen. The antigen-binding portion of a binding protein can be performed by fragments of a full-length antibody, as well as bispecific, dual specific, or multi-specific formats; specifically binding to two or more different antigens. Examples of binding fragments encompassed within the term "antigen-binding portion" of an binding protein include (i) an Fab fragment, a monovalent fragment consisting of the VL, VH, CL and CH1 domains; (ii) an F(ab')₂ fragment, a bivalent fragment comprising two Fab fragments linked by a disulfide bridge at the hinge region; (iii) an Fd fragment consisting of the VH and CH1 domains; (iv) an Fv fragment consisting of the VL and VH domains of a single arm of an antibody, (v) a dAb fragment, which comprises a single variable domain; and (vi) an isolated complementarity determining region (CDR). Furthermore, although the two domains of the Fv fragment, VL and VH, encoded by separate genes, they can be joined, using recombinant methods, by a synthetic linker that enables them to be made as a single protein chain in which the VL and VH regions pair to form monovalent molecules (known as single chain Fv (scFv). Such single chain antibodies are also intended to be encompassed within the term "antigen-binding portion" of an antibody. Other forms of single chain antibodies, such as diabodies are also encompassed. In addition, single chain antibodies also include "linear antibodies" comprising a pair of tandem Fv segments (VH-CH1-VH-CH1) which, together with complementary light chain polypeptides, form a pair of antigen binding regions.

The term "multivalent binding protein" means a binding protein comprising two or more antigen binding sites. In an embodiment, the multivalent binding protein is engineered to have three or more antigen binding sites, and is not a naturally occurring antibody. The term "multispecific binding protein" refers to a binding protein capable of binding two or more related or unrelated targets. In an embodiment, the dual variable domain (DVD) binding proteins provided herein comprise two or more antigen binding sites and are tetravalent or multivalent binding proteins.

The term "linker" means an amino acid residue or a polypeptide comprising two or more amino acid residues joined by peptide bonds that are used to link two polypeptides (e.g., two VH or two VL domains). Such linker polypeptides are well known in the art (see, e.g., Holliger et al. (1993) Proc. Natl. Acad. Sci. USA 90:6444-6448; Poljak et al. (1994) Structure 2:1121-1123).

The terms "Kabat numbering", "Kabat definitions" and "Kabat labeling" are used interchangeably herein. These terms, which are recognized in the art, refer to a system of numbering amino acid residues which are more variable (i.e., hypervariable) than other amino acid residues in the heavy and light chain variable regions of an antibody, or an antigen binding portion thereof (Kabat et al. (1971) Ann. NY Acad. Sci. 190:382-391 and, Kabat et al. (1991) Sequences of Proteins of Immunological Interest, Fifth Edition, U.S. Department of Health and Human Services, NIH Publication No. 91-3242). For the heavy chain variable region, the hypervariable region ranges from amino acid positions 31 to 35 for CDR1, amino acid positions 50 to 65 for CDR2, and amino acid positions 95 to 102 for CDR3. For the light chain variable region, the hypervariable region ranges from amino acid positions 24 to 34 for CDR1, amino acid positions 50 to 56 for CDR2, and amino acid positions 89 to 97 for CDR3.

The term "CDR" means a complementarity determining region within an immunoglobulin variable region sequence. There are three CDRs in each of the variable regions of the heavy chain and the light chain, which are designated CDR1, CDR2 and CDR3, for each of the heavy and light chain variable regions. The term "CDR set" refers to a group of three CDRs that occur in a single variable region capable of binding the antigen. The exact boundaries of these CDRs have been defined differently according to different systems. The system described by Kabat (Kabat et al. (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia and Lesk (1987) J. Mol. Biol. 196:901-917; Chothia et al. (1989) Nature 342:877-883) found that certain sub- portions within Kabat CDRs adopt nearly identical peptide backbone conformations, despite having great diversity at the level of amino acid sequence. These sub-portions were designated as L1, L2 and L3 or H1, H2 and H3 where the "L" and the "H" designates the light chain and the heavy chain regions, respectively. These regions may be referred to as Chothia CDRs, which have boundaries that overlap with Kabat CDRs. Other boundaries defining CDRs overlapping with the Kabat CDRs have been described by Padlan (1995) FASEB J. 9:133-139 and MacCallum (1996) J. Mol. Biol. 262(5):732-45). Still other CDR boundary definitions may not strictly follow one of the herein systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although certain embodiments use Kabat or Chothia defined CDRs.

The term "epitope" means a region of an antigen that is bound by a binding protein, e.g., a polypeptide and/or other determinant capable of specific binding to an immunoglobulin or T-cell receptor. In certain embodiments, epitope determinants include chemically active surface groupings of molecules such as amino acids, sugar side chains, phosphoryl, or sulfonyl, and, in certain embodiments, may have specific three dimensional structural characteristics, and/or specific charge characteristics. In an embodiment, an epitope comprises the amino acid residues of a region of an antigen (or fragment thereof) known to bind to the complementary site on the specific binding partner. An antigenic fragment can contain more than one epitope. In certain embodiments, a binding protein specifically binds an antigen when it recognizes its target antigen in a complex mixture of proteins and/or macromolecules. Binding proteins "bind to the same epitope" if the antibodies cross-compete (one prevents the binding or modulating effect of the other). In addition, structural definitions of epitopes (overlapping, similar, identical) are informative; and functional definitions encompass structural (binding) and functional (modulation, competition) parameters. Different regions of proteins may perform different functions. For example specific regions of a cytokine interact with its cytokine receptor to bring about receptor activation whereas other regions of the protein may be required for stabilizing the cytokine. To abrogate the negative effects of cytokine signaling, the cytokine may be targeted with a binding protein that binds specifically to the receptor interacting region(s), thereby preventing the binding of its receptor. Alternatively, a binding protein may target the regions responsible for cytokine stabilization, thereby designating the protein for degradation. The methods of visualizing and modeling epitope recognition are known to one skilled in the art (US 20090311253).

"Pharmacokinetics" refers to the process by which a drug is absorbed, distributed, metabolized, and excreted by an organism. To generate a multivalent binding protein molecule with a desired pharmacokinetic profile, parent monoclonal antibodies with similarly desired pharmacokinetic profiles are selected. The PK profiles of the selected parental monoclonal antibodies can be easily determined in rodents using methods known to one skilled in the art (US 20090311253).

"Bioavailability" refers to the amount of active drug that reaches its target following administration. Bioavailability is function of several of the previously described properties, including stability, solubility, immunogenicity and pharmacokinetics, and can be assessed using methods known to one skilled in the art (US 20090311253).

The term "surface plasmon resonance" means an optical phenomenon that allows for the analysis of real-time biospecific interactions by detection of alterations in protein concentrations within a biosensor matrix, for example using the BIAcore® system (BIAcore International AB, a GE Healthcare company, Uppsala, Sweden and Piscataway, NJ). For further descriptions, see Jönsson et al. (1993) Ann. Biol. Clin. 51:19-26. The term "Kₒₙ" means the on rate constant for association of a binding protein (e.g., an antibody or DVD-Ig) to the antigen to form the, e.g., DVD-Ig/antigen complex. The term "Kₒₙ" also means "association rate constant", or "ka", as is used interchangeably herein. This value indicating the binding rate of a binding protein to its target antigen or the rate of complex formation between a binding protein, e.g., an antibody, and antigen also is shown by the equation below:

Antibody ("Ab") + Antigen ("Ag")→Ab-Ag

The term "K_{off}" means the off rate constant for dissociation, or "dissociation rate constant", of a binding protein (e.g., an antibody or DVD-Ig) from the, e.g., DVD-Ig/antigen complex as is known in the art. This value indicates the dissociation rate of a binding protein, e.g., an antibody, from its target antigen or separation of Ab-Ag complex over time into free antibody and antigen as shown by the equation below:

Ab + Ag←Ab-Ag

The terms "K_{d}" and "equilibrium dissociation constant" means the value obtained in a titration measurement at equilibrium, or by dividing the dissociation rate constant (K_{off}) by the association rate constant (Kₒₙ). The association rate constant, the dissociation rate constant and the equilibrium dissociation constant, are used to represent the binding affinity of a binding protein (e.g., an antibody or DVD-Ig) to an antigen. Methods for determining association and dissociation rate constants are well known in the art. Using fluorescence-based techniques offers high sensitivity and the ability to examine samples in physiological buffers at equilibrium. Other experimental approaches and instruments such as a BIAcore® (biomolecular interaction analysis) assay, can be used (e.g., instrument available from BIAcore International AB, a GE Healthcare company, Uppsala, Sweden). Additionally, a KinExA® (Kinetic Exclusion Assay) assay, available from Sapidyne Instruments (Boise, Idaho), can also be used.

The term "variant" means a polypeptide that differs from a given polypeptide in amino acid sequence by the addition (e.g., insertion), deletion, or conservative substitution of amino acids, but that retains the biological activity of the given polypeptide (e.g., a variant TNF antibody can compete with anti-TNF antibody for binding to TNF). A conservative substitution of an amino acid, i.e., replacing an amino acid with a different amino acid of similar properties (e.g., hydrophilicity and degree and distribution of charged regions) is recognized in the art as typically involving a minor change. These minor changes can be identified, in part, by considering the hydropathic index of amino acids, as understood in the art (see, e.g., Kyte et al. (1982) J. Mol. Biol. 157: 105-132). The hydropathic index of an amino acid is based on a consideration of its hydrophobicity and charge. It is known in the art that amino acids of similar hydropathic indexes in a protein can be substituted and the protein still retains protein function. In one aspect, amino acids having hydropathic indexes of ± 2 are substituted. The hydrophilicity of amino acids also can be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity (see, e.g., US Patent No. 4,554,101). Substitution of amino acids having similar hydrophilicity values can result in peptides retaining biological activity, for example immunogenicity, as is understood in the art. In one aspect, substitutions are performed with amino acids having hydrophilicity values within ± 2 of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly the side chains of those amino acids, as revealed by the hydrophobicity, hydrophilicity, charge, size, and other properties. The term "variant" also includes polypeptide or fragment thereof that has been differentially processed, such as by proteolysis, phosphorylation, or other post-translational modification, yet retains its biological activity or antigen reactivity, e.g., the ability to bind to TNF or another antigen. The term "variant" encompasses fragments of a variant unless otherwise defined. A variant may be 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, or 75% identical to the wildtype sequence.

### I. Generation of binding proteins

Multivalent and/or multispecific binding proteins with a VH/VL crossover are provided. The binding proteins bind TNF and one of PGE2, SOST, NGF, and LPA. The binding protein can be generated using various techniques. Expression vectors, host cell and methods of generating the binding protein are provided and are well known in the art.

### A. Generation of parent monoclonal antibodies

The variable domains of the DVD binding protein can be obtained from parent antibodies, including polyclonal Abs and mAbs capable of binding antigens of interest. These antibodies may be naturally occurring or may be generated by recombinant technology. The person of ordinary skill in the art is well familiar with many methods for producing antibodies, including, but not limited to using hybridoma techniques, selected lymphocyte antibody method (SLAM), use of a phage, yeast, or RNA-protein fusion display or other library, immunizing a non-human animal comprising at least some of the human immunoglobulin locus, and preparation of chimeric, CDR-grafted, and humanized antibodies. See, e.g., US Patent Publication No. 20090311253 A1. Variable domains may also be prepared using affinity maturation techniques.

### B. Criteria for selecting parent monoclonal antibodies

An embodiment is provided comprising selecting parent antibodies with at least one or more properties desired in the DVD binding protein molecule. In an embodiment, the desired property is one or more antibody parameters, such as, for example, antigen specificity, affinity to antigen, potency, biological function, epitope recognition, stability, solubility, production efficiency, immunogenicity, pharmacokinetics, bioavailability, tissue cross reactivity, or orthologous antigen binding. See, e.g., US Patent Publication No. 20090311253.

### C. Construction of binding protein molecules

The binding proteins may be designed such that two different variable domains from two different parental monoclonal antibodies are linked in tandem directly or via a linker by recombinant DNA techniques, followed by a constant domain. In the present invention, the light chain comprises VH1 and VL2, whereas the heavy chain comprises VL1 and VH2. Specifically, in the outer, or VD1 position, instead of having VL1 appear on the light chain, and VH1 appear on the heavy chain, these positions are crossedover (or switched). VH1 appears on the light chain along with a VL2 and optionally other domains such as linkers and constant domains, as described herein, and VL1 appears on the heavy chain along with a VH2 and optionally other domains such as linkers and constant domains, as described herein.

The variable domains can be obtained using recombinant DNA techniques from parent antibodies generated by any one of the methods described herein. In an embodiment, the variable domain is a murine heavy or light chain variable domain. In another embodiment, the variable domain is a CDR grafted or a humanized variable heavy or light chain domain. In an embodiment, the variable domain is a human heavy or light chain variable domain.

The linker sequence may be a single amino acid or a polypeptide sequence. In an embodiment, the choice of linker sequences is based on crystal structure analysis of several Fab molecules. There is a natural flexible linkage between the variable domain and the CH1/CL constant domain in Fab or antibody molecular structure. This natural linkage comprises approximately 10-12 amino acid residues, contributed by 4-6 residues from the C-terminus of a V domain and 4-6 residues from the N-terminus of a CL/CH1 domain. DVD binding proteins were generated using N-terminal 5-6 amino acid residues, or 11-12 amino acid residues, of CL or CH1 as a linker in the light chain and heavy chains, respectively. The N-terminal residues of CL or CH1 domains, particularly the first 5-6 amino acid residues, can adopt a loop conformation without strong secondary structures, and therefore can act as flexible linkers between the two variable domains. The N-terminal residues of CL or CH1 domains are natural extension of the variable domains, as they are part of the Ig sequences, and therefore their use minimizes to a large extent any immunogenicity potentially arising from the linkers and junctions.

In a further embodiment, of any of the heavy chain, light chain, two chain, or four chain embodiments, includes at least one linker comprising AKTTPKLEEGEFSEAR (SEQ ID NO: 1); AKTTPKLEEGEFSEARV (SEQ ID NO: 2); AKTTPKLGG (SEQ ID NO: 3); SAKTTPKLGG (SEQ ID NO: 4); SAKTTP (SEQ ID NO: 5); RADAAP (SEQ ID NO: 6); RADAAPTVS (SEQ ID NO: 7); RADAAAAGGPGS (SEQ ID NO: 8); RADAAAA(G₄S)₄ (SEQ ID NO: 9); SAKTTPKLEEGEFSEARV (SEQ ID NO: 10); ADAAP (SEQ ID NO: 11); ADAAPTVSIFPP (SEQ ID NO: 12); TVAAP (SEQ ID NO: 13); TVAAPSVFIFPP (SEQ ID NO: 14); QPKAAP (SEQ ID NO: 15); QPKAAPSVTLFPP (SEQ ID NO: 16); AKTTPP (SEQ ID NO: 17); AKTTPPSVTPLAP (SEQ ID NO: 18); AKTTAP (SEQ ID NO: 19); AKTTAPSVYPLAP (SEQ ID NO: 20); ASTKGP (SEQ ID NO: 21); ASTKGPSVFPLAP (SEQ ID NO: 22), GGGGSGGGGSGGGGS (SEQ ID NO: 23); GENKVEYAPALMALS (SEQ ID NO: 24); GPAKELTPLKEAKVS (SEQ ID NO: 25); or GHEAAAVMQVQYPAS (SEQ ID NO: 26); TVAAPSVFIFPPTVAAPSVFIFPP (SEQ ID NO: 27); ASTKGPSVFPLAPASTKGPSVFPLAP (SEQ ID NO: 28); or G/S based sequences (e.g., G4S repeats; SEQ ID NO: 29). In an embodiment, X2 is an Fc region. In another embodiment, X2 is a variant Fc region.

Other linker sequences may include any sequence of any length of a CL/CH1 domain but not all residues of a CL/CH1 domain; for example the first 5-12 amino acid residues of a CL/CH1 domain; the light chain linkers can be from C_{K} or Cλ; and the heavy chain linkers can be derived from CH1 of any isotype, including Cγ1, Cγ2, Cγ3, Cγ4, Cα1, Cα2, Cδ, Cε, and Cµ. Linker sequences may also be derived from other proteins such as Ig-like proteins (e.g., TCR, FcR, KIR); G/S based sequences (e.g., G4S repeats; SEQ ID NO: 29); hinge region-derived sequences; and other natural sequences from other proteins.

In an embodiment, a constant domain is linked to the two linked variable domains using recombinant DNA techniques. In an embodiment, a sequence comprising linked heavy chain variable domains is linked to a heavy chain constant domain and a sequence comprising linked light chain variable domains is linked to a light chain constant domain. In an embodiment, the constant domains are human heavy chain constant domains and human light chain constant domains respectively. In an embodiment, the DVD heavy chain is further linked to an Fc region. The Fc region may be a native sequence Fc region or a variant Fc region. In another embodiment, the Fc region is a human Fc region. In another embodiment, the Fc region includes Fc region from IgG1, IgG2, IgG3, IgG4, IgA, IgM, IgE, or IgD.

In another embodiment, two heavy chain DVD polypeptides and two light chain DVD polypeptides are combined to form a DVD binding protein. Table 1 lists amino acid sequences of VH and VL regions of exemplary antibodies useful for treating disease. In an embodiment, a DVD comprising at least two of the VH and/or VL regions listed in Table 1, in any orientation, is provided. In some embodiments, VD1 and VD2 are independently chosen. Therefore, in some embodiments, VD1 and VD2 comprise the same SEQ ID NO and, in other embodiments, VD1 and VD2 comprise different SEQ ID NOS. The VH and VL domain sequences provided below comprise complementarity determining regions (CDRs) and framework sequences that are either known in the art or readily discernible using methods known in the art. In some embodiments, one or more of these CDRs and/or framework sequences are replaced, without loss of function, by other CDRs and/or framework sequences from binding proteins that are known in the art to bind to the same antigen.

**Table 1: List of Amino Acid Sequences of VH and VL Regions of Antibodies for Generating Binding Proteins, Including Multivalent Binding Proteins (CDR sequences in bold)**

| **SEQ ID No.** | **ABT Unique ID** | | **Sequence** |
|---|---|---|---|
| | | **Protein region** | 1234567890123456789012345678901234567890 |
| 30 | AB017VH | VH-TNF (Seq 1) | |
| 31 | AB017VL | VL-TNF (Seq 1) | DIQMTQSPSSLSASVGDRVTITCRASQGIRNYLAWYQQKP GKAPKLLIYAASTLQSGVPSRFSGSGSGTDFTLTISSLQP EDVATYYCQRYNRAPYTFGQGTKVEIKR |
| 32 | AB213VH | VH-TNF (Seq 2) | QVQLKESGPGLVAPSQSLSITCTVSGFSLTDYGVNWVRQP PGKGLEWLGMIWGDGSTDYDSTLKSRLSISKDNSKSQIFL KMNSLQTDDTARYYCAREWHHGPVAYWGQGTLVTVSA |
| 33 | AB213VL | VL-TNF (Seq 2) | DIVMTQSHKFMSTTVGDRVSITCKASQAVSSAVAWYQQKP GQSPKLLIYWASTRHTGVPDRFTGSGSVTDFTLTIHNLQA EDLALYYCQQHYSTPFTFGSGTKLEIKR |
| 34 | AB214VH | VH-TNF (Seq 3) | EVKLEESGGGLVQPGGSMKLSCVASGFIFSNHWMNWVRQS PEKGLEWVAEIRSKSINSATHYAESVKGRFTISRDDSKSA VYLQMTDLRTEDTGVYYCSRNYYGSTYDYWGQGTTLTVSS |
| 35 | AB214VL | VL-TNF (Seq 3) | DILLTQSPAILSVSPGERVSFSCRASQFVGSSIHWYQQRT NGSPRLLIKYASESMSGIPSRFSGSGSGTDFTLSINTVES EDIADYYCQESHSWPFTFGSGTNLEVKR |
| 36 | AB215VH | VH-TNF (Seq 4) | QVQLVESGGGVVQPGRSLRLSCAASGFTFSSYAMHWVRQA PGNGLEWVAFMSYDGSNKYAKDSVKGRFTISRDNSKNTLY LQMNSLRAEDTAVYYCARDRGIAAGGNYYYYGMDVWGQGT TVTVSS |
| 37 | AB215VL | VL-TNF (Seq 4) | EIVLTQSPATLSLSPGERATLSCRASQSVYSYLAWYQQKP GQAPRLLIYDASNRATGIPARFSGSGSGTDFTLTISSLEP EDFAVYYCQQRSNWPPFTFGPGTKVDIKR |
| 38 | AB217VH | VH-TNF (Seq 5) | QVQLVESGGGVVQPGRSLRLSCAASGFTFSSYDMHWVRQA PGKGLEWVAVIWSDGSIKYYADSVKGRFTISRDNSKNTLY LQMNSLRAEDTAVYYCAREVESAMGGFYYNGMDVWGQGTT VTVSS |
| 39 | AB217VL | VL-TNF (Seq 5) | DIQMTQSPSSLSASVGDRVTITCRASQGIRIDLGWYQQKP GKAPKRLIYAASTLQSGVPSRFSGSGSGTEFIFTISSLQP EDFASYYCLQHKSYPLTFGGGTKVEIKR |
| 40 | AB218VH | VH-TNF (Seq 6) | EVQLVESGGGLIQPGGSLRLSCAASGFTVSRNYMSWVRQA PGKGLEWVSVIYSGDRTYYADSVKGRFTISRDNSKNTLYL QMNSLRAEDTAVYYCARGEGGFDYWGQGTLVTVSS |
| 41 | AB218VL | VL-TNF (Seq 6) | EIVMTQS PATLSVSPGERATLSCRASQSVSSNLAWYQQKP GQAPRLLIHGASIRATGLPARFSGSGSGTEFTLTISSLQS EDFAVYYCQQYNYWWTFGQGTKVEIKR |
| 42 | AB216VH | VH-LPA | QVQLQQSGAELVRPGTSVKVSCKASGYGFINYLIEWIKQR PGQGLEWIGLINPGSDYTNYNENFKGKATLTADKSSSTAY MHLSSLTSEDSAVYFCARRFGYYGSGNYFDYWGQGTTLTV SS |
| 43 | AB216VL | VL-LPA | DVVMTQTPLSLPVSLGDQASISCTSGQSLVHINGNTYLHW YLQKPGQSPKLLIYKVSNLFSGVPDRFSGSGSGTDFTLKI SRVEAEDLGVYFCSQSTHFPFTFGTGTKLEIKR |
| 44 | AB020VH | VH-NGF | |
| 45 | AB020VL | VL-NGF | DIQMTQSPSSLSASVGDRVTITCRASQSISNNLNWYQQKP GKAPKLLIYYTSRFHSGVPSRFSGSGSGTDFTFTISSLQP EDIATYYCQQEHTLPYTFGQGTKLEIKR |
| 46 | AB048VH | VH-PGE2 | EVQLVQSGAEVKKPGASVKVSCKASGYTFTKYWLGWVRQA PGQGLEWMGDIYPGYDYTHYNEKFKDRVTLTTDTSTSTAY MELRSLRSDDTAVYYCARSDGSSTYWGQGTLVTVSS |
| 47 | AB048VL | VL-PGE2 | DVLMTQTPLSLPVTPGEPASISCTSSQNIVHSNGNTYLEW YLQKPGQSPQLLIYKVSNRFSGVPDRFSGSGSGTDFTLKI SRVEAEDVGVYYCFQVSHVPYTFGGGTKVEIKR |
| 48 | AB022VH | VH-SOST | EVQLQQSGPELVTPGASVKISCKASGYTFTDHYMSWVKQS HGKSLEWIGDINPYSGETTYNQKFKGTATLTVDKSSSIAY MEIRGLTSEDSAVYYCARDDYDASPFAYWGQGTLVTVSA |
| 49 | AB022VL | VL-SOST | DVQMIQSPSSLSASLGDIVTMTCQASQGTSINLNWFQQKP GKAPKLLIYGSSNLEDGVPSRFSGSRYGTDFTLTISSLED EDLATYFCLQHSYLPYTFGGGTKLEIKR |

Detailed description of specific DVD binding proteins capable of binding specific targets, and methods of making the same, is provided in the Examples section below.

### D. Production of binding proteins

The binding proteins provided herein may be produced by any of a number of techniques known in the art. For example, expression from host cells, wherein expression vector(s) encoding the DVD heavy and DVD light chains is (are) transfected into a host cell by standard techniques. Although it is possible to express the DVD binding proteins provided herein in either prokaryotic or eukaryotic host cells, DVD binding proteins are expressed in eukaryotic cells, for example, mammalian host cells, because such eukaryotic cells (and in particular mammalian cells) are more likely than prokaryotic cells to assemble and secrete a properly folded and immunologically active DVD binding protein.

In an exemplary system for recombinant expression of DVD proteins, a recombinant expression vector encoding both the DVD heavy chain and the DVD light chain is introduced into dhfr- CHO cells by calcium phosphate-mediated transfection. Within the recombinant expression vector, the DVD heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the DVD heavy and light chains and intact DVD protein is recovered from the culture medium. Standard molecular biology techniques are used to prepare the recombinant expression vector, transfect the host cells, select for transformants, culture the host cells and recover the DVD protein from the culture medium. A method of synthesizing a DVD protein provided herein by culturing a host cell provided herein in a suitable culture medium until a DVD protein is synthesized is also provided. The method can further comprise isolating the DVD protein from the culture medium.

An important feature of DVD binding protein is that it can be produced and purified in a similar way as a conventional antibody. The production of DVD binding protein results in a homogeneous, single major product with desired dual-specific activity, without the need for sequence modification of the constant region or chemical modifications. Other previously described methods to generate "bi-specific", "multi-specific", and "multi-specific multivalent" full length binding proteins can lead to the intracellular or secreted production of a mixture of assembled inactive, mono-specific, multi-specific, multivalent, full length binding proteins, and multivalent full length binding proteins with a combination of different binding sites.

Surprisingly, the design of the "dual-specific multivalent full length binding proteins" provided herein leads to a dual variable domain light chain and a dual variable domain heavy chain that assemble primarily to the desired "dual-specific multivalent full length binding proteins".

At least 50%, at least 75% and at least 90% of the assembled, and expressed dual variable domain immunoglobulin molecules are the desired dual-specific tetravalent protein, and therefore possess enhanced commercial utility. Thus, a method to express a dual variable domain light chain and a dual variable domain heavy chain in a single cell leading to a single primary product of a "dual-specific tetravalent full length binding protein" is provided.

Methods of expressing a dual variable domain light chain and a dual variable domain heavy chain in a single cell leading to a "primary product" of a "dual-specific tetravalent full length binding protein", where the "primary product" is more than 50%, such as more than 75% and more than 90%, of all assembled protein, comprising a dual variable domain light chain and a dual variable domain heavy chain are provided.

### II. Uses of binding proteins

Given their ability to bind to two or more antigens the binding proteins provided herein can be used to detect the antigens (e.g., in a biological sample, such as serum or plasma), using a conventional immunoassay, such as an enzyme linked immunosorbent assays (ELISA), a radioimmunoassay (RIA), or tissue immunohistochemistry. The binding protein is directly or indirectly labeled with a detectable substance to facilitate detection of the bound or unbound antibody. Suitable detectable substances include various enzymes, prosthetic groups, fluorescent materials, luminescent materials and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin. An example of a luminescent material is luminol and examples of suitable radioactive materials include ³H, ¹⁴C, ³⁵S, ⁹⁰Y, ⁹⁹TC, ¹¹¹In, ¹²⁵I, ¹³¹I, ¹⁷⁷Lu, ¹⁶⁶Ho, and ¹⁵³Sm.

In an embodiment, the binding proteins provided herein are capable of neutralizing the activity of their antigen targets both *in vitro* and *in vivo.* Accordingly, such binding proteins can be used to inhibit antigen activity, e.g., in a cell culture containing the antigens, in human subjects or in other mammalian subjects having the antigens with which a binding protein provided herein cross-reacts. In another embodiment, a method for reducing antigen activity in a subject suffering from a disease or disorder in which the antigen activity is detrimental is provided. A binding protein provided herein can be administered to a human subject for therapeutic purposes.

The term "a disorder in which antigen activity is detrimental" is intended to include diseases and other disorders in which the presence of the antigen in a subject suffering from the disorder has been shown to be or is suspected of being either responsible for the pathophysiology of the disorder or a factor that contributes to a worsening of the disorder. Accordingly, a disorder in which antigen activity is detrimental is a disorder in which reduction of antigen activity is expected to alleviate the symptoms and/or progression of the disorder. Such disorders may be evidenced, for example, by an increase in the concentration of the antigen in a biological fluid of a subject suffering from the disorder (e.g., an increase in the concentration of antigen in serum, plasma, synovial fluid, etc., of the subject). Non-limiting examples of disorders that can be treated with the binding proteins provided herein include those disorders discussed below and in the section pertaining to pharmaceutical compositions comprising the binding proteins.

DVD binding proteins are useful as therapeutic agents to simultaneously block two different targets to enhance efficacy/safety and/or increase patient coverage.

Additionally, DVD binding proteins provided herein can be employed for tissue-specific delivery (target a tissue marker and a disease mediator for enhanced local PK thus higher efficacy and/or lower toxicity), including intracellular delivery (targeting an internalizing receptor and an intracellular molecule), delivering to inside brain (targeting transferrin receptor and a CNS disease mediator for crossing the blood-brain barrier). DVD binding protein can also serve as a carrier protein to deliver an antigen to a specific location via binding to a non-neutralizing epitope of that antigen and also to increase the half-life of the antigen. Furthermore, DVD binding protein can be designed to either be physically linked to medical devices implanted into patients or target these medical devices (see Burke et al. (2006)Advanced Drug Deliv. Rev. 58(3): 437-446; Hildebrand et al. (2006) Surface and Coatings Technol. 200(22-23): 6318-6324; Drug/ device combinations for local drug therapies and infection prophylaxis, Wu (2006) Biomaterials 27(11):2450-2467; Mediation of the cytokine network in the implantation of orthopedic devices, Marques (2005) Biodegradable Systems in Tissue Engineer. Regen. Med. 377-397). Briefly, directing appropriate types of cell to the site of medical implant may promote healing and restoring normal tissue function. Alternatively, inhibition of mediators (including but not limited to cytokines), released upon device implantation by a DVD coupled to or target to a device is also provided.

### A. Use of binding proteins in various diseases

Binding protein molecules provided herein are useful as therapeutic molecules to treat various diseases, e.g., wherein the targets that are recognized by the binding proteins are detrimental. Such binding proteins may bind one or more targets involved in a specific disease.

Without limiting the disclosure, further information on certain disease conditions is provided.

TNF-α plays a role in the pathology associated with a variety of diseases involving immune and inflammatory elements, such as autoimmune diseases, particularly those assocated with inflammation, including Crohn's disease, psoriasis (including plaque psoriasis), arthritis (including rheumatoid arthritis, psoratic arthritis, osteoarthritis, or juvenile idiopathic arthritis), multiple sclerosis, systemic lupus erythematosus, and ankylosing spondylitis. Therefore, the binding proteins herein may be used to treat these disorders. In another embodiment, the disorder is a respiratory disorder; asthma; allergic and nonallergic asthma; asthma due to infection; asthma due to infection with respiratory syncytial virus (RSV); chronic obstructive pulmonary disease (COPD); a condition involving airway inflammation; eosinophilia; fibrosis and excess mucus production; cystic fibrosis; pulmonary fibrosis; an atopic disorder; atopic dermatitis; urticaria; eczema; allergic rhinitis; allergic enterogastritis; an inflammatory and/or autoimmune condition of the skin; an inflammatory and/or autoimmune condition of gastrointestinal organs; inflammatory bowel diseases (IBD); ulcerative colitis; an inflammatory and/or autoimmune condition of the liver; liver cirrhosis; liver fibrosis; liver fibrosis caused by hepatitis B and/or C virus; scleroderma; tumors or cancers; hepatocellular carcinoma; glioblastoma; lymphoma; Hodgkin's lymphoma; a viral infection; a bacterial infection; a parasitic infection; HTLV-1 infection; suppression of expression of protective type 1 immune responses, suppression of expression of a protective type 1 immune response during vaccination, neurodegenerative diseases, neuronal regeneration, and spinal cord injury.

### 1. Human autoimmune and inflammatory response

TNF has been implicated in general autoimmune and inflammatory responses, including, for example, asthma, allergies, allergic lung disease, allergic rhinitis, atopic dermatitis, chronic obstructive pulmonary disease (COPD), fibrosis, cystic fibrosis (CF), fibrotic lung disease, idiopathic pulmonary fibrosis, liver fibrosis, lupus, hepatitis B-related liver diseases and fibrosis, sepsis, systemic lupus erythematosus (SLE), glomerulonephritis, inflammatory skin diseases, psoriasis, diabetes, insulin dependent diabetes mellitus, inflammatory bowel disease (IBD), ulcerative colitis (UC), Crohn's disease (CD), rheumatoid arthritis (RA), osteoarthritis (OA), multiple sclerosis (MS), graft-versus-host disease (GVHD), transplant rejection, ischemic heart disease (IHD), celiac disease, contact hypersensitivity, alcoholic liver disease, Behcet's disease, atherosclerotic vascular disease, occular surface inflammatory diseases, or Lyme disease.

The binding proteins provided herein can be used to treat neurological disorders. In an embodiment, the binding proteins provided herein or antigen-binding portions thereof, are used to treat neurodegenerative diseases, and conditions involving neuronal regeneration and spinal cord injury.

### 2. Asthma

Allergic asthma is characterized by the presence of eosinophilia, goblet cell metaplasia, epithelial cell alterations, airway hyperreactivity (AHR), and Th2 and Th1 cytokine expression, as well as elevated serum IgE levels. Corticosteroids are the most important anti-inflammatory treatment for asthma today, however their mechanism of action is non-specific and safety concerns exist, especially in the juvenile patient population. The development of more specific and targeted therapies is therefore warranted.

Animal models such as an OVA-induced asthma mouse model, where both inflammation and AHR can be assessed, are known in the art and may be used to determine the ability of various binding protein molecules to treat asthma. Animal models for studying asthma are disclosed in Coffman, et al. (2005) J. Exp. Med. 201(12):1875-1879; Lloyd et al. (2001) Adv. Immunol. 77: 263-295; Boyce et al. (2005) J. Exp. Med. 201 (12):1869-1873; and Snibson et al. (2005) J. Brit. Soc. Allergy Clin. Immunol. 35(2): 146-52. In addition to routine safety assessments of these target pairs specific tests for the degree of immunosuppression may be warranted and helpful in selecting the best target pairs (see Luster et al. (1994) Toxicol. 92(1-3):229-43; Descotes et al. (1992) Dev. Biol. Standard. 77:99-102; Hart et al. (2001) J. Allergy Clin. Immunol. 108(2):250-257).

### 3. Rheumatoid arthritis

Rheumatoid arthritis (RA), a systemic disease, is characterized by a chronic inflammatory reaction in the synovium of joints and is associated with degeneration of cartilage and erosion of juxta-articular bone. Many pro-inflammatory cytokines, chemokines, and growth factors are expressed in diseased joints. Whether a binding protein molecule will be useful for the treatment of rheumatoid arthritis can be assessed using pre-clinical animal RA models such as the collagen-induced arthritis mouse model. Other useful models are also well known in the art (see Brand (2005) Comp. Med. 55(2):114-22). Based on the cross-reactivity of the parental antibodies for human and mouse orthologues (e.g., reactivity for human and mouse TNF, human and mouse IL-15, etc.) validation studies in the mouse CIA model may be conducted with "matched surrogate antibody" derived binding protein molecules; briefly, a binding protein based on two (or more) mouse target specific antibodies may be matched to the extent possible to the characteristics of the parental human or humanized antibodies used for human binding protein construction (e.g., similar affinity, similar neutralization potency, similar half-life, etc.).

### 4. Systemic lupus erythematosus (SLE)

The immunopathogenic hallmark of SLE is the polyclonal B cell activation, which leads to hyperglobulinemia, autoantibody production and immune complex formation. Based on the cross-reactivity of the parental antibodies for human and mouse othologues (e.g., reactivity for human and mouse CD20, human and mouse interferon alpha, etc.) validation studies in a mouse lupus model may be conducted with "matched surrogate antibody" derived binding protein molecules. Briefly, a binding protein based two (or more) mouse target specific antibodies may be matched to the extent possible to the characteristics of the parental human or humanized antibodies used for human binding protein construction (e.g., similar affinity, similar neutralization potency, similar half-life, etc.).

### 5. Multiple sclerosis

Multiple sclerosis (MS) is a complex human autoimmune-type disease with a predominantly unknown etiology. Immunologic destruction of myelin basic protein (MBP) throughout the nervous system is the major pathology of multiple sclerosis. Of major consideration are immunological mechanisms that contribute to the development of autoimmunity. In particular, antigen expression, cytokine and leukocyte interactions, and regulatory T-cells, which help balance/modulate other T-cells such as Th1 and Th2 cells, are important areas for therapeutic target identification.

Several animal models for assessing the usefulness of the binding proteins to treat MS are known in the art (see Steinman et al. (2005) Trends Immunol. 26(11):565-71; Lublin et al. (1985) Springer Semin. Immunopathol.8(3):197-208; Genain et al. (1997) J. Mol. Med. 75(3):187-97; Tuohy et al. (1999) J. Exp. Med. 189(7):1033-42; Owens et al. (1995) Neurol. Clin. 13(1):51-73; and Hart et al. (2005) J. Immunol. 175(7):4761-8.) Based on the cross-reactivity of the parental antibodies for human and animal species othologues validation studies in the mouse EAE model may be conducted with "matched surrogate antibody" derived binding protein molecules. Briefly, a binding protein based on two (or more) mouse target specific antibodies may be matched to the extent possible to the characteristics of the parental human or humanized antibodies used for human binding protein construction (e.g., similar affinity, similar neutralization potency, similar half-life, etc.). The same concept applies to animal models in other non-rodent species, where a "matched surrogate antibody" derived binding protein would be selected for the anticipated pharmacology and possibly safety studies. In addition to routine safety assessments of these target pairs specific tests for the degree of immunosuppression may be warranted and helpful in selecting the best target pairs (see Luster et al. (1994) Toxicol. 92(1-3): 229-43; Descotes et al. (1992) Devel. Biol. Standard. 77: 99-102; Jones (2000) IDrugs 3(4):442-6).

### 6. Sepsis

Overwhelming inflammatory and immune responses are essential features of septic shock and play a central part in the pathogenesis of tissue damage, multiple organ failure, and death induced by sepsis. Cytokines have been shown to be mediators of septic shock. These cytokines have a direct toxic effect on tissues; they also activate phospholipase A2. These and other effects lead to increased concentrations of platelet-activating factor, promotion of nitric oxide synthase The efficacy of such binding proteins for treating sepsis can be assessed in preclinical animal models known in the art (see Buras et al. (2005) Nat. Rev. Drug Discov. 4(10):854-65 and Calandra et al. (2000) Nat. Med. 6(2):164-70).

### 7. Neurological disorders

### a. Neurodegenerative diseases

Neurodegenerative diseases are either chronic in which case they are usually age-dependent or acute (e.g., stroke, traumatic brain injury, spinal cord injury, etc.). They are characterized by progressive loss of neuronal functions (e.g., neuronal cell death, axon loss, neuritic dystrophy, demyelination), loss of mobility and loss of memory. These chronic neurodegenerative diseases represent a complex interaction between multiple cell types and mediators. Treatment strategies for such diseases are limited and mostly constitute either blocking inflammatory processes with non-specific anti-inflammatory agents (e.g., corticosteroids, COX inhibitors) or agents to prevent neuron loss and/or synaptic functions. These treatments fail to stop disease progression. Specific therapies targeting more than one disease mediator may provide even better therapeutic efficacy for chronic neurodegenerative diseases than observed with targeting a single disease mechanism (see Deane et al. (2003) Nature Med. 9:907-13; and Masliah et al. (2005) Neuron. 46:857).

The binding protein molecules provided herein can bind one or more targets involved in chronic neurodegenerative diseases such as Alzheimers. The efficacy of binding protein molecules can be validated in pre-clinical animal models such as the transgenic mice that over-express amyloid precursor protein or RAGE and develop Alzheimer's disease-like symptoms. In addition, binding protein molecules can be constructed and tested for efficacy in the animal models and the best therapeutic binding protein can be selected for testing in human patients. Binding protein molecules can also be employed for treatment of other neurodegenerative diseases such as Parkinson's disease.

### b. Neuronal regeneration and spinal cord injury

Despite an increase in knowledge of the pathologic mechanisms, spinal cord injury (SCI) is still a devastating condition and represents a medical indication characterized by a high medical need. Most spinal cord injuries are contusion or compression injuries and the primary injury is usually followed by secondary injury mechanisms (inflammatory mediators e.g., cytokines and chemokines) that worsen the initial injury and result in significant enlargement of the lesion area, sometimes more than 10-fold.

The efficacy of binding protein molecules can be validated in pre-clinical animal models of spinal cord injury. In addition, these binding protein molecules can be constructed and tested for efficacy in the animal models and the best therapeutic binding protein can be selected for testing in human patients. In general, antibodies do not cross the blood brain barrier (BBB) in an efficient and relevant manner. However, in certain neurologic diseases, e.g., stroke, traumatic brain injury, multiple sclerosis, etc., the BBB may be compromised and allows for increased penetration of binding proteins and antibodies into the brain. In other neurological conditions, where BBB leakage is not occurring, one may employ the targeting of endogenous transport systems, including carrier-mediated transporters such as glucose and amino acid carriers and receptor-mediated transcytosis-mediating cell structures/receptors at the vascular endothelium of the BBB, thus enabling trans-BBB transport of the binding protein. Structures at the BBB enabling such transport include but are not limited to the insulin receptor, transferrin receptor, LRP and RAGE. In addition, strategies enable the use of binding proteins also as shuttles to transport potential drugs into the CNS including low molecular weight drugs, nanoparticles and nucleic acids (Coloma et al. (2000) Pharm Res. 17(3):266-74; Boado et al. (2007) Bioconjug. Chem. 18(2):447-55).

### 8. Oncological disorders

Monoclonal antibody therapy has emerged as an important therapeutic modality for cancer (von Mehren et al. (2003) Annu. Rev. Med. 54:343-69). The use of dual-specific antibody that targets two separate tumor mediators will likely give additional benefit compared to a mono-specific therapy. In an embodiment, diseases that can be treated or diagnosed with the compositions and methods provided herein include, but are not limited to, primary and metastatic cancers, including carcinomas of breast, colon, rectum, lung, oropharynx, hypopharynx, esophagus, stomach, pancreas, liver, gallbladder and bile ducts, small intestine, urinary tract (including kidney, bladder and urothelium), female genital tract (including cervix, uterus, and ovaries as well as choriocarcinoma and gestational trophoblastic disease), male genital tract (including prostate, seminal vesicles, testes and germ cell tumors), endocrine glands (including the thyroid, adrenal, and pituitary glands), and skin, as well as hemangiomas, melanomas, sarcomas (including those arising from bone and soft tissues as well as Kaposi's sarcoma), tumors of the brain, nerves, eyes, and meninges (including astrocytomas, gliomas, glioblastomas, retinoblastomas, neuromas, neuroblastomas, Schwannomas, and meningiomas), solid tumors arising from hematopoietic malignancies such as leukemias, and lymphomas (both Hodgkin's and non-Hodgkin's lymphomas).

In an embodiment, the antibodies provided herein or antigen-binding portions thereof, are used to treat cancer or in the prevention of metastases from the tumors described herein either when used alone or in combination with radiotherapy and/or other chemotherapeutic agents.

### 9. Gene therapy

In a specific embodiment, nucleic acid sequences encoding a binding protein provided herein or another prophylactic or therapeutic agent provided herein are administered to treat, prevent, manage, or ameliorate a disorder or one or more symptoms thereof by way of gene therapy. Gene therapy refers to therapy performed by the administration to a subject of an expressed or expressible nucleic acid. In this embodiment, the nucleic acids produce their encoded antibody or prophylactic or therapeutic agent provided herein that mediates a prophylactic or therapeutic effect.

Any of the methods for gene therapy available in the art can be used in the methods provided herein. For general reviews of the methods of gene therapy, see Goldspiel et al. (1993) Clin. Pharmacy 12:488-505; Wu and Wu (1991) Biotherapy 3:87-95; Tolstoshev (1993) Ann. Rev. Pharmacol. Toxicol. 32:573-596; Mulligan (1993) Science 260:926- 932; Morgan and Anderson (1993) Ann. Rev. Biochem. 62:191-217; and May (1993) TIBTECH 11(5):155-215. Methods commonly known in the art of recombinant DNA technology which can be used are described in Ausubel et al. (eds.), Current Protocols in Molecular Biology, John Wiley &Sons, NY (1993); and Kriegler, Gene Transfer and Expression, A Laboratory Manual, Stockton Press, NY (1990). Detailed description of various methods of gene therapy are disclosed in US Patent Publication No. US20050042664.

### III. Pharmaceutical compositions

Pharmaceutical compositions comprising one or more binding proteins, either alone or in combination with prophylactic agents, therapeutic agents, and/or pharmaceutically acceptable carriers are provided. The pharmaceutical compositions comprising binding proteins provided herein are for use in, but not limited to, diagnosing, detecting, or monitoring a disorder, in preventing, treating, managing, or ameliorating a disorder or one or more symptoms thereof, and/or in research. The formulation of pharmaceutical compositions, either alone or in combination with prophylactic agents, therapeutic agents, and/or pharmaceutically acceptable carriers, are known to one skilled in the art (US Patent Publication No. 20090311253 A1).

Methods of administering a prophylactic or therapeutic agent provided herein include, but are not limited to, parenteral administration (e.g., intradermal, intramuscular, intraperitoneal, intravenous and subcutaneous), epidural administration, intratumoral administration, mucosal administration (e.g., intranasal and oral routes) and pulmonary administration (e.g., aerosolized compounds administered with an inhaler or nebulizer). The formulation of pharmaceutical compositions for specific routes of administration, and the materials and techniques necessary for the various methods of administration are available and known to one skilled in the art (US Patent Publication No. 20090311253 A1).

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. The term "dosage unit form" refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms provided herein are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of a binding protein provided herein is 0.1-20 mg/kg, for example, 1-10 mg/kg. It is to be noted that dosage values may vary with the type and severity of the condition to be alleviated. It is to be further understood that for any particular subject, specific dosage regimens may be adjusted over time according to the individual need and the professional judgment of the person administering or supervising the administration of the compositions, and that dosage ranges set forth herein are exemplary only and are not intended to limit the scope or practice of the claimed composition.

### IV. Combination therapy

A binding protein provided herein also can also be administered with one or more additional therapeutic agents useful in the treatment of various diseases, the additional agent being selected by the skilled artisan for its intended purpose. For example, the additional agent can be a therapeutic agent art-recognized as being useful to treat the disease or condition being treated by the antibody provided herein. The combination can also include more than one additional agent, e.g., two or three additional agents.

Combination therapy agents include, but are not limited to, antineoplastic agents, radiotherapy, chemotherapy such as DNA alkylating agents, cisplatin, carboplatin, anti-tubulin agents, paclitaxel, docetaxel, taxol, doxorubicin, gemcitabine, gemzar, anthracyclines, adriamycin, topoisomerase I inhibitors, topoisomerase II inhibitors, 5-fluorouracil (5-FU), leucovorin, irinotecan, receptor tyrosine kinase inhibitors (e.g., erlotinib, gefitinib), COX-2 inhibitors (e.g., celecoxib), kinase inhibitors, and siRNAs.

Combinations to treat autoimmune and inflammatory diseases are non-steroidal anti-inflammatory drug(s) also referred to as NSAIDS which include drugs like ibuprofen. Other combinations are corticosteroids including prednisolone; the well known side-effects of steroid use can be reduced or even eliminated by tapering the steroid dose required when treating patients in combination with the binding proteins provided herein. Non-limiting examples of therapeutic agents for rheumatoid arthritis with which an antibody provided herein, or antibody binding portion thereof, can be combined include the following: cytokine suppressive anti-inflammatory drug(s) (CSAIDs); antibodies to or antagonists of other human cytokines or growth factors, for example, TNF, LT, IL-1, IL-2, IL-3, IL-4, IL-5, IL-6, IL-7, IL-8, IL-15, IL-16, IL-18, IL-21, IL-23, interferons, EMAP-II, GM-CSF, FGF, and PDGF. Binding proteins provided herein, or antigen binding portions thereof, can be combined with antibodies to cell surface molecules such as CD2, CD3, CD4, CD8, CD25, CD28, CD30, CD40, CD45, CD69, CD80 (B7.1), CD86 (B7.2), CD90, CTLA or their ligands including CD154 (gp39 or CD40L).

Combinations of therapeutic agents may interfere at different points in the autoimmune and subsequent inflammatory cascade;. Examples include a binding protein disclosed herein and a TNF antagonist like a chimeric, humanized or human TNF antibody, Adalimumab, (PCT Publication No. WO 97/29131), CA2 (Remicade™), CDP 571, a soluble p55 or p75 TNF receptor, or derivative thereof (p75TNFR1gG (Enbrel™) or p55TNFR1gG (Lenercept)), a TNFα converting enzyme (TACE) inhibitor; or an IL-1 inhibitor (an Interleukin-1-converting enzyme inhibitor, IL-1RA, etc.). Other combinations include a binding protein disclosed herein and Interleukin 11. Yet another combination include key players of the autoimmune response which may act parallel to, dependent on or in concert with IL-12 function; especially relevant are IL-18 antagonists including an IL-18 antibody, a soluble IL-18 receptor, or an IL-18 binding protein. It has been shown that IL-12 and IL-18 have overlapping but distinct functions and a combination of antagonists to both may be most effective. Yet another combination is a binding protein disclosed herein and a non-depleting anti-CD4 inhibitor. Yet other combinations include a binding protein disclosed herein and an antagonist of the co-stimulatory pathway CD80 (B7.1) or CD86 (B7.2) including an antibody, a soluble receptor, or an antagonistic ligand.

The binding proteins provided herein may also be combined with an agent, such as methotrexate, 6-MP, azathioprine sulphasalazine, mesalazine, olsalazine chloroquinine/hydroxychloroquine, pencillamine, aurothiomalate (intramuscular and oral), azathioprine, cochicine, a corticosteroid (oral, inhaled and local injection), a beta-2 adrenoreceptor agonist (salbutamol, terbutaline, salmeteral), a xanthine (theophylline, aminophylline), cromoglycate, nedocromil, ketotifen, ipratropium, oxitropium, cyclosporin, FK506, rapamycin, mycophenolate mofetil, leflunomide, an NSAID, for example, ibuprofen, a corticosteroid such as prednisolone, a phosphodiesterase inhibitor, an adensosine agonist, an antithrombotic agent, a complement inhibitor, an adrenergic agent, an agent which interferes with signalling by proinflammatory cytokines such as TNF-α or IL-1 (e.g., IRAK, NIK, IKK, p38 or a MAP kinase inhibitor), an IL-1β converting enzyme inhibitor, a TNFα converting enzyme (TACE) inhibitor, a T-cell signalling inhibitor such as a kinase inhibitor, a metalloproteinase inhibitor, sulfasalazine, azathioprine, a 6-mercaptopurine, an angiotensin converting enzyme inhibitor, a soluble cytokine receptor or derivative thereof (e.g., a soluble p55 or p75 TNF receptor or the derivative p75TNFRIgG (Enbrel™) or p55TNFRIgG (Lenercept), sIL-1RI, sIL-1RII, sIL-6R), an antiinflammatory cytokine (e.g., IL-4, IL-10, IL-11, IL-13 and TGFβ), celecoxib, folic acid, hydroxychloroquine sulfate, rofecoxib, etanercept, infliximab, naproxen, valdecoxib, sulfasalazine, methylprednisolone, meloxicam, methylprednisolone acetate, gold sodium thiomalate, aspirin, triamcinolone acetonide, propoxyphene napsylate/apap, folate, nabumetone, diclofenac, piroxicam, etodolac, diclofenac sodium, oxaprozin, oxycodone hcl, hydrocodone bitartrate/apap, diclofenac sodium/misoprostol, fentanyl, anakinra, human recombinant, tramadol hcl, salsalate, sulindac, cyanocobalamin/fa/pyridoxine, acetaminophen, alendronate sodium, prednisolone, morphine sulfate, lidocaine hydrochloride, indomethacin, glucosamine sulf/chondroitin, amitriptyline hcl, sulfadiazine, oxycodone hcl/acetaminophen, olopatadine hcl, misoprostol, naproxen sodium, omeprazole, cyclophosphamide, rituximab, IL-1 TRAP, MRA, CTLA4-IG, IL-18 BP, anti-IL-18, Anti-IL15, BIRB-796, SCIO-469, VX-702, AMG-548, VX-740, Roflumilast, IC-485, CDC-801, or Mesopram. Combinations include methotrexate or leflunomide and in moderate or severe rheumatoid arthritis cases, cyclosporine.

In one embodiment, the binding protein or antigen-binding portion thereof, is administered in combination with one of the following agents for the treatment of rheumatoid arthritis: a small molecule inhibitor of KDR, a small molecule inhibitor of Tie-2; methotrexate; prednisone; celecoxib; folic acid; hydroxychloroquine sulfate; rofecoxib; etanercept; infliximab; leflunomide; naproxen; valdecoxib; sulfasalazine; methylprednisolone; ibuprofen; meloxicam; methylprednisolone acetate; gold sodium thiomalate; aspirin; azathioprine; triamcinolone acetonide; propxyphene napsylate/apap; folate; nabumetone; diclofenac; piroxicam; etodolac; diclofenac sodium; oxaprozin; oxycodone hcl; hydrocodone bitartrate/apap; diclofenac sodium/misoprostol; fentanyl; anakinra, human recombinant; tramadol hcl; salsalate; sulindac; cyanocobalamin/fa/pyridoxine; acetaminophen; alendronate sodium; prednisolone; morphine sulfate; lidocaine hydrochloride; indomethacin; glucosamine sulfate/chondroitin; cyclosporine; amitriptyline hcl; sulfadiazine; oxycodone hcl/acetaminophen; olopatadine hcl; misoprostol; naproxen sodium; omeprazole; mycophenolate mofetil; cyclophosphamide; rituximab; IL-1 TRAP; MRA; CTLA4-IG; IL-18 BP; IL-12/23; anti-IL 18; anti-IL 15; BIRB-796; SCIO-469; VX-702; AMG-548; VX-740; Roflumilast; IC-485; CDC-801; or mesopram.

Non-limiting examples of therapeutic agents for inflammatory bowel disease with which a binding protein provided herein can be combined include the following: budenoside; epidermal growth factor; a corticosteroid; cyclosporin, sulfasalazine; aminosalicylates; 6-mercaptopurine; azathioprine; metronidazole; a lipoxygenase inhibitor; mesalamine; olsalazine; balsalazide; an antioxidant; a thromboxane inhibitor; an IL-1 receptor antagonist; an anti-IL-1β mAb; an anti-IL-6 mAb; a growth factor; an elastase inhibitor; a pyridinyl-imidazole compound; an antibody to or antagonist of other human cytokines or growth factors, for example, TNF, LT, IL-1, IL-2, IL-6, IL-7, IL-8, IL-15, IL-16, IL-17, IL-18, EMAP-II, GM-CSF, FGF, or PDGF. Antibodies provided herein, or antigen binding portions thereof, can be combined with an antibody to a cell surface molecule such as CD2, CD3, CD4, CD8, CD25, CD28, CD30, CD40, CD45, CD69, CD90 or their ligands. The antibodies provided herein, or antigen binding portions thereof, may also be combined with an agent, such as methotrexate, cyclosporin, FK506, rapamycin, mycophenolate mofetil, leflunomide, an NSAID, for example, ibuprofen, a corticosteroid such as prednisolone, a phosphodiesterase inhibitor, an adenosine agonist, an antithrombotic agent, a complement inhibitor, an adrenergic agent, an agent which interferes with signalling by proinflammatory cytokines such as TNFα or IL-1 (e.g., an IRAK, NIK, IKK, p38 or MAP kinase inhibitor), an IL-1β converting enzyme inhibitor, a TNFα converting enzyme inhibitor, a T-cell signalling inhibitor such as a kinase inhibitor, a metalloproteinase inhibitor, sulfasalazine, azathioprine, a 6-mercaptopurine, an angiotensin converting enzyme inhibitor, a soluble cytokine receptor or derivative thereof (e.g., a soluble p55 or p75 TNF receptor, sIL-1RI, sIL-1RII, sIL-6R) or an antiinflammatory cytokine (e.g., IL-4, IL-10, IL-11, IL-13 or TGFβ) or a bcl-2 inhibitor.

Examples of therapeutic agents for Crohn's disease in which a binding protein can be combined include the following: a TNF antagonist, for example, an anti-TNF antibody, Adalimumab (PCT Publication No. WO 97/29131; HUMIRA), CA2 (REMICADE), CDP 571, a TNFR-Ig construct, (p75TNFRIgG (ENBREL) or a p55TNFRIgG (LENERCEPT)) inhibitor or a PDE4 inhibitor. Antibodies provided herein, or antigen binding portions thereof, can be combined with a corticosteroid, for example, budenoside and dexamethasone. Binding proteins provided herein or antigen binding portions thereof, may also be combined with an agent such as sulfasalazine, 5-aminosalicylic acid and olsalazine, or an agent that interferes with the synthesis or action of a proinflammatory cytokine such as IL-1, for example, an IL-1β converting enzyme inhibitor or IL-1 ra. Antibodies provided herein or antigen binding portion thereof may also be used with a T cell signaling inhibitor, for example, a tyrosine kinase inhibitor or an 6-mercaptopurine. Binding proteins provided herein, or antigen binding portions thereof, can be combined with IL-11. Binding proteins provided herein, or antigen binding portions thereof, can be combined with mesalamine, prednisone, azathioprine, mercaptopurine, infliximab, methylprednisolone sodium succinate, diphenoxylate/atrop sulfate, loperamide hydrochloride, methotrexate, omeprazole, folate, ciprofloxacin/dextrose-water, hydrocodone bitartrate/apap, tetracycline hydrochloride, fluocinonide, metronidazole, thimerosal/boric acid, cholestyramine/sucrose, ciprofloxacin hydrochloride, hyoscyamine sulfate, meperidine hydrochloride, midazolam hydrochloride, oxycodone hcl/acetaminophen, promethazine hydrochloride, sodium phosphate, sulfamethoxazole/trimethoprim, celecoxib, polycarbophil, propoxyphene napsylate, hydrocortisone, multivitamins, balsalazide disodium, codeine phosphate/apap, colesevelam hcl, cyanocobalamin, folic acid, levofloxacin, methylprednisolone, natalizumab or interferon-gamma

Non-limiting examples of therapeutic agents for multiple sclerosis with which binding proteins provided herein can be combined include the following: a corticosteroid; prednisolone; methylprednisolone; azathioprine; cyclophosphamide; cyclosporine; methotrexate; 4-aminopyridine; tizanidine; interferon-β1a (AVONEX; Biogen); interferon-β1b (BETASERON; Chiron/Berlex); interferon α-n3) (Interferon Sciences/Fujimoto), interferon-α (Alfa Wassermann/J&J), interferon β1A-IF (Serono/Inhale Therapeutics), Peginterferon α 2b (Enzon/Schering-Plough), Copolymer 1 (Cop-1; COPAXONE; Teva Pharmaceutical Industries, Inc.); hyperbaric oxygen; intravenous immunoglobulin; clabribine; an antibody to or antagonist of other human cytokines or growth factors and their receptors, for example, TNF, LT, IL-1, IL-2, IL-6, IL-7, IL-8, IL-23, IL-15, IL-16, IL-18, EMAP-II, GM-CSF, FGF, or PDGF. Binding proteins provided herein can be combined with an antibody to a cell surface molecule such as CD2, CD3, CD4, CD8, CD19, CD20, CD25, CD28, CD30, CD40, CD45, CD69, CD80, CD86, CD90 or their ligands. Binding proteins provided herein, may also be combined with an agent, such as methotrexate, cyclosporine, FK506, rapamycin, mycophenolate mofetil, leflunomide, an NSAID, for example, ibuprofen, a corticosteroid such as prednisolone, a phosphodiesterase inhibitor,an adensosine agonist,an antithrombotic agent, a complement inhibitor, an adrenergic agent, an agent which interferes with signalling by a proinflammatory cytokine such as TNFα or IL-1 (e.g.,IRAK, NIK, IKK, p38 or a MAP kinase inhibitor), an IL-1β converting enzyme inhibitor, a TACE inhibitor, a T-cell signaling inhibitor such as a kinase inhibitor, a metalloproteinase inhibitor, sulfasalazine, azathioprine, a 6-mercaptopurine, an angiotensin converting enzyme inhibitor, a soluble cytokine receptor or derivatives thereof (e.g., a soluble p55 or p75 TNF receptor, sIL-1RI, sIL-1RII, sIL-6R), an antiinflammatory cytokine (e.g., IL-4, IL-10, IL-13 or TNFβ) or a bcl-2 inhibitor.

Examples of therapeutic agents for multiple sclerosis in which binding proteins provided herein can be combined include interferon-β, for example, IFNβ1a and IFNβ1b; copaxone, corticosteroids, caspase inhibitors, for example inhibitors of caspase-1, IL-1 inhibitors, TNF inhibitors, and antibodies to CD40 ligand and CD80.

Non-limiting examples of therapeutic agents for asthma with which binding proteins provided herein can be combined include the following: albuterol, salmeterol/fluticasone, montelukast sodium, fluticasone propionate, budesonide, prednisone, salmeterol xinafoate, levalbuterol hcl, albuterol sulfate/ipratropium, prednisolone sodium phosphate, triamcinolone acetonide, beclomethasone dipropionate, ipratropium bromide, azithromycin, pirbuterol acetate, prednisolone, theophylline anhydrous, methylprednisolone sodium succinate, clarithromycin, zafirlukast, formoterol fumarate, influenza virus vaccine, methylprednisolone, amoxicillin trihydrate, flunisolide, allergy injection, cromolyn sodium, fexofenadine hydrochloride, flunisolide/menthol, amoxicillin/clavulanate, levofloxacin, inhaler assist device, guaifenesin, dexamethasone sodium phosphate, moxifloxacin hcl, doxycycline hyclate, guaifenesin/d-methorphan, p-ephedrine/cod/chlorphenir, gatifloxacin, cetirizine hydrochloride, mometasone furoate, salmeterol xinafoate, benzonatate, cephalexin, pe/hydrocodone/chlorphenir, cetirizine hcl/pseudoephed, phenylephrine/cod/promethazine, codeine/promethazine, cefprozil, dexamethasone, guaifenesin/pseudoephedrine, chlorpheniramine/hydrocodone, nedocromil sodium, terbutaline sulfate, epinephrine, methylprednisolone, metaproterenol sulfate.

Non-limiting examples of therapeutic agents for COPD with which binding proteins provided herein can be combined include the following: albuterol sulfate/ipratropium, ipratropium bromide, salmeterol/fluticasone, albuterol, salmeterol xinafoate, fluticasone propionate, prednisone, theophylline anhydrous, methylprednisolone sodium succinate, montelukast sodium, budesonide, formoterol fumarate, triamcinolone acetonide, levofloxacin, guaifenesin, azithromycin, beclomethasone dipropionate, levalbuterol hcl, flunisolide, ceftriaxone sodium, amoxicillin trihydrate, gatifloxacin, zafirlukast, amoxicillin/clavulanate, flunisolide/menthol, chlorpheniramine/hydrocodone, metaproterenol sulfate, methylprednisolone, mometasone furoate, p-ephedrine/cod/chlorphenir, pirbuterol acetate, p-ephedrine/loratadine, terbutaline sulfate, tiotropium bromide, (R,R)-formoterol, TgAAT, Cilomilast, Roflumilast.

Non-limiting examples of therapeutic agents for psoriasis with which binding proteins provided herein can be combined include the following: small molecule inhibitor of KDR, small molecule inhibitor of Tie-2, calcipotriene, clobetasol propionate, triamcinolone acetonide, halobetasol propionate, tazarotene, methotrexate, fluocinonide, betamethasone diprop augmented, fluocinolone acetonide, acitretin, tar shampoo, betamethasone valerate, mometasone furoate, ketoconazole, pramoxine/fluocinolone, hydrocortisone valerate, flurandrenolide, urea, betamethasone, clobetasol propionate/emoll, fluticasone propionate, azithromycin, hydrocortisone, moisturizing formula, folic acid, desonide, pimecrolimus, coal tar, diflorasone diacetate, etanercept folate, lactic acid, methoxsalen, hc/bismuth subgal/znox/resor, methylprednisolone acetate, prednisone, sunscreen, halcinonide, salicylic acid, anthralin, clocortolone pivalate, coal extract, coal tar/salicylic acid, coal tar/salicylic acid/sulfur, desoximetasone, diazepam, emollient, fluocinonide/emollient, mineral oil/castor oil/na lact, mineral oil/peanut oil, petroleum/isopropyl myristate, psoralen, salicylic acid, soap/tribromsalan, thimerosal/boric acid, celecoxib, infliximab, cyclosporine, alefacept, efalizumab, tacrolimus, pimecrolimus, PUVA, UVB, sulfasalazine.

Examples of therapeutic agents for SLE (Lupus) in which binding proteins provided herein can be combined include the following: NSAIDS, for example, diclofenac, naproxen, ibuprofen, piroxicam, indomethacin; COX2 inhibitors, for example, Celecoxib, rofecoxib, valdecoxib; anti-malarials, for example, hydroxychloroquine; Steroids, for example, prednisone, prednisolone, budenoside, dexamethasone; Cytotoxics, for example, azathioprine, cyclophosphamide, mycophenolate mofetil, methotrexate; inhibitors of PDE4 or purine synthesis inhibitor, for example Cellcept. Binding proteins provided herein may also be combined with agents such as sulfasalazine, 5-aminosalicylic acid, olsalazine, Imuran and agents which interfere with synthesis, production or action of proinflammatory cytokines such as IL-1, for example, caspase inhibitors like IL-1β converting enzyme inhibitors and IL-1 ra. Binding proteins provided herein may also be used with T cell signaling inhibitors, for example, tyrosine kinase inhibitors; or molecules that target T cell activation molecules, for example, CTLA-4-IgG or anti-B7 family antibodies, anti-PD-1 family antibodies. Binding proteins provided herein, can be combined with IL-11 or anti-cytokine antibodies, for example, fonotolizumab (anti-IFNg antibody), or anti-receptor receptor antibodies, for example, anti-IL-6 receptor antibody and antibodies to B-cell surface molecules. Antibodies provided herein or antigen binding portion thereof may also be used with LJP 394 (abetimus), agents that deplete or inactivate B-cells, for example, Rituximab (anti-CD20 antibody), lymphostat-B (anti-BlyS antibody), TNF antagonists, for example, anti-TNF antibodies, Adalimumab (PCT Publication No. WO 97/29131; HUMIRA), CA2 (REMICADE), CDP 571, TNFR-Ig constructs, (p75TNFRIgG (ENBREL⁾ and p55TNFRIgG (LENERCEPT)) and bcl-2 inhibitors, because bcl-2 overexpression in transgenic mice has been demonstrated to cause a lupus like phenotype (see MarquinaThe pharmaceutical compositions provided herein may include a "therapeutically effective amount" or a "prophylactically effective amount" of a binding protein provided herein. A "therapeutically effective amount" refers to an amount effective, at dosages and for periods of time necessary, to achieve the desired therapeutic result. A therapeutically effective amount of the binding protein may be determined by a person skilled in the art and may vary according to factors such as the disease state, age, sex, and weight of the individual, and the ability of the binding protein to elicit a desired response in the individual. A therapeutically effective amount is also one in which any toxic or detrimental effects of the antibody, or antibody binding portion, are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

### V. Diagnostics

The disclosure herein also provides diagnostic applications including, but not limited to, diagnostic assay methods, diagnostic kits containing one or more binding proteins, and adaptation of the methods and kits for use in automated and/or semi-automated systems. The methods, kits, and adaptations provided may be employed in the detection, monitoring, and/or treatment of a disease or disorder in an individual. This is further elucidated below.

### A. Method of assay

The present disclosure also provides a method for determining the presence, amount or concentration of an analyte, or fragment thereof, in a test sample using at least one binding protein as described herein. Any suitable assay as is known in the art can be used in the method. Examples include, but are not limited to, immunoassays and/or methods employing mass spectrometry.

Immunoassays provided by the present disclosure may include sandwich immunoassays, radioimmunoassay (RIA), enzyme immunoassay (EIA), enzyme-linked immunosorbent assay (ELISA), competitive-inhibition immunoassays, fluorescence polarization immunoassay (FPIA), enzyme multiplied immunoassay technique (EMIT), bioluminescence resonance energy transfer (BRET), and homogenous chemiluminescent assays, among others.

A chemiluminescent microparticle immunoassay, in particular one employing the ARCHITECT® automated analyzer (Abbott Laboratories, Abbott Park, IL), is an example of an immunoassay.

Methods employing mass spectrometry are provided by the present disclosure and include, but are not limited to MALDI (matrix-assisted laser desorption/ionization) or by SELDI (surface-enhanced laser desorption/ionization).

Methods for collecting, handling, processing, and analyzing biological test samples using immunoassays and mass spectrometry would be well-known to one skilled in the art, are provided for in the practice of the present disclosure (US 2009-0311253 A1).

### B. Kit

A kit for assaying a test sample for the presence, amount or concentration of an analyte, or fragment thereof, in a test sample is also provided. The kit comprises at least one component for assaying the test sample for the analyte, or fragment thereof, and instructions for assaying the test sample for the analyte, or fragment thereof. The at least one component for assaying the test sample for the analyte, or fragment thereof, can include a composition comprising a binding protein, as disclosed herein, and/or an anti-analyte binding protein (or a fragment, a variant, or a fragment of a variant thereof), which is optionally immobilized on a solid phase.

Optionally, the kit may comprise a calibrator or control, which may comprise isolated or purified analyte. The kit can comprise at least one component for assaying the test sample for an analyte by immunoassay and/or mass spectrometry. The kit components, including the analyte, binding protein, and/or anti-analyte binding protein, or fragments thereof, may be optionally labeled using any art-known detectable label. The materials and methods for the creation provided for in the practice of the present disclosure would be known to one skilled in the art (US 2009-0311253 A1).

### C. Adaptation of kit and method

The kit (or components thereof), as well as the method of determining the presence, amount or concentration of an analyte in a test sample by an assay, such as an immunoassay as described herein, can be adapted for use in a variety of automated and semi-automated systems (including those wherein the solid phase comprises a microparticle), as described, for example, in US Patent Nos. 5,089,424 and 5,006,309, and as commercially marketed, for example, by Abbott Laboratories (Abbott Park, IL) as ARCHITECT®.

Other platforms available from Abbott Laboratories include, but are not limited to, AxSYM®, IMx® (see, for example, US Patent No. 5,294,404, PRISM®, EIA (bead), and Quantum™ II, as well as other platforms. Additionally, the assays, kits and kit components can be employed in other formats, for example, on electrochemical or other hand-held or point-of-care assay systems. The present disclosure is, for example, applicable to the commercial Abbott Point of Care (i-STAT®, Abbott Laboratories) electrochemical immunoassay system that performs sandwich immunoassays. Immunosensors and their methods of manufacture and operation in single-use test devices are described, for example in, US Patent No. 5,063,081, 7,419,821, and 7,682,833; and US Publication Nos. 20040018577, 20060160164 and US 20090311253.

It will be readily apparent to those skilled in the art that other suitable modifications and adaptations of the methods described herein are obvious and may be made using suitable equivalents without departing from the scope of the embodiments disclosed herein. Having now described certain embodiments in detail, the same will be more clearly understood by reference to the following examples, which are included for purposes of illustration only and are not intended to be limiting.

### EXAMPLES

### Example 1: Generation and Characterization of Dual Variable Domain (DVD) Binding Proteins

Four-chain dual variable domain (DVD) binding proteins using parent antibodies with known amino acid sequences were generated by synthesizing polynucleotide fragments encoding DVD binding protein variable heavy and DVD binding protein variable light chain sequences and cloning the fragments into a pHybC-D2 vector according to art known methods. The DVD binding protein constructs were cloned into and expressed in 293 cells and purified according to art known methods. DVD VH and VL chains for the DVD binding proteins are provided below. The binding proteins were designed such that two different variable domains from two different parental monoclonal antibodies were linked via a linker by recombinant DNA techniques. In the heavy chain embodiments (designated below with a DVD number ending in the letter H), the outer domain (or VD1 domain) included a VL and the inner domain (or VD2 domain) included a VH. In the light chain embodiments (designated below with a DVD number ending in the letter L), the outer domain (or VD1 domain) included a VH and the inner domain (or VD2 domain) included a VL. Thus, the heavy and light chain domains at the VD1 position were crossed over or switched.

The linkers used in the construction of the DVDs of Table 4B are provided in Table 4A.

**Table 4A**

| Linker Name | Sequence | SEQ ID NO: |
|---|---|---|
| HG-short | ASTKGP | 21 |
| LK-short | TVAAP | 13 |

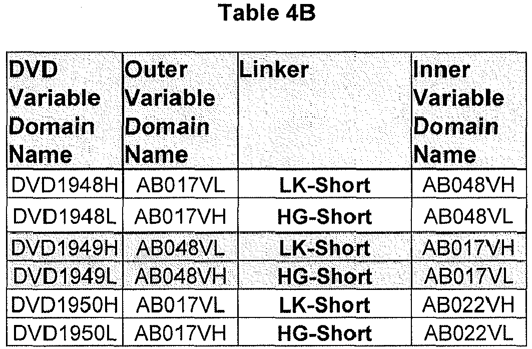

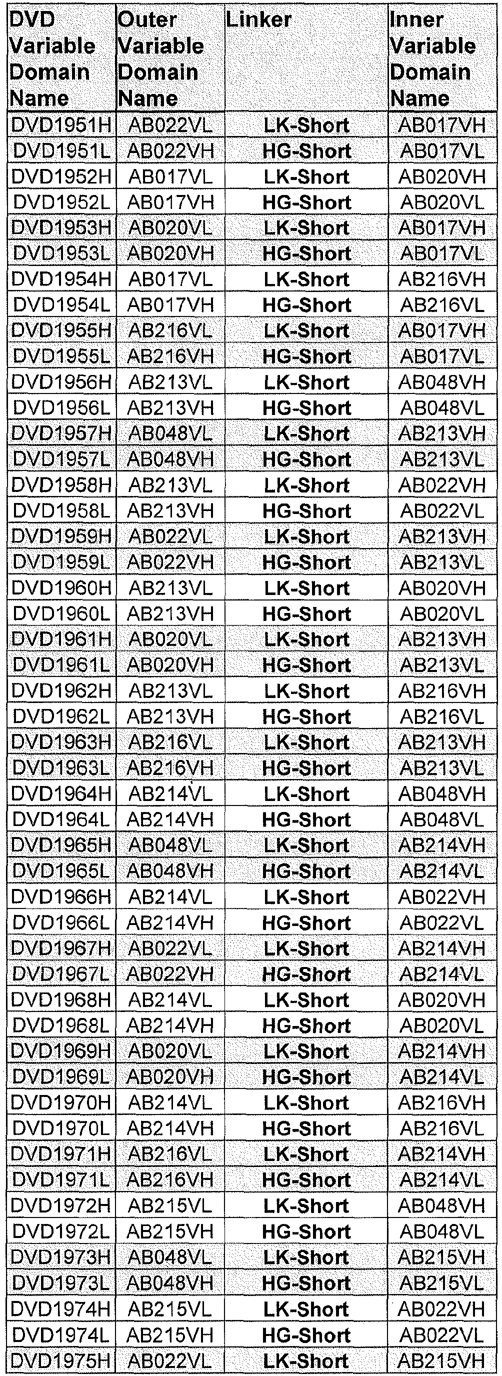

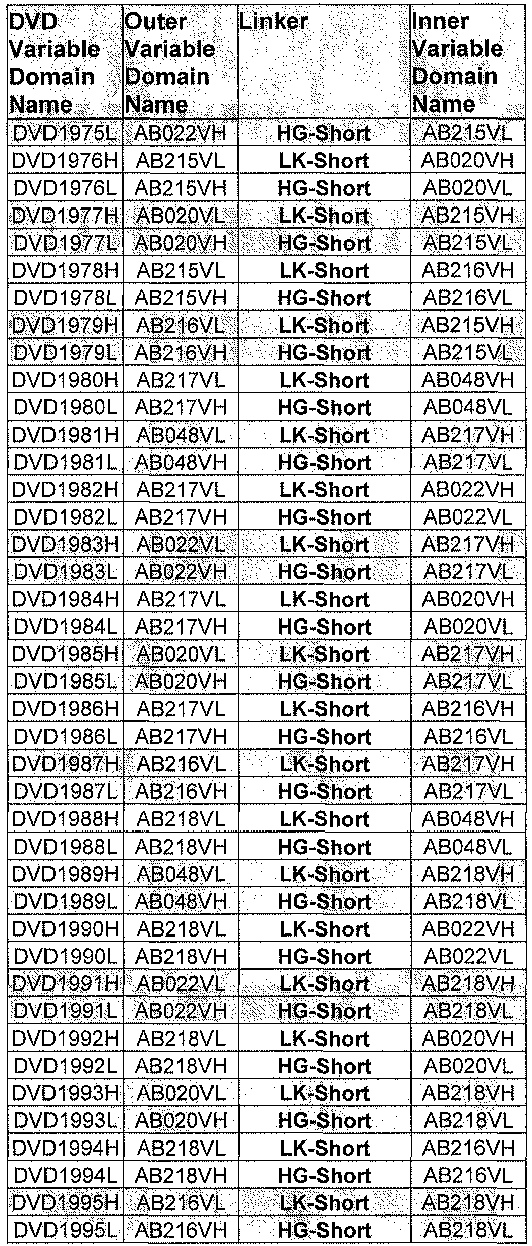

Table 5 contains the yield data for parent antibodies and DVD-Ig proteins expressed as milligrams per liter in 293 cells.

**Table 5: Transient Expression in Yields of Parent Antibodies and DVD-Ig Proteins in 293 Cells**

| **Parent Antibody or DVD-Ig ID** | **N-terminal Variable Domain (VD)** | **C-terminal Variable Domain (VD)** | **Expression yield (mg/L)** |
|---|---|---|---|
| DVD1948 | TNF (Seq 1) | PGE2 | 12.94 |
| DVD1949 | PGE2 | TNF (Seq 1) | 19.2 |
| DVD1950 | TNF (Seq 1) | SOST | 15.4 |
| DVD1951 | SOST | TNF (Seq 1) | 12.18 |
| DVD1952 | TNF (Seq 1) | NGF | 19.54 |
| DVD1953 | NGF | TNF (Seq 1) | 0 |
| DVD1954 | TNF (Seq 1) | LPA | 8.7 |
| DVD1955 | LPA | TNF (Seq 1) | 0.94 |
| DVD1956 | TNF (Seq 2) | PGE2 | 0 |
| DVD1957 | PGE2 | TNF (Seq 2) | 3.32 |
| DVD1958 | TNF (Seq 2) | SOST | 1.68 |
| DVD1959 | SOST | TNF (Seq 2) | 13.86 |
| DVD1960 | TNF (Seq 2) | NGF | 0.346 |
| DVD1961 | NGF | TNF (Seq 2) | 1.66 |
| DVD1962 | TNF (Seq 2) | LPA | 0 |
| DVD1963 | LPA | TNF (Seq 2) | 1.56 |
| DVD1980 | TNF (Seq 5) | PGE2 | 12.34 |
| DVD1981 | PGE2 | TNF (Seq 5) | 9.38 |
| DVD1982 | TNF (Seq 5) | SOST | 28.2 |
| DVD1983 | SOST | TNF (Seq 5) | 29.6 |
| DVD1984 | TNF (Seq 5) | NGF | 1.59 |
| DVD1985 | NGF | TNF (Seq 5) | 0.92 |
| DVD1986 | TNF (Seq 5) | LPA | 6.52 |
| DVD1987 | LPA | TNF (Seq 5) | 2.02 |
| DVD1988 | TNF (Seq 6) | PGE2 | 6.3 |
| DVD1989 | PGE2 | TNF (Seq 6) | 35.06 |
| DVD1990 | TNF (Seq 6) | SOST | 62.84 |
| DVD1991 | SOST | TNF (Seq 6) | 55.8 |
| DVD1992 | TNF (Seq 6) | NGF | 0 |
| DVD1993 | NGF | TNF (Seq 6) | 5.46 |
| DVD1994 | TNF (Seq 6) | LPA | 7.02 |
| DVD1995 | LPA | TNF (Seq 6) | 7.6 |

All DVD-Ig proteins expressed well in 293 cells. DVD-Ig proteins could be easily purified over a protein A column. In most cases, >5 mg/L purified DVD-Ig protein could be obtained easily from supernatants of 293 cells.

### Example 2: Assays Used To Determine the Functional Activity Of Parent Antibodies And DVD-Ig Proteins

### Example 2.1: Affinity Determination Using BIACORE Technology

**Table 6: Reagents Used in Biacore Analyses**

| **Assay** | **Antigen** | **Vendor Designation** | **Vendor** | **Catalog** # |
|---|---|---|---|---|
| | NGF | Recombinant Human β-NGF | R&D systems | 256-GF |
| | TNFα | Recombinant Human TNF-α/TNFSF1A | R&D systems | 210-TA |
| | SOST | Recombinant Human SOST | R&D systems | 1406-ST |

### BIACORE Methods:

The BIACORE assay (Biacore, Inc, Piscataway, NJ) determined the affinity of antibodies or DVD-Ig proteins with kinetic measurements of on-rate and off-rate constants. Binding of antibodies or DVD-Ig proteins to a target antigen (for example, a purified recombinant target antigen) was determined by surface plasmon resonance-based measurements with a Biacore® 1000 or 3000 instrument (Biacore® AB, Uppsala, Sweden) using running HBS-EP (10 mM HEPES [pH 7.4], 150 mM NaCl, 3 mM EDTA, and 0.005% surfactant P20) at 25°C. All chemicals were obtained from Biacore® AB (Uppsala, Sweden) or otherwise from a different source as described in the text. For example, approximately 5000 RU of goat anti-mouse IgG, (Fcγ), fragment specific polyclonal antibody (Pierce Biotechnology Inc, Rockford, IL) diluted in 10 mM sodium acetate (pH 4.5) was directly immobilized across a CM5 research grade biosensor chip using a standard amine coupling kit according to manufacturer's instructions and procedures at 25 µg/ml. Unreacted moieties on the biosensor surface were blocked with ethanolamine. Modified carboxymethyl dextran surface in flowcell 2 and 4 was used as a reaction surface. Unmodified carboxymethyl dextran without goat anti-mouse IgG in flow cell 1 and 3 was used as the reference surface. For kinetic analysis, rate equations derived from the 1:1 Langmuir binding model were fitted simultaneously to association and dissociation phases of all eight injections (using global fit analysis) with the use of Biaevaluation 4.0.1 software. Purified antibodies or DVD-Ig proteins were diluted in HEPES-buffered saline for capture across goat anti-mouse IgG specific reaction surfaces. Antibodies or DVD-Ig proteins to be captured as a ligand (25 µg/ml) were injected over reaction matrices at a flow rate of 5 µl/minute. The association and dissociation rate constants, kₒₙ (M⁻¹s⁻¹) and k_{off} (s⁻¹) were determined under a continuous flow rate of 25 µl/minute. Rate constants were derived by making kinetic binding measurements at different antigen concentrations ranging from 10 - 200 nM. The equilibrium dissociation constant (M) of the reaction between antibodies or DVD-Ig proteins and the target antigen was then calculated from the kinetic rate constants by the following formula: K_{D} = k_{off}/kₒₙ. Binding was recorded as a function of time and kinetic rate constants were calculated. In this assay, on-rates as fast as 10⁶ M⁻¹s⁻¹ and off-rates as slow as 10⁻⁶ s⁻¹ were measured.

**Table 7: BIACORE Analysis of Parental Antibodies and DVD-Ig Proteins**

| **Parent Antibody or DVD-Ig ID** | **N-terminal Variable Domain (VD)** | **C-terminal Variable Domain (VD)** | **kₒₙ (M-1s-1)** | **k_{off} (s-1)** | **k_{D} (M)** |
|---|---|---|---|---|---|
| AB017 | TNF (Seq 1) | | 1.70E+06 | 1.30E-04 | 7.40E-11 |
| AB020 | NGF | | 2.50E+06 | 6.80E-06 | 2.70E-12 |
| AB022 | SOST | | 2.10E+07 | 3.80E-04 | 1.80E-11 |
| AB213 | TNF (Seq 2) | | 3.20E+06 | 8.20E-05 | 2.50E-11 |
| AB217 | TNF (Seq 5) | | 1.70E+06 | 4.80E-05 | 2.80E-11 |
| AB218 | TNF (Seq 6) | | 1.80E+06 | 7.30E-05 | 4.10E-11 |
| AB017 | TNF (Seq 1) | | 1.70E+06 | 1.30E-04 | 7.40E-11 |
| DVD1948 | TNF (Seq 1) | PGE2 | 2.00E+06 | 9.10E-05 | 4.50E-11 |
| DVD1949 | PGE2 | TNF (Seq 1) | 7.70E+04 | 3.80E-04 | 5.00E-09 |
| AB017 | TNF (Seq 1) | | 1.70E+06 | 1.30E-04 | 7.40E-11 |
| AB022 | SOST | | 2.10E+07 | 3.80E-04 | 1.80E-11 |
| DVD1951 | SOST (seq1) | | 1.00E+07 | 2.20E-04 | 2.10E-11 |
| DVD1951 | | TNF (Seq 1) | 1.30E+05 | 1.50E-04 | 1.10E-09 |
| AB017 | TNF (Seq 1) | | 1.70E+06 | 1.30E-04 | 7.40E-11 |
| AB020 | NGF | | 2.50E+06 | 6.80E-06 | 2.70E-12 |
| DVD1952 | TNF (Seq 1) | | 1.90E+06 | 9.30E-05 | 4.80E-11 |
| DVD1952 | | NGF (Rinat) | 6.30E+05 | 9.60E-05 | 1.50E-10 |
| AB017 | TNF (Seq 1) | | 1.70E+06 | 1.30E-04 | 7.40E-11 |
| DVD1955 | LPA | TNF (Seq 1) | 5.30E+04 | 2.20E-04 | 4.10E-09 |
| AB213 | TNF (Seq 2) | | 3.20E+06 | 8.20E-05 | 2.50E-11 |
| DVD1957 | PGE2 | TNF (Seq 2) | 1.50E+05 | 1.30E-04 | 9.00E-10 |
| AB213 | TNF (Seq 2) | | 3.20E+06 | 8.20E-05 | 2.50E-11 |
| AB020 | NGF | | 2.50E+06 | 6.80E-06 | 2.70E-12 |
| DVD1961 | NGF (Rinat) | | 3.20E+06 | <1.0E-06 | <3.1E-13 |
| DVD1961 | | TNF (Seq 2) | 1.70E+05 | 9.90E-05 | 5.80E-10 |
| AB213 | TNF (Seq 2) | | 3.20E+06 | 8.20E-05 | 2.50E-11 |
| DVD1963 | LPA | TNF (Seq 2) | 1.40E+05 | 1.20E-04 | 8.40E-10 |
| AB217 | TNF (Seq 5) | | 1.70E+06 | 4.80E-05 | 2.80E-11 |
| DVD1980 | TNF (Seq 5) | PGE2 | 2.00E+06 | 6.50E-05 | 3.20E-11 |
| DVD1981 | PGE2 | TNF (Seq 5) | 3.70E+04 | 3.30E-05 | 9.00E-10 |
| AB217 | TNF (Seq 5) | | 1.70E+06 | 4.80E-05 | 2.80E-11 |
| AB020 | NGF | | 2.50E+06 | 6.80E-06 | 2.70E-12 |
| DVD 1984 | TNF (Seq 5) | | 2.50E+06 | 3.70E-05 | 1.50E-11 |
| DVD1984 | | NGF (Rinat) | 4.50E+05 | 4.30E-05 | 9.60E-11 |
| DVD1985 | NGF (Rinat) | | 3.10E+06 | 3.70E-05 | 1.20E-11 |
| DVD1985 | | TNF (Seq 5) | 4.50E+04 | 1.90E-05 | 4.30E-10 |
| AB217 | TNF (Seq 5) | | 1.70E+06 | 4.80E-05 | 2.80E-11 |
| DVD1986 | TNF (Seq 5) | LPA | 2.60E+06 | 5.50E-05 | 2.10E-11 |
| DVD1987 | LPA | TNF (Seq 5) | 3.80E+04 | 1.20E-04 | 3.20E-09 |
| AB218 | TNF (Seq 6) | | 1.80E+06 | 7.30E-05 | 4.10E-11 |
| DVD1988 | TNF (Seq 6) | PGE2 | 2.70E+06 | 5.50E-05 | 2.00E-11 |
| DVD1989 | PGE2 | TNF (Seq 6) | 4.90E+04 | <1.0E-06 | <2.0E-11 |
| AB218 | TNF (Seq 6) | | 1.80E+06 | 7.30E-05 | 4.10E-11 |
| AB022 | SOST | | 2.10E+07 | 3.80E-04 | 1.80E-11 |
| DVD1990 | TNF (Seq 6) | | 2.80E+06 | 4.50E-05 | 1.60E-11 |
| DVD1990 | | SOST (seq1) | 1.80E+06 | 5.80E-04 | 3.30E-10 |
| DVD1991 | SOST (seq1) | | 1.50E+07 | 2.50E-04 | 1.70E-11 |
| DVD1991 | | TNF (Seq 6) | 1.10E+05 | 7.10E-05 | 6.60E-10 |
| AB218 | TNF (Seq 6) | | 1.80E+06 | 7.30E-05 | 4.10E-11 |
| AB020 | NGF | | 2.50E+06 | 6.80E-06 | 2.70E-12 |
| DVD1993 | NGF (Rinat) | | 3.30E+06 | 1.50E-05 | 4.70E-12 |
| DVD1993 | | TNF (Seq 6) | 8.10E+04 | 3.50E-05 | 4.30E-10 |
| AB218 | TNF (Seq 6) | | 1.80E+06 | 7.30E-05 | 4.10E-11 |
| DVD 1994 | TNF (Seq 6) | LPA | 2.70E+06 | 5.30E-05 | 2.00E-11 |
| DVD1995 | LPA | TNF (Seq 6) | 8.20E+04 | 1.10E-04 | 1.40E-09 |

Binding of all DVD-Ig proteins characterized by Biacore technology was maintained and comparable to that of parent antibodies. All variable domains bound with similar affinity as the parent antibodies.

### Example 2.2: Neutralization of huTNFα

L929 cells were grown to a semi-confluent density and harvested using 0.05% tryspin (Gibco#25300). The cells were washed with PBS, counted and resuspended at 1 E6 cells/mL in assay media containing 4 µg/mL actinomycin D. The cells were seeded in a 96-well plate (Costar#3599) at a volume of 50 µL and 5E4 cells/well. The DVD-Ig™ protein and control IgG were diluted to a 4x concentration in assay media and serial 1:3 dilutions were prepared. HuTNFα was diluted to 400 pg/mL in assay media. An antibody or DVD-Ig protein sample (200 µL) was added to the huTNFα (200 µL) in a 1:2 dilution scheme and allowed to incubate for 0.5 hour at room temperature.

The antibody or DVD-Ig™ protein/ huTNFα solution was added to the plated cells at 100 µL for a final concentration of 100 pg/mL huTNFα and 25 nM-0.00014 nM antibody or DVD-Ig™protein, The plates were incubated for 20 hours at 37°C, 5 % CO₂. To quantitate viability, 100 µL was removed from the wells and 10 µL of WST-1 reagent (Roche cat# 11644807001) was added. Plates were incubated under assay conditions for 3.5 hours, centrifuged at 500 xg and 75 µL supernatant transferred to an ELISA plate (Costar cat#3369). The plates were read at OD 420-600 nm on a Spectromax 190 ELISA plate reader. An average EC50 from several assays is included in Table 8 for the DVD-Ig proteins containing the various TNF sequences as the parent antibodies.

**Table 8: HuTNFα Neutralization Assay With Anti-huTNFα Parent Antibody and DVD-Ig Proteins**

| **Parent Antibody or DVD-Ig ID** | **N-terminal Variable Domain (VD)** | **C-terminal Variable Domain (VD)** | **N-Terminal VD TNFa Neutralization Assay IC50 nM** | **C-Terminal VD TNFα Neutralization AssayIC50 nM** |
|---|---|---|---|---|
| AB017 | TNF (Seq 1) | | 0.021 | |
| AB213 | TNF (Seq 2) | | 0.244 | |
| AB217 | TNF (Seq 3) | | 0.3133 | |
| AB218 | TNF (Seq 4) | | 0.179 | |
| DVD1948 | TNF (Seq 1) | PGE2 | 0.003 | NA |
| DVD1949 | PGE2 | TNF (Seq 1) | NA | 17.64 |
| DVD1950 | TNF (Seq 1) | SOST | 0.042 | NA |
| DVD1951 | SOST | TNF (Seq 1) | NA | 4.527 |
| DVD1952 | TNF (Seq 1) | NGF | 0.016 | NA |
| DVD1954 | TNF (Seq 1) | LPA | 0.016 | NA |
| DVD1955 | LPA | TNF (Seq 1) | NA | 4.725 |
| DVD1957 | PGE2 | TNF (Seq 2) | NA | 3.103 |
| DVD1958 | TNF (Seq 2) | SOST | 0.019 | NA |
| DVD1959 | SOST | TNF (Seq 2) | NA | 1.518 |
| DVD1960 | TNF (Seq 2) | NGF | 0.086 | NA |
| DVD1961 | NGF | TNF (Seq 2) | NA | 4.019 |
| DVD1963 | LPA | TNF (Seq 2) | NA | 3.002 |
| DVD1980 | TNF (Seq 3) | PGE2 | 0.026 | NA |
| DVD1981 | PGE2 | TNF (Seq 3) | NA | 3.084 |
| DVD1982 | TNF (Seq 3) | SOST | 0.017 | NA |
| DVD1983 | SOST | TNF (Seq 3) | NA | 0.127 |
| DVD1984 | TNF (Seq 3) | NGF | 0.017 | NA |
| DVD1985 | NGF | TNF (Seq 3) | NA | 4.513 |
| DVD1986 | TNF (Seq 3) | LPA | 0.023 | NA |
| DVD1987 | LPA | TNF (Seq 3) | NA | 2.092 |
| DVD1990 | TNF (Seq 4) | SOST | 0.408 | NA |
| DVD1991 | SOST | TNF (Seq 4) | NA | 0.4008 |
| DVD1993 | NGF | TNF (Seq 4) | NA | 2.081 |
| DVD1994 | TNF (Seq 4) | LPA | 0.007 | NA |
| DVD1995 | LPA | TNF (Seq 4) | NA | 1.948 |

All DVD-Ig proteins containing VDs from AB017, AB213, AB217, or AB218 in either the N-terminal or C-terminal position showed neutralization in the L929 TNFα neutralization assay.

### Example 2.3: Inhibition of PGE2 in EP4 Bioassay

The ability of anti-PGE2 antibodies and anti-PGE2 containing DVD-Ig molecules to inhibit the cellular response of PGE2 was determined in a Ca++ flux assay in HEK293Gα16 cells stably transfected with human EP4 receptor. Cells were plated in black/clear poly-D-lysine plates, (Corning #3667, Corning, N.Y.) and incubated with Ca++sensitive dye (Molecular Devices) for 60 minutes. Stock PGE2 (in 200 proof ethanol) was diluted with FLIPR buffer (containing 1xHBSS (Invitrogen, Carlsbad, California), 20 mM HEPES (Invitrogen, Carlsbad, California), 0.1 % BSA (Sigma, St. Louis, Mo.) and 2.5 mM Probenecid (Sigma, St. Louis, Mo.)). Anti-PGE2 antibodies, DVD-Ig molecules or isotype matched control antibodies were also pre-diluted in FLIPR buffer. 50 µl of PGE2 or pre-incubated PGE2/antibody mixture or pre-incubated PGE2/DVD-Ig molecule mixture was added to the wells pre-plated with cells. A dose response of PGE2 was done by a serial titration of PGE2 and was determined on FLIPR1 or Tetra (Molecular Devices). EC50 was determined using GraphPad Prism 5 (GraftPad Software, La Jolla, California). For testing antibodies and DVD-Ig molecules, PGE2 at EC50 concentration was incubated with varying concentrations of test articles or isotype matched antibody (negative control) for 20 minutes, added to dye-loaded human EP4 in HEK293Gα16 cells. Ca++ flux was monitored using Tetra Molecular Devices) and data was analyzed using GraphPad Prism 5. PGE2 inhibition results are shown in Table 9.

**Table 9: PGE2 Neutralization Assay with Anti-PGE2 Parental Antibodies and DVD-Ig Proteins**

| **Parent Antibody or DVD-Ig ID** | **N-terminal Variable Domain (VD)** | **C-terminal Variable Domain (VD)** | **N-Terminal VD PGE2 Neutralization Assay IC50 nM** | **C-Terminal VD PGE2 Neutralization AssayIC50 nM** |
|---|---|---|---|---|
| AB048 | PGE2 | | 0.401 | |
| DVD 1948 | TNF (Seq 1) | PGE2 | NA | 337.5 |
| DVD1957 | PGE2 | TNF (Seq 2) | 5.151 | NA |
| DVD1980 | TNF (Seq 3) | PGE2 | NA | 13.11 |
| DVD1981 | PGE2 | TNF (Seq 3) | 9.203 | NA |
| DVD1988 | TNF (Seq 4) | PGE2 | NA | 30.21 |
| DVD1989 | PGE2 | TNF (Seq 4) | 6.832 | NA |

All DVD-Ig proteins containing VDs from AB048 in either the N-terminal or C-terminal position showed neutralization in the PGE2 neutralization assay.

### Example 2.4: Inhibition of NGF in TF-1 Cell Proliferation Bioassay

TF-1cells were cultured in RPMI 1640 (Invitrogen) +10% Fetal Bovine Serum (Hyclone) +L-glutamine (Invitrogen)+rhu GM-CSF (R&D Systems). TF-1 cells were serum starved 24 hours in RPMI 1640 + L-glutamine at 1 x 10⁵ cells per mL and incubated overnight at 37°C, 5% CO₂. The day of the experiment TF- 1 cells were plated in opaque walled 96-well plates at 2.5 x 10⁴ cells per well in a 100 µL volume + assay media (RPMI-1640 +L-glutamine + 4% FBS) The cells were stimulated by adding NGF/DVD-Ig protein or antibody to the cells. The DVD-Ig™ protein and control IgG were diluted to a 4x concentration in assay media and serial 1:5 dilutions were performed. The huNGF was diluted to 8 ng/mL in assay media. The DVD-Ig™ protein (50 µl) and huNGF (50 µL) solutions were added to the plated for a final concentration of 2 ng/mL huNGF and 25 nM - 0.000003 nM DVD-Ig™protein. The plates were incubated for 72 hours at 37°C, 5 % CO₂. To quantitate viability, the Cell Titer Glo kit (Promega cat# TB288) was used (100 µl of solution added to each well following manufacturer's instructions). The plates were read using luminescence on a Spectromax 190 ELISA plate reader.

**Table 10: NGF Inhibition Assay With Anti-NGF Parent Antibodies and DVD-Ig Proteins**

| **Parent Antibody or DVD-Ig ID** | **N-terminal Variable Domain (VD)** | **C-terminal Variable Domain (VD)** | **N-Terminal VD NGF Neutralization Assay IC50 nM** | **C-Terminal VD NGF Neutralization AssayIC50 nM** |
|---|---|---|---|---|
| AB020 | NGF | | 0.008 | |
| DVD1952 | TNF (Seq 1) | NGF | NA | 0.26 |
| DVD1960 | TNF (Seq 2) | NGF | NA | 3.117 |
| DVD1961 | NGF | TNF (Seq 2) | 0.132 | NA |
| DVD1984 | TNF (Seq 3) | NGF | NA | 0.593 |

All DVD-Igs containing VDs from AB020 in either the N-terminal or C-terminal position showed neutralization in the NGF inhbition assay.

### Example 2.5: Inhibition of Sclerostin Activity in the Wnt-1/Luciferase Double Stable HEK Clone #14

HEK 293A cells were stably transfected with TopFlash plasmid (TCF reporter plasmid, Cell Signaling catalog #21-170, lot# 26217) and infected with Wnt-1 lentivirus (Origene Cat# SC303644), resulting in clones that co-express Luciferase and Wnt-1. One double stable clone (#14) was maintained in culture medium: DMEM (Invitrogen Cat#11965-092) with 10% Qualified FBS (Invitrogen Cat#26140-079), Pen-Strep (Invitrogen Cat#15140-122), and L-glutamine (Invitrogen Cat#25030-081 2mM final), Sodium Pyruvate (Invitrogen Cat#11360-070 final 1 mM) and 5µg/ml Puromycin (Invivogen Cat#ant-pr-1) in T75 flasks until 80-90% confluent on day of assay. The assay was performed in assay medium: culture medium without puromycin. Human Sclerostin (Abbott PR-1261069 Lot#1769536 1.06mg/mL) was aliquoted into 10µl and stored frozen at -80°C. On day 1 clone #14 cells are plated at 10,000 cells per well in 50µl assay medium in black-sided, clear bottomed tissue culture treated 96 well plates (Costar #3603) and incubated at 37°C overnight (20-24 hours). The next day (day 2) the Sclerostin stock was diluted to 200nM (4X) in the assay medium. Anti-Sclerostin antibodies were diluted to 4X (typically 400nM, 200nM, and 100nM) in assay medium. Media was removed and replaced with 50µl/well of fresh assay medium. Cells were next incubated with 25µl of Sclerostin at 200nM (4X) for 1 hour. After this anti-Sclerostin antibodies (4X conc, 25µl) were added to cells and plates were incubated overnight at 37°C (20-24 hours). The final volume is 100µl. The following day (day 3), cells were washed once with 200µl of PBS (RT). Promega Luciferase Kit #E1501 was used for cell lysis and Luciferase read out. Briefly, 5X cell lysis reagent (Promega, cat #E153A) was diluted with milliQ water to 1X and 20µl was added to each well. To ensure a complete lysis, plate was rotated 500rpm for 20 min. 100µl of Luciferase assay reagent (1vial cat #E151A substrate + 10ml cat #E152A assay buffer) was added to each well. The plate was read on TopCount machine (Program: Luciferase 96, Assay 17: 1 sec/well read).

**Table 11: SOST Inhibition Assay With Anti-SOST Parent Antibodies and DVD-Ig Proteins**

| **Parent Antibody or DVD-Ig ID** | **N-terminal Variable Domain (VD)** | **C-terminal Variable Domain (VD)** | **N-Terminal VD SOST Neutralization Assay IC50 nM** | **C-Terminal VD SOST Neutralization AssayIC50 nM** |
|---|---|---|---|---|
| AB022 | SOST | | 34.77 | |
| DVD1950 | TNF (Seq 1) | SOST | NA | >75 |
| DVD1951 | SOST | TNF (Seq 1) | 31.33 | NA |
| DVD1958 | TNF (Seq 2) | SOST | NA | 14.27 |
| DVD1959 | SOST | TNF (Seq 2) | 54.21 | NA |
| DVD1982 | TNF (Seq 3) | SOST | NA | >100 |
| DVD1983 | SOST | TNF (Seq 3) | >360 | NA |
| DVD1990 | TNF (Seq 4) | SOST | NA | >250 |
| DVD1991 | SOST | TNF (Seq 4) | 36 | NA |

All DVD-Ig proteins containing VDs from AB022 in either the N-terminal or C-terminal position showed neutralization in the NGF inhbition assay.

### Example 3: Characterization Of Antibodies and DVD-Ig Proteins

The ability of purified DVD-Ig protein to inhibit a functional activity was determined, e.g., using the cytokine bioassay as described in Example 2. The binding affinities of the DVD-Ig protein to recombinant human antigen were determined using surface plasmon resonance (Biacore®) measurement as described in Example 2. The IC₅₀ values from the bioassays and the affinity of the antibodies and DVD-Ig proteins were ranked. The DVD-Ig protein that fully maintain the activity of the parent mAbs were selected as candidates for future development. The top 2-3 most favorable DVD-Ig proteins were further characterized.

### Example 3.1: Pharmacokinetic Analysis Of Humanized Antibodies or DVD-Ig Proteins

Pharmacokinetic studies are carried out in Sprague-Dawley rats and cynomolgus monkeys. Male and female rats and cynomolgus monkeys are dosed intravenously or subcutaneously with a single dose of 4mg/kg mAb or DVD-Ig protein and samples are analyzed using antigen capture ELISA, and pharmacokinetic parameters are determined by noncompartmental analysis. Briefly, ELISA plates are coated with goat anti-biotin antibody (5 mg/ml, 4°C, overnight), blocked with Superblock (Pierce), and incubated with biotinylated human antigen at 50 ng/ml in 10% Superblock TTBS at room temperature for 2 hours. Serum samples are serially diluted (0.5% serum, 10% Superblock in TTBS) and incubated on the plate for 30 minutes at room temperature. Detection is carried out with HRP-labeled goat anti human antibody and concentrations are determined with the help of standard curves using the four parameter logistic fit. Values for the pharmacokinetic parameters are determined by non-compartmental model using WinNonlin software (Pharsight Corporation, Mountain View, CA). Humanized mAbs with good pharmacokinetics profile (T1/2 is 8-13 days or better, with low clearance and excellent bioavailability 50-100%) are selected.

### Example 3.2: Physicochemical And In Vitro Stability Analysis Of Humanized Monoclonal Antibodies and DVD-Ig Proteins

### Size Exclusion Chromatography

Antibodies or DVD-Ig proteins were diluted to 2.5 mg/mL with water and 20 mL was analyzed on a Shimadzu HPLC system using a TSK gel G3000 SWXL column (Tosoh Bioscience, cat# k5539-05k). Samples were eluted from the column with 211 mM sodium sulfate, 92 mM sodium phosphate, pH 7.0, at a flow rate of 0.3 mL/minutes. The HPLC system operating conditions were as follows:
Mobile phase: 211 mM Na₂SO₄, 92 mM Na₂HPO₄*7H₂O, pH 7.0
Gradient: Isocratic
Flow rate: 0.3 mL/minute
Detector wavelength: 280 nm
Autosampler cooler temp: 4°C
Column oven temperature: Ambient
Run time: 50 minutes

Table 10 contains purity data of parent antibodies and DVD-Ig proteins expressed as percent monomer (unaggregated protein of the expected molecular weight) as determined by the above protocol.

**Table 10: Purity of Parent Antibodies and DVD-Ig Proteins as Determined by Size Exclusion Chromatography**

| **Parent Antibody or DVD-Ig ID** | **N-terminal Variable Domain (VD)** | **C-terminal Variable Domain (VD)** | **% Monomer** (**purity**) |
|---|---|---|---|
| AB017 | TNF (Seq 1) | | 97.5 |
| AB020 | NGF | | 88.2 |
| AB022 | SOST | | 93.2 |
| AB048 | PGE2 | | 100 |
| AB213 | TNF (Seq 2) | | 100 |
| AB216 | LPA | | 100 |
| AB218 | TNF (Seq 6) | | 100 |
| DVD1948 | TNF (Seq 1) | PGE2 | 92.6 |
| DVD1949 | PGE2 | TNF (Seq 1) | 98.6 |
| DVD1950 | TNF (Seq 1) | SOST | 59.6 |
| DVD1951 | SOST | TNF (Seq 1) | 82.7 |
| DVD1952 | TNF (Seq 1) | NGF | 85.5 |
| DVD1954 | TNF (Seq 1) | LPA | 74.6 |
| DVD1955 | LPA | TNF (Seq 1) | 89.9 |
| DVD1957 | PGE2 | TNF (Seq 2) | 94 |
| DVD1958 | TNF (Seq 2) | SOST | 43.5 |
| DVD1959 | SOST | TNF (Seq 2) | 34.2 |
| DVD1960 | TNF (Seq 2) | NGF | 55 |
| DVD1961 | NGF | TNF (Seq 2) | 93.8 |
| DVD1963 | LPA | TNF (Seq 2) | 89.7 |
| DVD1980 | TNF (Seq 5) | PGE2 | 96.7 |
| DVD1981 | PGE2 | TNF (Seq 5) | 95 |
| DVD1982 | TNF (Seq 5) | SOST | 76.8 |
| DVD1983 | SOST | TNF (Seq 5) | 52.4 |
| DVD1984 | TNF (Seq 5) | NGF | 98.7 |
| DVD1985 | NGF | TNF (Seq 5) | 96.4 |
| DVD1986 | TNF (Seq 5) | LPA | 96.1 |
| DVD1987 | LPA | TNF (Seq 5) | 98 |
| DVD1988 | TNF (Seq 6) | PGE2 | 97.6 |
| DVD1989 | PGE2 | TNF (Seq 6) | 98.4 |
| DVD1990 | TNF (Seq 6) | SOST | 83.7 |
| DVD1991 | SOST | TNF (Seq 6) | 82 |
| DVD1994 | TNF (Seq 6) | LPA | 88.9 |
| DVD1995 | LPA | TNF (Seq 6) | 88.8 |

DVD-Ig proteins showed an excellent SEC profile with most DVD-Ig proteins showing >90% monomer. This DVD-Ig protein profile was similar to that observed for parent antibodies.

### SDS-PAGE

Antibodies and DVD-Ig proteins are analyzed by sodium dodecyl sulfate - polyacrylamide gel electrophoresis (SDS-PAGE) under both reducing and non-reducing conditions. Adalimumab lot AFP04C is used as a control. For reducing conditions, the samples are mixed 1:1 with 2X tris glycine SDS-PAGE sample buffer (Invitrogen, cat# LC2676, lot# 1323208) with 100 mM DTT, and heated at 60°C for 30 minutes. For non-reducing conditions, the samples are mixed 1:1 with sample buffer and heated at 100°C for 5 minutes. The reduced samples (10 mg per lane) are loaded on a 12% pre-cast tris-glycine gel (Invitrogen, cat# EC6005box, lot# 6111021), and the non-reduced samples (10 mg per lane) are loaded on an 8%-16% pre-cast tris-glycine gel (Invitrogen, cat# EC6045box, lot# 6111021). SeeBlue Plus 2 (Invitrogen, cat#LC5925, lot# 1351542) is used as a molecular weight marker. The gels are run in a XCell SureLock mini cell gel box (Invitrogen, cat# E10001) and the proteins are separated by first applying a voltage of 75 to stack the samples in the gel, followed by a constant voltage of 125 until the dye front reached the bottom of the gel. The running buffer used is 1X tris glycine SDS buffer, prepared from a 10X tris glycine SDS buffer (ABC, MPS-79-080106)). The gels are stained overnight with colloidal blue stain (Invitrogen cat# 46-7015, 46-7016) and destained with Milli-Q water until the background is clear. The stained gels are then scanned using an Epson Expression scanner (model 1680, S/N DASX003641).

### Sedimentation Velocity Analysis

Antibodies or DVD-Ig proteins are loaded into the sample chamber of each of three standard two-sector carbon epon centerpieces. These centerpieces have a 1.2 cm optical path length and are built with sapphire windows. PBS is used for a reference buffer and each chamber contained 140 µL. All samples are examined simultaneously using a 4-hole (AN-60Ti) rotor in a Beckman ProteomeLab XL-I analytical ultracentrifuge (serial # PL106C01).

Run conditions are programmed and centrifuge control is performed using ProteomeLab (v5.6). The samples and rotor are allowed to thermally equilibrate for one hour prior to analysis (20.0 ± 0.1 °C). Confirmation of proper cell loading is performed at 3000 rpm and a single scan is recorded for each cell. The sedimentation velocity conditions are the following:
Sample Cell Volume: 420 mL
Reference Cell Volume: 420 mL
Temperature: 20°C
Rotor Speed: 35,000 rpm
Time: 8:00 hours
UV Wavelength: 280 nm
Radial Step Size: 0.003 cm
Data Collection: One data point per step without signal averaging.
Total Number of Scans: 100

### LC-MS molecular weight measurement of intact antibodies

Molecular weight of intact antibodies and DVD-Ig proteins are analyzed by LC-MS. Each antibody or DVD-Ig protein is diluted to approximately 1 mg/mL with water. An 1100 HPLC (Agilent) system with a protein microtrap (Michrom Bioresources, Inc, cat# 004/25109/03) is used to desalt and introduce 5 mg of the sample into an API Qstar pulsar i mass spectrometer (Applied Biosystems). A short gradient is used to elute the samples. The gradient is run with mobile phase A (0.08% FA, 0.02% TFA in HPLC water) and mobile phase B (0.08% FA and 0.02% TFA in acetonitrile) at a flow rate of 50 mL/minute. The mass spectrometer is operated at 4.5 kvolts spray voltage with a scan range from 2000 to 3500 mass to charge ratio.

### LC-MS Molecular Weight Measurement of Antibody and DVD-Ig Protein Light and Heavy Chains

Molecular weight measurement of antibody and DVD-Ig protein light chain (LC), heavy chain (HC) and deglycosylated HC are analyzed by LC-MS. Antibodies and DVD-Ig proteins are diluted to 1 mg/mL with water and the sample is reduced to LC and HC with a final concentration of 10 mM DTT for 30 minutes at 37°C. To deglycosylate the antibodies and DVD-Ig proteins, 100 mg of the antibody or DVD-Ig protein is incubated with 2 mL of PNGase F, 5 mL of 10% N-octylglucoside in a total volume of 100 mL overnight at 37 °C. After deglycosylation the sample is reduced with a final concentration of 10 mM DTT for 30 minutes at 37°C. An Agilent 1100 HPLC system with a C4 column (Vydac, cat# 214TP5115, S/N 060206537204069) is used to desalt and introduce the sample (5 mg) into an API Qstar pulsar i mass spectrometer (Applied Biosystems). A short gradient is used to elute the sample. The gradient is run with mobile phase A (0.08% FA, 0.02% TFA in HPLC water) and mobile phase B (0.08% FA and 0.02% TFA in acetonitrile) at a flow rate of 50 mL/minute. The mass spectrometer is operated at 4.5 kvolts spray voltage with a scan range from 800 to 3500 mass to charge ratio.

### Peptide Mapping

The antibody or DVD-Ig protein is denatured for 15 minutes at room temperature with a final concentration of 6 M guanidine hydrochloride in 75 mM ammonium bicarbonate. The denatured samples are reduced with a final concentration of 10 mM DTT at 37°C for 60 minutes, followed by alkylation with 50 mM iodoacetic acid (IAA) in the dark at 37°C for 30 minutes. Following alkylation, the sample is dialyzed overnight against four liters of 10 mM ammonium bicarbonate at 4°C. The dialyzed sample is diluted to 1 mg/mL with 10 mM ammonium bicarbonate, pH 7.8 and 100 mg of antibody or DVD-Ig protein is either digested with trypsin (Promega, cat# V5111) or Lys-C (Roche, cat# 11 047 825 001) at a 1:20 (w/w) trypsin/Lys-C:antibody or DVD-Ig protein ratio at 37°C for 4 hours. Digests are quenched with 1 mL of 1 N HCl. For peptide mapping with mass spectrometer detection, 40 mL of the digests are separated by reverse phase high performance liquid chromatography (RPHPLC) on a C18 column (Vydac, cat# 218TP51, S/N NE9606 10.3.5) with an Agilent 1100 HPLC system. The peptide separation is run with a gradient using mobile phase A (0.02% TFA and 0.08% FA in HPLC grade water) and mobile phase B (0.02% TFA and 0.08% FA in acetonitrile) at a flow rate of 50 mL/minutes. The API QSTAR Pulsar i mass spectromer is operated in positive mode at 4.5 kvolts spray voltage and a scan range from 800 to 2500 mass to charge ratio.

### Disulfide Bond Mapping

To denature the antibody or DVD-Ig protein, 100 mL of the antibody or DVD-Ig protein is mixed with 300 mL of 8 M guanidine HCl in 100 mM ammonium bicarbonate. The pH is checked to ensure that it is between 7 and 8 and the samples are denatured for 15 minutes at room temperature in a final concentration of 6 M guanidine HCl. A portion of the denatured sample (100 mL) is diluted to 600 mL with Milli-Q water to give a final guanidine-HCl concentration of 1 M. The sample (220 mg) is digested with either trypsin (Promega, cat # V5111, lot# 22265901) or Lys-C (Roche, cat# 11047825001, lot# 12808000) at a 1:50 trypsin or 1:50 Lys-C: antibody or DVD-Ig protein (w/w) ratios (4.4 mg enzyme: 220 mg sample) at 37°C for approximately 16 hours. An additional 5 mg of trypsin or Lys-C is added to the samples and digestion is allowed to proceed for an additional 2 hours at 37°C. Digestions are stopped by adding 1 mL of TFA to each sample. Digested samples are separated by RPHPLC using a C18 column (Vydac, cat# 218TP51 S/N NE020630-4-1A) on an Agilent HPLC system. The separation is run with the same gradient used for peptide mapping using mobile phase A (0.02% TFA and 0.08% FA in HPLC grade water) and mobile phase B (0.02% TFA and 0.08% FA in acetonitrile) at a flow rate of 50 mL/minute. The HPLC operating conditions are the same as those used for peptide mapping. The API QSTAR Pulsar i mass spectromer is operated in positive mode at 4.5 kvolts spray voltage and a scan range from 800 to 2500 mass-to-charge ratio. Disulfide bonds are assigned by matching the observed MWs of peptides with the predicted MWs of tryptic or Lys-C peptides linked by disulfide bonds.

### Free Sulfhydryl Determination

The method used to quantify free cysteines in an antibody or DVD-Ig protein is based on the reaction of Ellman's reagent, 5,5¢- dithio-bis (2-nitrobenzoic acid) (DTNB), with sulfhydryl groups (SH) which gives rise to a characteristic chromophoric product, 5-thio-(2-nitrobenzoic acid) (TNB). The reaction is illustrated in the formula:

DTNB + RSH ® RS-TNB + TNB- + H+

The absorbance of the TNB- is measured at 412 nm using a Cary 50 spectrophotometer. An absorbance curve is plotted using dilutions of 2 mercaptoethanol (b-ME) as the free SH standard and the concentrations of the free sulfhydryl groups in the protein are determined from absorbance at 412 nm of the sample.

The b-ME standard stock is prepared by a serial dilution of 14.2 M b-ME with HPLC grade water to a final concentration of 0.142 mM. Then standards in triplicate for each concentration are prepared. Antibody or DVD-Ig protein is concentrated to 10 mg/mL using an amicon ultra 10,000 MWCO centrifugal filter (Millipore, cat# UFC801096, lot# L3KN5251) and the buffer is changed to the formulation buffer used for adalimumab (5.57 mM sodium phosphate monobasic, 8.69 mM sodium phosphate dibasic, 106.69 mM NaCl, 1.07 mM sodium citrate, 6.45 mM citric acid, 66.68 mM mannitol, pH 5.2, 0.1% (w/v) Tween). The samples are mixed on a shaker at room temperature for 20 minutes. Then 180 mL of 100 mM Tris buffer, pH 8.1 is added to each sample and standard followed by the addition of 300 mL of 2 mM DTNB in 10 mM phosphate buffer, pH 8.1. After thorough mixing, the samples and standards are measured for absorption at 412 nm on a Cary 50 spectrophotometer. The standard curve is obtained by plotting the amount of free SH and OD₄₁₂ nm of the b-ME standards. Free SH content of samples are calculated based on this curve after subtraction of the blank.

### Weak Cation Exchange Chromatography

Antibody or DVD-Ig protein is diluted to 1 mg/mL with 10 mM sodium phosphate, pH 6.0. Charge heterogeneity is analyzed using a Shimadzu HPLC system with a WCX-10 ProPac analytical column (Dionex, cat# 054993, S/N 02722). The samples are loaded on the column in 80% mobile phase A (10 mM sodium phosphate, pH 6.0) and 20% mobile phase B (10 mM sodium phosphate, 500 mM NaCl, pH 6.0) and eluted at a flow rate of 1.0 mL/minute.

### Oligosaccharide Profiling

Oligosaccharides released after PNGase F treatment of antibody or DVD-Ig protein are derivatized with 2-aminobenzamide (2-AB) labeling reagent. The fluorescent-labeled oligosaccharides are separated by normal phase high performance liquid chromatography (NPHPLC) and the different forms of oligosaccharides are characterized based on retention time comparison with known standards.

The antibody or DVD-Ig protein is first digested with PNGaseF to cleave N-linked oligosaccharides from the Fc portion of the heavy chain. The antibody or DVD-Ig protein (200 mg) is placed in a 500 mL Eppendorf tube along with 2 mL PNGase F and 3 mL of 10% N-octylglucoside. Phosphate buffered saline is added to bring the final volume to 60 mL. The sample is incubated overnight at 37°C in an Eppendorf thermomixer set at 700 RPM. Adalimumab lot AFP04C is also digested with PNGase F as a control.

After PNGase F treatment, the samples are incubated at 95°C for 5 minutes in an Eppendorf thermomixer set at 750 RPM to precipitate out the proteins, then the samples are placed in an Eppendorf centrifuge for 2 minutes at 10,000 RPM to spin down the precipitated proteins. The supernatent containing the oligosaccharides are transferred to a 500 mL Eppendorf tube and dried in a speed-vac at 65°C.

The oligosaccharides are labeled with 2AB using a 2AB labeling kit purchased from Prozyme (cat# GKK-404, lot# 132026). The labeling reagent is prepared according to the manufacturer's instructions. Acetic acid (150 mL, provided in kit) is added to the DMSO vial (provided in kit) and mixed by pipeting the solution up and down several times. The acetic acid/DMSO mixture (100 mL) is transferred to a vial of 2-AB dye (just prior to use) and mixed until the dye is fully dissolved. The dye solution is then added to a vial of reductant (provided in kit) and mixed well (labeling reagent). The labeling reagent (5 mL) is added to each dried oligosaccharide sample vial, and mixed thoroughly. The reaction vials are placed in an Eppendorf thermomixer set at 65°C and 700-800 RPM for 2 hours of reaction.

After the labeling reaction, the excess fluorescent dye is removed using GlycoClean S Cartridges from Prozyme (cat# GKI-4726). Prior to adding the samples, the cartridges are washed with 1 mL of milli-Q water followed with 5 washes of 1 mL 30% acetic acid solution. Just prior to adding the samples, 1 mL of acetonitrile (Burdick and Jackson, cat# AH015-4) is added to the cartridges.

After all of the acetonitrile passed through the cartridge, the sample is spotted onto the center of the freshly washed disc and allowed to adsorb onto the disc for 10 minutes. The disc is washed with 1 mL of acetonitrile followed by five washes of 1 mL of 96% acetonitrile. The cartridges are placed over a 1.5 mL Eppendorf tube and the 2-AB labeled oligosaccharides are eluted with 3 ishes (400 mL each ish) of milli Q water.

The oligosaccharides are separated using a Glycosep N HPLC (cat# GKI-4728) column connected to a Shimadzu HPLC system. The Shimadzu HPLC system consisted of a system controller, degasser, binary pumps, autosampler with a sample cooler, and a fluorescent detector.

### Stability at Elevated Temperatures

The buffer of antibody or DVD-Ig protein is either 5.57 mM sodium phosphate monobasic, 8.69 mM sodium phosphate dibasic, 106.69 mM NaCl, 1.07 mM sodium citrate, 6.45 mM citric acid, 66.68 mM mannitol, 0.1 % (w/v) Tween, pH 5.2; or 10 mM histidine, 10 mM methionine, 4% mannitol, pH 5.9 using Amicon ultra centrifugal filters. The final concentration of the antibodies or DVD-Ig proteins is adjusted to 2 mg/mL with the appropriate buffers. The antibody or DVD-Ig protein solutions are then filter sterized and 0.25 mL aliquots are prepared under sterile conditions. The aliquots are left at either -80°C, 5°C, 25°C, or 40°C for 1, 2 or 3 weeks. At the end of the incubation period, the samples are analyzed by size exclusion chromatography and SDS-PAGE.

The stability samples are analyzed by SDS-PAGE under both reducing and non-reducing conditions. The procedure used is the same as described herein. The gels are stained overnight with colloidal blue stain (Invitrogen cat# 46-7015, 46-7016) and destained with Milli-Q water until the background is clear. The stained gels are then scanned using an Epson Expression scanner (model 1680, S/N DASX003641). To obtain more sensitivity, the same gels are silver stained using silver staining kit (Owl Scientific) and the recommended procedures given by the manufacturer is used.

### Incorporation by Reference

The contents of all cited references (including literature references, patents, patent applications, and websites) that maybe cited throughout this application are hereby expressly incorporated by reference in their entirety for any purpose, as are the references cited therein. The disclosure will employ, unless otherwise indicated, conventional techniques of immunology, molecular biology and cell biology, which are well known in the art.

The present disclosure also incorporates by reference in their entirety techniques well known in the field of molecular biology and drug delivery. These techniques include, but are not limited to, techniques described in the following publications:
Ausubel et al. (eds.), CURRENT PROTOCOLS IN MOLECULAR BIOLOGY, John Wiley &Sons, NY (1993);
Ausubel, F.M. et al. eds., SHORT PROTOCOLS IN MOLECULAR BIOLOGY (4th Ed. 1999) John Wiley & Sons, NY. (ISBN 0-471-32938-X);
CONTROLLED DRUG BIOAVAILABILITY, DRUG PRODUCT DESIGN AND PERFORMANCE, Smolen and Ball (eds.), Wiley, New York (1984);
Giege, R. and Ducruix, A. Barrett, CRYSTALLIZATION OF NUCLEIC ACIDS AND PROTEINS, a Practical Approach, 2nd ea., pp. 20 1-16, Oxford University Press, New York, New York, (1999);
Goodson, in MEDICAL APPLICATIONS OF CONTROLLED RELEASE, vol. 2, pp. 115-138 (1984);
Hammerling, et al., in: MONOCLONAL ANTIBODIES AND T-CELL HYBRIDOMAS 563-681 (Elsevier, N.Y., 1981;
Harlow et al. , ANTIBODIES: A LABORATORY MANUAL, (Cold Spring Harbor Laboratory Press, 2nd ed. 1988);
Kabat et al., SEQUENCES OF PROTEINS OF IMMUNOLOGICAL INTEREST (National Institutes of Health, Bethesda, Md. (1987) and (1991);
Kabat, E.A., et al. (1991) SEQUENCES OF PROTEINS OF IMMUNOLOGICAL INTEREST, Fifth Edition, U.S. Department of Health and Human Services, NIH Publication No. 91-3242;
Kontermann and Dubel eds., ANTIBODY ENGINEERING (2001) Springer-Verlag. New York. 790 pp. (ISBN 3-540-41354-5).
Kriegler, Gene Transfer and Expression, A Laboratory Manual, Stockton Press, NY (1990);
Lu and Weiner eds., CLONING AND EXPRESSION VECTORS FOR GENE FUNCTION ANALYSIS (2001) BioTechniques Press. Westborough, MA. 298 pp. (ISBN 1-881299-21-X).
MEDICAL APPLICATIONS OF CONTROLLED RELEASE, Langer and Wise (eds.), CRC Pres., Boca Raton, Fla. (1974);
Old, R.W. & S.B. Primrose, PRINCIPLES OF GENE MANIPULATION: AN INTRODUCTION To GENETIC ENGINEERING (3d Ed. 1985) Blackwell Scientific Publications, Boston. Studies in Microbiology; V.2:409 pp. (ISBN 0-632-01318-4).
Sambrook, J. et al. eds., MOLECULAR CLONING: A LABORATORY MANUAL (2d Ed. 1989) Cold Spring Harbor Laboratory Press, NY. Vols. 1-3. (ISBN 0-87969-309-6).
SUSTAINED AND CONTROLLED RELEASE DRUG DELIVERY SYSTEMS, J.R. Robinson, ed., Marcel Dekker, Inc., New York, 1978
Winnacker, E.L. FROM GENES To CLONES: INTRODUCTION To GENE TECHNOLOGY (1987) VCH Publishers, NY (translated by Horst Ibelgaufts). 634 pp. (ISBN 0-89573-614-4).

### Equivalents

The disclosure may be embodied in other specific forms without departing from the spirit or essential characteristics thereof. The foregoing embodiments are therefore to be considered in all respects illustrative rather than limiting of the disclosure. Scope of the disclosure is thus indicated by the appended claims rather than by the foregoing description, and all changes that come within the meaning and range of equivalency of the claims are therefore intended to be embraced herein.

Also described herein are the following aspects:
1. A binding protein comprising first and second polypeptide chains, each independently comprising VD1-(X1)n-VD2-C-(X2)n, wherein
   VD1 is a first variable domain;
   VD2 is a second variable domain;
   C is a constant domain;
   X1 is a linker with the proviso that X1 is not CH1;
   X2 is an Fc region; and
   n is 0 or 1;
   wherein the VD1 domains on the first and second polypeptide chains form a first functional target binding site and the VD2 domains on the first and second polypeptide chains form a second functional target binding site; and
   wherein the binding protein is capable of binding TNF and PGE2, TNF and SOST, TNF and NGF, or TNF and LPA, wherein:
   (i) the variable domains that form a functional target binding site for TNF comprise:
      three CDRs from SEQ ID NO: 30 and three CDRs from SEQ ID NO: 31,
      three CDRs from SEQ ID NO: 32 and three CDRs from SEQ ID NO: 33,
      three CDRs from SEQ ID NO: 34 and three CDRs from SEQ ID NO: 35,
      three CDRs from SEQ ID NO: 36 and three CDRs from SEQ ID NO: 37,
      three CDRs from SEQ ID NO: 38 and three CDRs from SEQ ID NO: 39, or
      three CDRs from SEQ ID NO: 40 and three CDRs from SEQ ID NO: 41; and
   (ii) the variable domains that form a functional target binding site for PGE2 comprise:
      three CDRs from SEQ ID NO: 46 and three CDRs from SEQ ID NO: 47;
   (iii) the variable domains that form a functional target binding site for SOST comprise:
      three CDRs from SEQ ID NO: 48 and three CDRs from SEQ ID NO: 49;
   (iv) the variable domains that form a functional target binding site for NGF comprise:
      three CDRs from SEQ ID NO: 44 and three CDRs from SEQ ID NO: 45; and
   (vi) the variable domains that form a functional target binding site for LPA comprise:
      three CDRs from SEQ ID NO: 42 and three CDRs from SEQ ID NO: 43.
2. A binding protein comprising two first and two second polypeptide chains, each independently comprising VD1-(X1)n-VD2-C-(X2)n, wherein
   VD1 is a first variable domain;
   VD2 is a second variable domain;
   C is a constant domain;
   X1 is a linker with the proviso that X1 is not CH1;
   X2 is an Fc region; and
   n is 0 or 1;
   wherein the VD1 domains on each set of first and second polypeptide chains form first functional target binding sites and the VD2 domains on each set of first and second polypeptide chains form a second functional target binding site; and
   wherein the binding protein is capable of binding TNF and PGE2, TNF and SOST, TNF and NGF, or TNF and LPA, wherein:
   (i) the variable domains that form a functional target binding site for TNF comprise:
      three CDRs from SEQ ID NO: 30 and three CDRs from SEQ ID NO: 31,
      three CDRs from SEQ ID NO: 32 and three CDRs from SEQ ID NO: 33,
      three CDRs from SEQ ID NO: 34 and three CDRs from SEQ ID NO: 35,
      three CDRs from SEQ ID NO: 36 and three CDRs from SEQ ID NO: 37,
      three CDRs from SEQ ID NO: 38 and three CDRs from SEQ ID NO: 39, or
      three CDRs from SEQ ID NO: 40 and three CDRs from SEQ ID NO: 41; and
   (ii) the variable domains that form a functional target binding site for PGE2 comprise:
      three CDRs from SEQ ID NO: 46 and three CDRs from SEQ ID NO: 47;
   (iii) the variable domains that form a functional target binding site for SOST comprise:
      three CDRs from SEQ ID NO: 48 and three CDRs from SEQ ID NO: 49;
   (iv) the variable domains that form a functional target binding site for NGF comprise:
      three CDRs from SEQ ID NO: 44 and three CDRs from SEQ ID NO: 45; and
   (vi) the variable domains that form a functional target binding site for LPA comprise:
      three CDRs from SEQ ID NO: 42 and three CDRs from SEQ ID NO: 43.
3. The binding protein according to aspect 1 or aspect 2, wherein:
   (a) the binding protein binds TNF and PGE2 with:
      (i) an IC₅₀ of at most about 17.64 nM for TNF and/or at most about 30.21 nM for PGE2, as measured by direct bind ELISA;
      (ii) an on rate constant (Kₒₙ) of at least about 3.70 x 10⁴ M⁻¹s⁻¹ for TNF, as measured by surface plasmon resonance;
      (iii) an off rate constant (K_{off}) of at most about 1.30 x 10⁻⁴ s⁻¹ for TNF, as measured by surface plasmon resonance; and/or
      (iv) a dissociation constant (K_{d}) of at most about 5.00 x 10⁻⁹ M for TNF, as measured by surface plasmon resonance.
   (b) the binding protein binds TNF and SOST with:
      (i) an IC₅₀ of at most about 4.527 nM for TNF and/or at most about 360 nM for SOST, as measured by direct bind ELISA;
      (ii) an on rate constant (Kₑₙ) of at least about 1.10 x 10⁵ M⁻¹s⁻¹ for TNF and/or at most about 1.80 x 10⁶ M⁻¹s⁻¹ for SOST, as measured by surface plasmon resonance;
      (iii) an off rate constant (K_{off}) of at most about 1.50 x 10⁻⁴ s⁻¹ for TNF and/or at most about 5.80 x 10⁻⁴ s⁻¹ for SOST, as measured by surface plasmon resonance; and/or
      (iv) a dissociation constant (K_{d}) of at most about 1.10 x 10⁻⁹ M for TNF and/or at most about 3.30 x 10⁻¹⁰ M for SOST, as measured by surface plasmon resonance
   (c) the binding protein binds TNF and NGF with:
      (i) an IC₅₀ of at most about 4.513 nM for TNF and/or at most about 3.117 nM for NGF, as measured by direct bind ELISA;
      (ii) an on rate constant (Kₒₙ) of at least about 4.50 x 104 M⁻¹s⁻¹ for TNF and/or at most about 4.50 x 10⁵ M⁻¹s⁻¹ for NGF, as measured by surface plasmon resonance;
      (iii) an off rate constant (K_{off}) of at most about 9.90 x 10⁻⁵ s⁻¹ for TNF and/or at most about 9.60 x 10⁻⁵ s⁻¹ for NGF, as measured by surface plasmon resonance; and/or
      (iv) a dissociation constant (K_{d}) of at most about 4.30 x 10⁻¹⁰ M for TNF and/or at most about 1.50 x 10⁻¹⁰ M for NGF, as measured by surface plasmon resonance; or
   (d) the binding protein binds TNF and LPA with:
      (i) an IC₅₀ of at most about 4.725 nM for TNF, as measured by direct bind ELISA;
      (ii) an on rate constant (Kₑₙ) of at least about 5.30 x 10⁴ M⁻¹s⁻¹ for TNF, as measured by surface plasmon resonance;
      (iii) an off rate constant (K_{off}) of at most about 2.20 x 10⁻⁴ s⁻¹ for TNF, as measured by surface plasmon resonance; and/or
      (iv) a dissociation constant (K_{d}) of at most about 4.10 x 10⁻⁹ M for TNF, as measured by surface plasmon resonance.
4. The binding protein according to any one of aspects 1-3, wherein:
   (i) X1 is any one of SEQ ID NOs: 1-29 or a G4S repeating sequence;
   (ii) X1 is not CL;
   (iii) (X1)n is (X1)0 and/or (X2)n is (X2)0;
   (iv) the Fc region is a variant sequence Fc region;
   (v) the Fc region is an Fc region from an IgG1, IgG2, IgG3, IgG4, IgA, IgM, IgE, or IgD;
   (iv) the binding protein is a crystallized binding protein;
   (v) the variable domains that form a functional target binding site for TNF comprise:
      SEQ ID NO: 30 and SEQ ID NO: 31,
      SEQ ID NO: 32 and SEQ ID NO: 33,
      SEQ ID NO: 34 and SEQ ID NO: 35,
      SEQ ID NO: 36 and SEQ ID NO: 37,
      SEQ ID NO: 38 and SEQ ID NO: 39, or
      SEQ ID NO: 40 and SEQ ID NO: 41;
   (vi) the variable domains that form a functional target binding site fpr PGE2 comprise SEQ ID NO: 46 and SEQ ID NO: 47;
   (vii) the variable domains that form a functional target binding site for SOST comprise SEQ ID NO: 48 and SEQ ID NO: 49;
   (vii) the variable domains that form a functional target binding site for NGF comprise SEQ ID NO: 44 and SEQ ID NO: 45; and/or
   (viii) the variable domains that form a functional target binding site for LPA comprise SEQ ID NO: 42 and SEQ ID NO: 43.
5. A binding protein conjugate comprising a binding protein according to any one of aspects 1-4, the binding protein conjugate further comprising an agent, wherein the agent is an immunoadhension molecule, an imaging agent, a therapeutic agent, or a cytotoxic agent, wherein:
   (i) the imaging agent is a radiolabel, an enzyme, a fluorescent label, a luminescent label, a bioluminescent label, a magnetic label, or biotin and wherein optionally said radiolabel is ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm; or
   (ii) the therapeutic or cytotoxic agent is an anti-metabolite, an alkylating agent, an antibiotic, a growth factor, a cytokine, an anti-angiogenic agent, an anti-mitotic agent, an anthracycline, toxin, or an apoptotic agent.
6. An isolated nucleic acid encoding the amino acid sequece of a binding protein according to any one of aspects 1-5.
7. A vector comprising the isolated nucleic acid according to aspect 6, wherein the vector is optionally selected from pcDNA, pTT, pTT3, pEFBOS, pBV, pJV, pcDNA3.1 TOPO, pEF6 TOPO, pHybE, pBOS or pBJ.
8. A host cell comprising a vector according to aspect 7.
9. The host cell according toaspect 8, wherein the host cell is a prokaryotic cell; an *Escherichia coli*; a eukaryotic cell, from a protist cell, an animal cell, a plant cell, a fungal cell, a yeast cell, an insect cell, an Sf9 cell, a mammalian cell, an avian cell, a CHO cell, or a COS cell.
10. A method of producing a binding protein, comprising culturing a host cell according to aspect 8 or aspect 9 in culture medium under conditions sufficient to produce the binding protein.
11. A protein produced by the method according to aspect 10.
12. A pharmaceutical composition comprising the binding protein according to aspect 1 oraspect 2, and a pharmaceutically acceptable carrier.
13. The pharmaceutical composition according to aspect 12 further comprising at least one additional therapeutic agent, wherein the additional therapeutic agent is optionally selected from an imaging agent, a cytotoxic agent, an angiogenesis inhibitor, a kinase inhibitor, a co-stimulation molecule blocker, an adhesion molecule blocker, an anti-cytokine antibody or functional fragment thereof, methotrexate, cyclosporin, rapamycin, FK506, a detectable label or reporter, a TNF antagonist, an antirheumatic, a muscle relaxant, a narcotic, a non-steroid anti-inflammatory drug (NSAID), an analgesic, an anesthetic, a sedative, a local anesthetic, a neuromuscular blocker, an antimicrobial, an antipsoriatic, a corticosteriod, an anabolic steroid, an erythropoietin, an immunization, an immunoglobulin, an immunosuppressive, a growth hormone, a hormone replacement drug, a radiopharmaceutical, an antidepressant, an antipsychotic, a stimulant, an asthma medication, a beta agonist, an inhaled steroid, an epinephrine or analog, a cytokine, or a cytokine antagonist.
14. Use of the binding protein according to aspect 1 oraspect 2 in the manufacture of a medicament for treating a subject for a disease or a disorder by administering to the subject the binding protein such that treatment is achieved.
15. The use according to aspect 14, wherein the disorder is rheumatoid arthritis, osteoarthritis, juvenile chronic arthritis, septic arthritis, Lyme arthritis, psoriatic arthritis, reactive arthritis, spondyloarthropathy, systemic lupus erythematosus, Crohn's disease, ulcerative colitis, inflammatory bowel disease, insulin dependent diabetes mellitus, thyroiditis, asthma, allergic diseases, psoriasis, dermatitis scleroderma, graft versus host disease, organ transplant rejection, acute or chronic immune disease associated with organ transplantation, sarcoidosis, atherosclerosis, disseminated intravascular coagulation, Kawasaki's disease, Grave's disease, nephrotic syndrome, chronic fatigue syndrome, Wegener's granulomatosis, Henoch-Schoenlein purpurea, microscopic vasculitis of the kidneys, chronic active hepatitis, uveitis, septic shock, toxic shock syndrome, sepsis syndrome, cachexia, infectious diseases, parasitic diseases, acquired immunodeficiency syndrome, acute transverse myelitis, Huntington's chorea, Parkinson's disease, Alzheimer's disease, stroke, primary biliary cirrhosis, hemolytic anemia, malignancies, heart failure, Addison's disease, sporadic, polyglandular deficiency type I, polyglandular deficiency type II, Schmidt's syndrome, adult (acute) respiratory distress syndrome, alopecia, alopecia areata, arthopathy, arthropathy, Reiter's disease, psoriatic arthropathy, ulcerative colitic arthropathy, enteropathic synovitis, chlamydia, yersinia associated arthropathy, salmonella associated arthropathy, atheromatous disease, arteriosclerosis, atopic allergy, autoimmune bullous disease, pemphigus vulgaris, pemphigus foliaceus, pemphigoid, linear IgA disease, autoimmune haemolytic anaemia, Coombs positive haemolytic anaemia, acquired pernicious anaemia, juvenile pernicious anaemia, myalgic encephalitis/Royal Free Disease, chronic mucocutaneous candidiasis, giant cell arteritis, primary sclerosing hepatitis, cryptogenic autoimmune hepatitis, acquired immunodeficiency related diseases, hepatitis B, hepatitis C, common varied immunodeficiency (common variable hypogammaglobulinaemia), dilated cardiomyopathy, female infertility, ovarian failure, premature ovarian failure, fibrotic lung disease, cryptogenic fibrosing alveolitis, post-inflammatory interstitial lung disease, interstitial pneumonitis, connective tissue disease associated interstitial lung disease, mixed connective tissue disease associated lung disease, systemic sclerosis associated interstitial lung disease, rheumatoid arthritis associated interstitial lung disease, systemic lupus erythematosus associated lung disease, dermatomyositis associated lung disease, polymyositis associated lung disease, Sjögren's disease associated lung disease, ankylosing spondylitis associated lung disease, vasculitic diffuse lung disease, haemosiderosis associated lung disease, drug-induced interstitial lung disease, fibrosis, radiation fibrosis, bronchiolitis obliterans, chronic eosinophilic pneumonia, lymphocytic infiltrative lung disease, postinfectious interstitial lung disease, gouty arthritis, autoimmune hepatitis, type-1 autoimmune hepatitis (classical autoimmune or lupoid hepatitis), type-2 autoimmune hepatitis (anti-LKM antibody hepatitis), autoimmune mediated hypoglycaemia, type B insulin resistance with acanthosis nigricans, hypoparathyroidism, acute immune disease associated with organ transplantation, chronic immune disease associated with organ transplantation, osteoarthrosis, primary sclerosing cholangitis, psoriasis type 1, psoriasis type 2, idiopathic leucopaenia, autoimmune neutropaenia, renal disease NOS, glomerulonephritides, microscopic vasulitis of the kidneys, lyme disease, discoid lupus erythematosus, male infertility idiopathic, male infertility NOS, sperm autoimmunity, multiple sclerosis (all subtypes), sympathetic ophthalmia, pulmonary hypertension secondary to connective tissue disease, Goodpasture's syndrome, pulmonary manifestation of polyarteritis nodosa, acute rheumatic fever, rheumatoid spondylitis, Still's disease, systemic sclerosis, Sjörgren's syndrome, Takayasu's disease/arteritis, autoimmune thrombocytopaenia, idiopathic thrombocytopaenia, autoimmune thyroid disease, hyperthyroidism, goitrous autoimmune hypothyroidism (Hashimoto's disease), atrophic autoimmune hypothyroidism, primary myxoedema, phacogenic uveitis, primary vasculitis, vitiligo acute liver disease, chronic liver diseases, alcoholic cirrhosis, alcohol-induced liver injury, choleosatatis, idiosyncratic liver disease, drug-induced hepatitis, non-alcoholic steatohepatitis, allergy and asthma, group B streptococci (GBS) infection, mental disorders, depression, schizophrenia, Th2 Type and Th1 Type mediated diseases, acute and chronic pain, different forms of pain, cancers, lung cancer, breast cancer, stomach cancer, bladder cancer, colon cancer, pancreatic cancer, ovarian cancer, prostate cancer, rectal cancer, hematopoietic malignancies, leukemia, lymphoma, Abetalipoprotemia, acrocyanosis, acute and chronic parasitic or infectious processes, acute leukemia, acute lymphoblastic leukemia (ALL), acute myeloid leukemia (AML), acute bacterial infection, chronic bacterial infection, acute pancreatitis, acute renal failure, adenocarcinomas, aerial ectopic beats, AIDS dementia complex, alcohol-induced hepatitis, allergic conjunctivitis, allergic contact dermatitis, allergic rhinitis, allograft rejection, alpha-I-antitrypsin deficiency, amyotrophic lateral sclerosis, anemia, angina pectoris, anterior horn cell degeneration, anti cd3 therapy, antiphospholipid syndrome, anti-receptor hypersensitivity reactions, aortic aneuryism, peripheralaneuryism, aortic dissection, arterial hypertension, arteriosclerosis, arteriovenous fistula, ataxia, sustained atrial fibrillation, paroxysmal atrial fibrillation, atrial flutter, atrioventricular block, B cell lymphoma, bone graft rejection, bone marrow transplant (BMT) rejection, bundle branch block, Burkitt's lymphoma, burns, cardiac arrhythmias, cardiac stun syndrome, cardiac tumors, cardiomyopathy, cardiopulmonary bypass inflammation response, cartilage transplant rejection, cerebellar cortical degenerations, cerebellar disorders, chaotic or multifocal atrial tachycardia, chemotherapy associated disorders, chronicmyelocytic leukemia (CML), chronic alcoholism, chronic inflammatory pathologies, chronic lymphocytic leukemia (CLL), chronic obstructive pulmonary disease (COPD), chronic salicylate intoxication, colorectal carcinoma, congestive heart failure, conjunctivitis, contact dermatitis, cor pulmonale, coronary artery disease, Creutzfeldt-Jakob disease, culture negative sepsis, cystic fibrosis, cytokine therapy associated disorders, dementia pugilistica, demyelinating diseases, dengue hemorrhagic fever, dermatitis, dermatologic conditions, diabetes, diabetes mellitus, diabetic ateriosclerotic disease, diffuse Lewy body disease, dilated congestive cardiomyopathy, disorders of the basal ganglia, Down's syndrome in middle age, drug-induced movement disorders induced by drugs which block CNS dopamine receptors, drug sensitivity, eczema, encephalomyelitis, endocarditis, endocrinopathy, epiglottitis, epstein-barr virus infection, erythromelalgia, extrapyramidal and cerebellar disorders, familial hematophagocytic lymphohistiocytosis, fetal thymus implant rejection, Friedreich's ataxia, functional peripheral arterial disorders, fungal sepsis, gas gangrene, gastric ulcer, glomerular nephritis, graft rejection of any organ, graft rejection of any tissue, gram negative sepsis, gram positive sepsis, granulomas due to intracellular organisms, hairy cell leukemia, Hallervorden-Spatz disease, Hashimoto'sthyroiditis, hay fever, heart transplant rejection, hemachromatosis, hemodialysis, hemolytic uremic syndrome/thrombolytic thrombocytopenic purpura, hemorrhage, hepatitis A, His bundle arrythmias, HIV infection/HIV neuropathy, Hodgkin's disease, hyperkinetic movement disorders, hypersensitity reactions, hypersensitivity pneumonitis, hypertension, hypokinetic movement disorders, hypothalamic-pituitary-adrenal axis evaluation, idiopathic Addison's disease, idiopathic pulmonary fibrosis, antibody mediated cytotoxicity, Asthenia, infantile spinal muscular atrophy, inflammation of the aorta, influenza A, ionizing radiation exposure, iridocyclitis, uveitis, optic neuritis, ischemia- reperfusion injury, ischemic stroke, juvenile rheumatoid arthritis, juvenile spinal muscular atrophy, Kaposi's sarcoma, kidney transplant rejection, legionella, leishmaniasis, leprosy, lesions of the corticospinal system, lipedema, liver transplant rejection, lymphederma, malaria, malignamt lymphoma, malignant histiocytosis, malignant melanoma, meningitis, meningococcemia, metabolic/idiopathic, migraine headache, mitochondrial multi.system disorder, mixed connective tissue disease, monoclonal gammopathy, multiple myeloma, multiple systems degeneration, Mencel degeneration, Dejerine-Thomas degeneration, Shi-Drager degeneration, Machado-Joseph degeneration, myasthenia gravis, mycobacterium avium intracellulare, mycobacterium tuberculosis, myelodyplastic syndrome, myocardial infarction, myocardial ischemic disorders, nasopharyngeal carcinoma, neonatal chronic lung disease, nephritis, nephrosis, neurodegenerative diseases, neurogenic muscular atrophies, neutropenic fever, non-hodgkins lymphoma, occlusion of the abdominal aorta and its branches, occulsive arterial disorders, okt3 therapy, orchitis/epidydimitis, orchitis/vasectomy reversal procedures, organomegaly, osteoporosis, pancreas transplant rejection, pancreatic carcinoma, paraneoplastic syndrome/hypercalcemia of malignancy, parathyroid transplant rejection, pelvic inflammatory disease, perennial rhinitis, pericardial disease, peripheral atherlosclerotic disease, peripheral vascular disorders, peritonitis, pernicious anemia, pneumocystis carinii pneumonia, pneumonia, POEMS syndrome (polyneuropathy, organomegaly, endocrinopathy, monoclonal gammopathy, and skin changes syndrome), post perfusion syndrome, post pump syndrome, post-MI cardiotomy syndrome, preeclampsia, progressive supranucleo palsy, primary pulmonary hypertension, radiation therapy, Raynaud's phenomenon and disease, Raynoud's disease, Refsum's disease, regular narrow QRS tachycardia, renovascular hypertension, reperfusion injury, restrictive cardiomyopathy, sarcomas, scleroderma, senile chorea, senile dementia of Lewy body type, seronegative arthropathies, shock, sickle cell anemia, skin allograft rejection, skin changes syndrome, small bowel transplant rejection, solid tumors, specific arrythmias, spinal ataxia, spinocerebellar degenerations, streptococcal myositis, structural lesions of the cerebellum, subacute sclerosing panencephalitis, syncope, syphilis of the cardiovascular system, systemic anaphalaxis, systemic inflammatory response syndrome, systemic onset juvenile rheumatoid arthritis, T-cell or FAB ALL, telangiectasia, thromboangitis obliterans, thrombocytopenia, toxicity, transplants, trauma, hemorrhage, type III hypersensitivity reactions, type IV hypersensitivity, unstable angina, uremia, urosepsis, urticaria, valvular heart diseases, varicose veins, vasculitis, venous diseases, venous thrombosis, ventricular fibrillation, viral infections, fungal infections, vital encephalitis, aseptic meningitis, vital-associated hemaphagocytic syndrome, Wernicke-Korsakoff syndrome, Wilson's disease, xenograft rejection of any organ, xenograft rejection of any tissue, acute coronary syndromes, acute idiopathic polyneuritis, acute inflammatory demyelinating polyradiculoneuropathy, acute ischemia, adult Still's disease, anaphylaxis, anti-phospholipid antibody syndrome, aplastic anemia, atopic eczema, atopic dermatitis, autoimmune dermatitis, autoimmune disorder associated with streptococcus infection, autoimmune enteropathy, autoimmune hearing loss, autoimmune lymphoproliferative syndrome (ALPS), autoimmune myocarditis, autoimmune premature ovarian failure, blepharitis, bronchiectasis, bullous pemphigoid, cardiovascular disease, catastrophic antiphospholipid syndrome, celiac disease, cervical spondylosis, chronic ischemia, cicatricial pemphigoid, clinically isolated syndrome (cis) with risk for multiple sclerosis, childhood onset psychiatric disorder, dacryocystitis, dermatomyositis, diabetic retinopathy, disk herniation, disk prolapse, drug induced immune hemolytic anemia, endometriosis, endophthalmitis, episcleritis, erythema multiforme, erythema multiforme major, gestational pemphigoid, Guillain-Barré syndrome (GBS), Hughes syndrome, idiopathic Parkinson's disease, idiopathic interstitial pneumonia, IgE-mediated allergy, immune hemolytic anemia, inclusion body myositis, infectious ocular inflammatory disease, inflammatory demyelinating disease, inflammatory heart disease, inflammatory kidney disease, IPF/UIP, iritis, keratitis, keratojuntivitis sicca, Kussmaul disease or Kussmaul-Meier disease, Landry's paralysis, Langerhan's cell histiocytosis, livedo reticularis, macular degeneration, microscopic polyangiitis, morbus bechterev, motor neuron disorders, mucous membrane pemphigoid, multiple organ failure, myelodysplastic syndrome, myocarditis, nerve root disorders, neuropathy, non-A non-B hepatitis, optic neuritis, osteolysis, pauciarticular JRA, peripheral artery occlusive disease (PAOD), peripheral vascular disease (PVD), peripheral artery, disease (PAD), phlebitis, polyarteritis nodosa, periarteritis nodosa, polychondritis, polymyalgia rheumatica, poliosis, polyarticular JRA, polyendocrine deficiency syndrome, polymyositis, polymyalgia rheumatica (PMR), primary Parkinsonism, prostatitis, pure red cell aplasia, primary adrenal insufficiency, recurrent neuromyelitis optica, restenosis, rheumatic heart disease, sapho (synovitis, acne, pustulosis, hyperostosis, and osteitis), secondary amyloidosis, shock lung, scleritis, sciatica, secondary adrenal insufficiency, silicone associated connective tissue disease, sneddon-wilkinson dermatosis, spondilitis ankylosans, Stevens-Johnson syndrome (SJS), temporal arteritis, toxoplasmic retinitis, toxic epidermal necrolysis, transverse myelitis, TRAPS (tumor necrosis factor receptor, type 1 allergic reaction, type II diabetes, usual interstitial pneumonia (UIP), vasculitis, vernal conjunctivitis, viral retinitis, Vogt-Koyanagi-Harada syndrome (VKH syndrome), wet macular degeneration, or wound healing.
16. The use according to aspect 14 or aspect 15, wherein the medicament is formulated for parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, or transdermal administration.
17. A method of determining the presence, amount or concentration of at least one target or fragment thereof in a test sample by an immunoassay,
   wherein the immunoassay comprises contacting the test sample with at least one binding protein and at least one detectable label,
   wherein the at least one binding protein comprises the binding protein according to aspect 1 or aspect 2.
18. The method according to aspect 17, further comprising:
   (i) contacting the test sample with the at least one binding protein, wherein the binding protein binds to an epitope on the target or fragment thereof so as to form a first complex, (ii) contacting the complex with the at least one detectable label, wherein the detectable label binds to the binding protein or an epitope on the target or fragment thereof that is not bound by the binding protein to form a second complex, and (iii) detecting the presence, amount or concentration of the target or fragment thereof in the test sample based on the signal generated by the detectable label in the second complex, wherein the presence, amount or concentration of the target or fragment thereof is directly correlated with the signal generated by the detectable label.
19. The method according to aspect 17, further comprising:
   (i) contacting the test sample with the at least one binding protein, wherein the binding protein binds to an epitope on the target or fragment thereof so as to form a first complex, (ii) contacting the complex with the at least one detectable label, wherein the detectable label competes with the target or fragment thereof for binding to the binding protein so as to form a second complex, and (iii) detecting the presence, amount or concentration of the target or fragment thereof in the test sample based on the signal generated by the detectable label in the second complex, wherein the presence, amount or concentration of the target or fragment thereof is indirectly correlated with the signal generated by the detectable label.
20. The method according to any one of aspects 17-19, wherein the test sample is from a patient and the method
   (i) further comprises diagnosing, prognosticating, or assessing the efficiency of therapeutic/prophylactic treatment of the patient,
   (ii) further comprises modifying the therapeutic/prophylactic treatment of the patient as needed to improve efficacy;
   (iii) is adapted for use in an automated system or a semi-automated system; and/or
   (iv) determines the presence, amount, or concentration of more than one target in the sample.
21. A kit for assaying a test sample for the presence, amount, or concentration of a target or fragment thereof, the kit comprising
   (i) instructions for assaying the test sample for the target or fragment thereof; and
   (ii) at least one binding protein comprising the binding protein according to any one of aspects 1-2.
22. A binding protein comprising first and second polypeptide chains, wherein the first polypeptide chain comprises VD1-(X1)n-VD2-C-(X2)n, wherein:
   VD1 is a first heavy chain variable domain;
   VD2 is a second heavy chain variable domain;
   C is a constant domain;
   X1 is a linker with the proviso that X1 is not CH1;
   X2 is an Fc region; and
   n is 0 or 1; and
   wherein the second polypeptide chain comprises VD1-(X1)n-VD2-C-(X2)n, wherein
   VD1 is a first light chain variable domain;
   VD2 is a second light chain variable domain;
   C is a constant domain;
   X1 is a linker with the proviso that X1 is not CL;
   X2 is an Fc region;
   n is 0 or 1; and
   wherein the VD1 domains on the first and second polypeptide chains form a first functional target binding site and the VD2 domains on the first and second polypeptide chains form a second functional target binding site; and
   wherein the binding protein is capable of binding TNF and PGE2, TNF and SOST, TNF and NGF, or TNF and LPA, wherein:
   (i) the variable domains that form a functional target binding site for TNF comprise:
      three CDRs from SEQ ID NO: 30 and three CDRs from SEQ ID NO: 31,
      three CDRs from SEQ ID NO: 32 and three CDRs from SEQ ID NO: 33,
      three CDRs from SEQ ID NO: 34 and three CDRs from SEQ ID NO: 35,
      three CDRs from SEQ ID NO: 36 and three CDRs from SEQ ID NO: 37,
      three CDRs from SEQ ID NO: 38 and three CDRs from SEQ ID NO: 39, or
      three CDRs from SEQ ID NO: 40 and three CDRs from SEQ ID NO: 41; and
   (ii) the variable domains that form a functional target binding site for PGE2 comprise:
      three CDRs from SEQ ID NO: 46 and three CDRs from SEQ ID NO: 47;
   (iii) the variable domains that form a functional target binding site for SOST comprise:
      three CDRs from SEQ ID NO: 48 and three CDRs from SEQ ID NO: 49;
   (iv) the variable domains that form a functional target binding site for NGF comprise:
      three CDRs from SEQ ID NO: 44 and three CDRs from SEQ ID NO: 45; and
   (vi) the variable domains that form a functional target binding site for LPA comprise:
      three CDRs from SEQ ID NO: 42 and three CDRs from SEQ ID NO: 43.
23. The binding protein according to aspect 22, wherein:
   (a) the binding protein binds TNF and PGE2 with:
      (i) an IC₅₀ of at most about 17.64 nM for TNF and/or at most about 30.21 nM for PGE2, as measured by direct bind ELISA;
      (ii) an on rate constant (Kₑₙ) of at least about 3.70 x 10⁴ M⁻¹s⁻¹ for TNF, as measured by surface plasmon resonance;
      (iii) an off rate constant (K_{off}) of at most about 1.30 x 10⁻⁴ s⁻¹ for TNF, as measured by surface plasmon resonance; and/or
      (iv) a dissociation constant (K_{d}) of at most about 5.00 x 10⁻⁹ M for TNF, as measured by surface plasmon resonance.
   (b) the binding protein binds TNF and SOST with:
      (i) an IC₅₀ of at most about 4.527 nM for TNF and/or at most about 360 nM for SOST, as measured by direct bind ELISA;
      (ii) an on rate constant (Kₑₙ) of at least about 1.10 x 10⁵ M⁻¹s⁻¹ for TNF and/or at most about 1.80 x 10⁶ M⁻¹s⁻¹ for SOST, as measured by surface plasmon resonance;
      (iii) an off rate constant (K_{off}) of at most about 1.50 x 10⁻⁴s⁻¹ for TNF and/or at most about 5.80 x 10⁻⁴ s⁻¹ for SOST, as measured by surface plasmon resonance; and/or
      (iv) a dissociation constant (K_{d}) of at most about 1.10 x 10⁻⁹ M for TNF and/or at most about 3.30 x 10⁻¹⁰ M for SOST, as measured by surface plasmon resonance
   (c) the binding protein binds TNF and NGF with:
      (i) an IC₅₀ of at most about 4.513 nM for TNF and/or at most about 3.117 nM for NGF, as measured by direct bind ELISA;
      (ii) an on rate constant (Kₒₙ) of at least about 4.50 x 104 M⁻¹s⁻¹ for TNF and/or at most about 4.50 x 10⁵ M⁻¹s⁻¹ for NGF, as measured by surface plasmon resonance;
      (iii) an off rate constant (K_{off}) of at most about 9.90 x 10⁻⁵ s⁻¹ for TNF and/or at most about 9.60 x 10⁻⁵ s⁻¹ for NGF, as measured by surface plasmon resonance; and/or
      (iv) a dissociation constant (K_{d}) of at most about 4.30 x 10⁻¹⁰ M for TNF and/or at most about 1.50 x 10⁻¹⁰ M for NGF, as measured by surface plasmon resonance; or
   (d) the binding protein binds TNF and LPA with:
      (i) an IC₅₀ of at most about 4.725 nM for TNF, as measured by direct bind ELISA;
      (ii) an on rate constant (Kₒₙ) of at least about 5.30 x 10⁴ M⁻¹s⁻¹ for TNF, as measured by surface plasmon resonance;
      (iii) an off rate constant (K_{off}) of at most about 2.20 x 10⁻⁴ s⁻¹ for TNF, as measured by surface plasmon resonance; and/or
      (iv) a dissociation constant (K_{d}) of at most about 4.10 x 10⁻⁹ M for TNF, as measured by surface plasmon resonance.
24. The binding protein according toaspect 22 or aspect 23, wherein the binding protein comprises two first polypeptide chains and two second polypeptide chains.
25. The binding protein of according to any one of aspects 1-3 or 22-24, wherein the binding protein comprises: DVD1948H (comprising SEQ ID NO: 30 and 46) and DVD1948L (comprising SEQ ID NOs: 31 and 47); DVD1949H (comprising SEQ ID NO: 46 and 30) and DVD1949L (comprising SEQ ID NOs: 47 and 31); DVD1950H (comprising SEQ ID NOs: 30 and 48) and DVD1950L (comprising SEQ ID NOs: 31 and 49); DVD1951H (comprising SEQ ID NOs: 48 and 30) and DVD1951 L (comprising SEQ ID NOs: 49 and 31); DVD1952H (comprising SEQ ID NOs: 30 and 44) and DVD1952L (comprising SEQ ID NOs: 31 and 45); DVD1953H (comprising SEQ ID NOs: 44 and 30) and DVD1953L (comprising SEQ ID NOs: 45 and 31); DVD 1954H (comprising SEQ ID NOs: 30 and 42) and DVD1954L (comprising SEQ ID NOs: 31 and 43); DVD1955H (comprising SEQ ID NOs: 42 and 30) and DVD1955L (comprising SEQ ID NOs: 43 and 31); DVD1956H (comprising SEQ ID NOs: 32 and 46) and DVD1956L (comprising SEQ ID NOs: 33 and 47); DVD1957H (comprising SEQ ID NOs: 46 and 32) and DVD1957L (comprising SEQ ID NOs: 47 and 33); DVD1958H (comprising SEQ ID NOs: 32 and 48) and DVD1958L (comprising SEQ ID NOs: 33 and 49); DVD1959H (comprising SEQ ID NOs: 48 and 32) and DVD1959L (comprising SEQ ID NOs: 49 and 33); DVD1960H (comprising SEQ ID NOs: 32 and 44) and DVD1960L (comprising SEQ ID NOs: 33 and 45); DVD1961H (comprising SEQ ID NOs: 44 and 32) and DVD1961L (comprising SEQ ID NOs: 45 and 33); DVD1962H (comprising SEQ ID NOs: 32 and 42) and DVD1962L (comprising SEQ ID NOs: 33 and 43); DVD1963H (comprising SEQ ID NOs: 42 and 32) and DVD1963L (comprising SEQ ID NOs: 43 and 32); DVD1964H (comprising SEQ ID NOs: 34 and 46) and DVD1964L (comprising SEQ ID NOs: 35 and 47); DVD1965H (comprising SEQ ID NOs: 46 and 34) and DVD1965L (comprising SEQ ID NOs: 47 and 35); DVD1966H (comprising SEQ ID NOs: 34 and 48) and DVD1966L (comprising SEQ ID NOs: 35 and 49); DVD1967H (comprising SEQ ID NOs: 48 and 34) and DVD1967L (comprising SEQ ID NOs: 49 and 35); DVD1968H (comprising SEQ ID NOs: 34 and 44) and DVD1968L (comprising SEQ ID NOs: 35 and 45); DVD1969H (comprising SEQ ID NOs: 44 and 34) and DVD1969L (comprising SEQ ID NOs: 45 and 35); DVD1970H (comprising SEQ ID NOs: 34 and 42) and DVD1970L (comprising SEQ ID NOs: 35 and 43); DVD1971H (comprising SEQ ID NOs: 42 and 34) and DVD1971L (comprising SEQ ID NOs: 43 and 35); DVD1972H (comprising SEQ ID NOs: 36 and 46) and DVD1972L (comprising SEQ ID NOs: 37 and 47); DVD1973H (comprising SEQ ID NOs: 46 and 36) and DVD1973L (comprising SEQ ID NOs: 47 and 37); DVD1974H (comprising SEQ ID NOs: 36 and 48) and DVD1974L (comprising SEQ ID NOs: 37 and 49); DVD1975H (comprising SEQ ID NOs: 48 and 36) and DVD1975L (comprising SEQ ID NOs: 49 and 37); DVD1976H (comprising SEQ ID NOs: 36 and 44) and DVD1976L (comprising SEQ ID NOs: 31 and 45); DVD1977H (comprising SEQ ID NOs: 44 and 36) and DVD1977L (comprising SEQ ID NOs: 45 and 37); DVD1978H (comprising SEQ ID NOs: 36 and 42) and DVD1978L (comprising SEQ ID NOs: 37 and 43); DVD1979H (comprising SEQ ID NOs: 42 and 36) and DVD1979L (comprising SEQ ID NOs: 43 and 37); DVD1980H (comprising SEQ ID NOs: 38 and 46) and DVD1980L (comprising SEQ ID NOs: 39 and 47); DVD1981 H (comprising SEQ ID NOs: 46 and 38) and DVD1981L (comprising SEQ ID NOs: 47 and 39); DVD1982H (comprising SEQ ID NOs: 38 and 48) and DVD1982L (comprising SEQ ID NOs: 39 and 49); DVD1983H (comprising SEQ ID NOs: 48 and 38) and DVD1983L (comprising SEQ ID NOs: 49 and 39); DVD1984H (comprising SEQ ID NOs: 38 and 44) and DVD1984L (comprising SEQ ID NOs: 39 and 45); DVD1985H (comprising SEQ ID NOs: 44 and 38) and DVD1985L (comprising SEQ ID NOs: 45 and 39); DVD1986H (comprising SEQ ID NOs: 38 and 42) and DVD1986L (comprising SEQ ID NOs: 39 and 43); DVD1987H (comprising SEQ ID NOs: 42 and 38) and DVD1987L (comprising SEQ ID NOs: 43 and 39); DVD1988H (comprising SEQ ID NOs: 40 and 46) and DVD1988L (comprising SEQ ID NOs: 41 and 47); DVD1989H (comprising SEQ ID NOs: 46 and 40) and DVD1989L (comprising SEQ ID NOs: 47 and 41); DVD1990H (comprising SEQ ID NOs: 40 and 48) and DVD1990L (comprising SEQ ID NOs: 41 and 49); DVD1991 H (comprising SEQ ID NOs: 48 and 40) and DVD1991 L (comprising SEQ ID NOs: 49 and 41); DVD1992H (comprising SEQ ID NOs: 40 and 44) and DVD1992L (comprising SEQ ID NOs: 41 and 45); DVD1993H (comprising SEQ ID NOs: 44 and 40) and DVD1993L (comprising SEQ ID NOs: 45 and 41); DVD1994H (comprising SEQ ID NOs: 40 and 42) and DVD1994L (comprising SEQ ID NOs: 41 and 43); or DVD1995H (comprising SEQ ID NOs: 42 and 40) and DVD1995L (comprising SEQ ID NOs: 43 and 41).

## Claims

1. A binding protein comprising first and second polypeptide chains, each independently comprising VD1-(X1)n-VD2-C-(X2)n, wherein
VD1 is a first variable domain;
VD2 is a second variable domain;
C is a constant domain;
X1 is a linker with the proviso that X1 is not CH1;
X2 is an Fc region; and
n is 0 or 1;
wherein the VD1 domains on the first and second polypeptide chains form a first functional target binding site and the VD2 domains on the first and second polypeptide chains form a second functional target binding site; and
wherein the binding protein is capable of binding TNF and PGE2, TNF and SOST, TNF and NGF, or TNF and LPA, wherein:
(i) the variable domains that form a functional target binding site for TNF comprise:
three CDRs from SEQ ID NO: 30 and three CDRs from SEQ ID NO: 31,
three CDRs from SEQ ID NO: 32 and three CDRs from SEQ ID NO: 33,
three CDRs from SEQ ID NO: 34 and three CDRs from SEQ ID NO: 35,
three CDRs from SEQ ID NO: 36 and three CDRs from SEQ ID NO: 37,
three CDRs from SEQ ID NO: 38 and three CDRs from SEQ ID NO: 39, or
three CDRs from SEQ ID NO: 40 and three CDRs from SEQ ID NO: 41; and
(ii) the variable domains that form a functional target binding site for PGE2 comprise: three CDRs from SEQ ID NO: 46 and three CDRs from SEQ ID NO: 47;
(iii) the variable domains that form a functional target binding site for SOST comprise: three CDRs from SEQ ID NO: 48 and three CDRs from SEQ ID NO: 49;
(iv) the variable domains that form a functional target binding site for NGF comprise: three CDRs from SEQ ID NO: 44 and three CDRs from SEQ ID NO: 45; and
(vi) the variable domains that form a functional target binding site for LPA comprise: three CDRs from SEQ ID NO: 42 and three CDRs from SEQ ID NO: 43.

2. A binding protein according to claim 1 comprising two first and two second polypeptide chains, each independently comprising VD1-(X1)n-VD2-C-(X2)n, wherein
VD1 is a first variable domain;
VD2 is a second variable domain;
C is a constant domain;
X1 is a linker with the proviso that X1 is not CH1;
X2 is an Fc region; and
n is 0 or 1;
wherein the VD1 domains on each set of first and second polypeptide chains form first functional target binding sites and the VD2 domains on each set of first and second polypeptide chains form a second functional target binding site; and
wherein the binding protein is capable of binding TNF and PGE2, TNF and SOST, TNF and NGF, or TNF and LPA, wherein:
(i) the variable domains that form a functional target binding site for TNF comprise:
three CDRs from SEQ ID NO: 30 and three CDRs from SEQ ID NO: 31,
three CDRs from SEQ ID NO: 32 and three CDRs from SEQ ID NO: 33,
three CDRs from SEQ ID NO: 34 and three CDRs from SEQ ID NO: 35,
three CDRs from SEQ ID NO: 36 and three CDRs from SEQ ID NO: 37,
three CDRs from SEQ ID NO: 38 and three CDRs from SEQ ID NO: 39, or
three CDRs from SEQ ID NO: 40 and three CDRs from SEQ ID NO: 41; and
(ii) the variable domains that form a functional target binding site for PGE2 comprise: three CDRs from SEQ ID NO: 46 and three CDRs from SEQ ID NO: 47;
(iii) the variable domains that form a functional target binding site for SOST comprise: three CDRs from SEQ ID NO: 48 and three CDRs from SEQ ID NO: 49;
(iv) the variable domains that form a functional target binding site for NGF comprise: three CDRs from SEQ ID NO: 44 and three CDRs from SEQ ID NO: 45; and
(vi) the variable domains that form a functional target binding site for LPA comprise: three CDRs from SEQ ID NO: 42 and three CDRs from SEQ ID NO: 43.

3. A binding protein according to claim 1, comprising first and second polypeptide chains, wherein the first polypeptide chain comprises VD1-(X1)n-VD2-C-(X2)n, wherein:
VD1 is a first heavy chain variable domain;
VD2 is a second heavy chain variable domain;
C is a constant domain;
X1 is a linker with the proviso that X1 is not CH1;
X2 is an Fc region; and
n is 0 or 1; and
wherein the second polypeptide chain comprises VD1-(X1)n-VD2-C-(X2)n, wherein
VD1 is a first light chain variable domain;
VD2 is a second light chain variable domain;
C is a constant domain;
X1 is a linker with the proviso that X1 is not CL;
X2 is an Fc region;
n is 0 or 1; and
wherein the VD1 domains on the first and second polypeptide chains form a first functional target binding site and the VD2 domains on the first and second polypeptide chains form a second functional target binding site; and
wherein the binding protein is capable of binding TNF and PGE2, TNF and SOST, TNF and NGF, or TNF and LPA, wherein:
(i) the variable domains that form a functional target binding site for TNF comprise:
three CDRs from SEQ ID NO: 30 and three CDRs from SEQ ID NO: 31,
three CDRs from SEQ ID NO: 32 and three CDRs from SEQ ID NO: 33,
three CDRs from SEQ ID NO: 34 and three CDRs from SEQ ID NO: 35,
three CDRs from SEQ ID NO: 36 and three CDRs from SEQ ID NO: 37,
three CDRs from SEQ ID NO: 38 and three CDRs from SEQ ID NO: 39, or
three CDRs from SEQ ID NO: 40 and three CDRs from SEQ ID NO: 41; and
(ii) the variable domains that form a functional target binding site for PGE2 comprise: three CDRs from SEQ ID NO: 46 and three CDRs from SEQ ID NO: 47;
(iii) the variable domains that form a functional target binding site for SOST comprise: three CDRs from SEQ ID NO: 48 and three CDRs from SEQ ID NO: 49;
(iv) the variable domains that form a functional target binding site for NGF comprise: three CDRs from SEQ ID NO: 44 and three CDRs from SEQ ID NO: 45; and
(vi) the variable domains that form a functional target binding site for LPA comprise: three CDRs from SEQ ID NO: 42 and three CDRs from SEQ ID NO: 43.

4. The binding protein according to claim 3, wherein the binding protein comprises two first polypeptide chains and two second polypeptide chains.

5. The binding protein according to any one of claims 1 to 3, wherein:
(a) the binding protein binds TNF and PGE2 with:
(i) an IC50 of at most about 17.64 nM for TNF and/or at most about 30.21 nM for PGE2, as measured by direct bind ELISA;
(ii) an on rate constant (Kon) of at least about 3.70 x 10⁴ M⁻¹ s⁻¹ for TNF, as measured by surface plasmon resonance;
(iii) an off rate constant (Koff) of at most about 1.30 x 10⁻⁴ s⁻¹ for TNF, as measured by surface plasmon resonance; and/or
(iv) a dissociation constant (Kd) of at most about 5.00 x 10⁻⁹ M for TNF, as measured by surface plasmon resonance
(b) the binding protein binds TNF and SOST with:
(i) an IC50 of at most about 4.527 nM for TNF and/or at most about 360 nM for SOST, as measured by direct bind ELISA;
(ii) an on rate constant (Kon) of at least about 1.10 x 10⁵ M⁻¹ s⁻¹ for TNF and/or at most about 1.80 x 10⁶ M⁻¹s⁻¹ for SOST, as measured by surface plasmon resonance;
(iii) an off rate constant (Koff) of at most about 1.50 x 10⁻⁴ s⁻¹ for TNF and/or at most about 5.80 x 10⁻⁴ s⁻¹ for SOST, as measured by surface plasmon resonance; and/or
(iv) a dissociation constant (Kd) of at most about 1.10 x 10⁻⁹ M for TNF and/or at most about 3.30 x 10⁻¹⁰ M for SOST, as measured by surface plasmon resonance
(c) the binding protein binds TNF and NGF with:
(i) an IC50 of at most about 4.513 nM for TNF and/or at most about 3.117 nM for NGF, as measured by direct bind ELISA;
(ii) an on rate constant (Kon) of at least about 4.50 x 10⁴ M⁻¹s⁻¹ for TNF and/or at most about 4.50 x 10⁵ M⁻¹s⁻¹ for NGF, as measured by surface plasmon resonance;
(iii) an off rate constant (Koff) of at most about 9.90 x 10⁻⁵ s⁻¹ for TNF and/or at most about 9.60 x 10⁻⁵ s⁻¹ for NGF, as measured by surface plasmon resonance; and/or
(iv) a dissociation constant (Kd) of at most about 4.30 x 10⁻¹⁰ M for TNF and/or at most about 1.50 x 10⁻¹⁰ M for NGF, as measured by surface plasmon resonance; or
(d) the binding protein binds TNF and LPA with:
(i) an IC50 of at most about 4.725 nM for TNF, as measured by direct bind ELISA;
(ii) an on rate constant (Kon) of at least about 5.30 x 10⁴ M⁻¹s⁻¹ for TNF, as measured by surface plasmon resonance;
(iii) an off rate constant (Koff) of at most about 2.20 x 10⁻⁴ s⁻¹ for TNF, as measured by surface plasmon resonance; and/or
(iv) a dissociation constant (Kd) of at most about 4.10 x 10⁻⁹ M for TNF, as measured by surface plasmon resonance.

6. The binding protein according to any one of claims 1-5, wherein:
(i) X1 is any one of SEQ ID NOs: 1 29 or a G4S repeating sequence;
(ii) X1 is not CL;
(iii) (X1)n is (X1)0 and/or (X2)n is (X2)0;
(iv) the Fc region is a variant sequence Fc region;
(v) the Fc region is an Fc region from an IgG1, IgG2, IgG3, IgG4, IgA, IgM, IgE, or IgD;
(iv) the binding protein is a crystallized binding protein;
(v) the variable domains that form a functional target binding site for TNF comprise: SEQ ID NO: 30 and SEQ ID NO: 31, SEQ ID NO: 32 and SEQ ID NO: 33, SEQ ID NO: 34 and SEQ ID NO: 35, SEQ ID NO: 36 and SEQ ID NO: 37, SEQ ID NO: 38 and SEQ ID NO: 39, or SEQ ID NO: 40 and SEQ ID NO: 41;
(vi) the variable domains that form a functional target binding site for PGE2 comprise SEQ ID NO: 46 and SEQ ID NO: 47;
(vii) the variable domains that form a functional target binding site for SOST comprise SEQ ID NO: 48 and SEQ ID NO: 49;
(vii) the variable domains that form a functional target binding site for NGF comprise SEQ ID NO: 44 and SEQ ID NO: 45; and/or
(viii) the variable domains that form a functional target binding site for LPA comprise SEQ ID NO: 42 and SEQ ID NO: 43.

7. The binding protein of according to any one of claims 1 to 5, wherein the binding protein comprises: DVD1948H (comprising SEQ ID NO: 30 and 46) and DVD1948L (comprising SEQ ID NOs: 31 and 47); DVD1949H (comprising SEQ ID NO: 46 and 30) and DVD1949L (comprising SEQ ID NOs: 47 and 31); DVD1950H (comprising SEQ ID NOs: 30 and 48) and DVD1950L (comprising SEQ ID NOs: 31 and 49); DVD1951H (comprising SEQ ID NOs: 48 and 30) and DVD1951L (comprising SEQ ID NOs: 49 and 31); DVD1952H (comprising SEQ ID NOs: 30 and 44) and DVD1952L (comprising SEQ ID NOs: 31 and 45); DVD1953H (comprising SEQ ID NOs: 44 and 30) and DVD1953L (comprising SEQ ID NOs: 45 and 31); DVD 1954H (comprising SEQ ID NOs: 30 and 42) and DVD1954L (comprising SEQ ID NOs: 31 and 43); DVD1955H (comprising SEQ ID NOs: 42 and 30) and DVD1955L (comprising SEQ ID NOs: 43 and 31); DVD1956H (comprising SEQ ID NOs: 32 and 46) and DVD1956L (comprising SEQ ID NOs: 33 and 47); DVD1957H (comprising SEQ ID NOs: 46 and 32) and DVD1957L (comprising SEQ ID NOs: 47 and 33); DVD1958H (comprising SEQ ID NOs: 32 and 48) and DVD1958L (comprising SEQ ID NOs: 33 and 49); DVD1959H (comprising SEQ ID NOs: 48 and 32) and DVD1959L (comprising SEQ ID NOs: 49 and 33); DVD1960H (comprising SEQ ID NOs: 32 and 44) and DVD1960L (comprising SEQ ID NOs: 33 and 45); DVD1961H (comprising SEQ ID NOs: 44 and 32) and DVD1961L (comprising SEQ ID NOs: 45 and 33); DVD1962H (comprising SEQ ID NOs: 32 and 42) and DVD1962L (comprising SEQ ID NOs: 33 and 43); DVD1963H (comprising SEQ ID NOs: 42 and 32) and DVD1963L (comprising SEQ ID NOs: 43 and 32); DVD1964H (comprising SEQ ID NOs: 34 and 46) and DVD1964L (comprising SEQ ID NOs: 35 and 47); DVD1965H (comprising SEQ ID NOs: 46 and 34) and DVD1965L (comprising SEQ ID NOs: 47 and 35); DVD1966H (comprising SEQ ID NOs: 34 and 48) and DVD1966L (comprising SEQ ID NOs: 35 and 49); DVD1967H (comprising SEQ ID NOs: 48 and 34) and DVD1967L (comprising SEQ ID NOs: 49 and 35); DVD1968H (comprising SEQ ID NOs: 34 and 44) and DVD1968L (comprising SEQ ID NOs: 35 and 45); DVD1969H (comprising SEQ ID NOs: 44 and 34) and DVD1969L (comprising SEQ ID NOs: 45 and 35); DVD1970H (comprising SEQ ID NOs: 34 and 42) and DVD1970L (comprising SEQ ID NOs: 35 and 43); DVD1971H (comprising SEQ ID NOs: 42 and 34) and DVD1971L (comprising SEQ ID NOs: 43 and 35); DVD1972H (comprising SEQ ID NOs: 36 and 46) and DVD1972L (comprising SEQ ID NOs: 37 and 47); DVD1973H (comprising SEQ ID NOs: 46 and 36) and DVD1973L (comprising SEQ ID NOs: 47 and 37); DVD1974H (comprising SEQ ID NOs: 36 and 48) and DVD1974L (comprising SEQ ID NOs: 37 and 49); DVD1975H (comprising SEQ ID NOs: 48 and 36) and DVD1975L (comprising SEQ ID NOs: 49 and 37); DVD1976H (comprising SEQ ID NOs: 36 and 44) and DVD1976L (comprising SEQ ID NOs: 31 and 45); DVD1977H (comprising SEQ ID NOs: 44 and 36) and DVD1977L (comprising SEQ ID NOs: 45 and 37); DVD1978H (comprising SEQ ID NOs: 36 and 42) and DVD1978L (comprising SEQ ID NOs: 37 and 43); DVD1979H (comprising SEQ ID NOs: 42 and 36) and DVD1979L (comprising SEQ ID NOs: 43 and 37); DVD1980H (comprising SEQ ID NOs: 38 and 46) and DVD1980L (comprising SEQ ID NOs: 39 and 47); DVD1981H (comprising SEQ ID NOs: 46 and 38) and DVD1981L (comprising SEQ ID NOs: 47 and 39); DVD1982H (comprising SEQ ID NOs: 38 and 48) and DVD1982L (comprising SEQ ID NOs: 39 and 49); DVD1983H (comprising SEQ ID NOs: 48 and 38) and DVD1983L (comprising SEQ ID NOs: 49 and 39); DVD1984H (comprising SEQ ID NOs: 38 and 44) and DVD1984L (comprising SEQ ID NOs: 39 and 45); DVD1985H (comprising SEQ ID NOs: 44 and 38) and DVD1985L (comprising SEQ ID NOs: 45 and 39); DVD1986H (comprising SEQ ID NOs: 38 and 42) and DVD1986L (comprising SEQ ID NOs: 39 and 43); DVD1987H (comprising SEQ ID NOs: 42 and 38) and DVD1987L (comprising SEQ ID NOs: 43 and 39); DVD1988H (comprising SEQ ID NOs: 40 and 46) and DVD1988L (comprising SEQ ID NOs: 41 and 47); DVD1989H (comprising SEQ ID NOs: 46 and 40) and DVD1989L (comprising SEQ ID NOs: 47 and 41); DVD1990H (comprising SEQ ID NOs: 40 and 48) and DVD1990L (comprising SEQ ID NOs: 41 and 49); DVD1991H (comprising SEQ ID NOs: 48 and 40) and DVD1991L (comprising SEQ ID NOs: 49 and 41); DVD1992H (comprising SEQ ID NOs: 40 and 44) and DVD1992L (comprising SEQ ID NOs: 41 and 45); DVD1993H (comprising SEQ ID NOs: 44 and 40) and DVD1993L (comprising SEQ ID NOs: 45 and 41); DVD1994H (comprising SEQ ID NOs: 40 and 42) and DVD1994L (comprising SEQ ID NOs: 41 and 43); or DVD1995H (comprising SEQ ID NOs: 42 and 40) and DVD1995L (comprising SEQ ID NOs: 43 and 41).

8. A binding protein conjugate comprising a binding protein according to any one of claims 1-7, the binding protein conjugate further comprising an agent, wherein the agent is an immunoadhesion molecule, an imaging agent, a therapeutic agent, or a cytotoxic agent, optionally wherein:
(i) the imaging agent is a radiolabel, an enzyme, a fluorescent label, a luminescent label, a bioluminescent label, a magnetic label, or biotin and wherein optionally said radiolabel is ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm; or
(ii) the therapeutic or cytotoxic agent is an anti-metabolite, an alkylating agent, an antibiotic, a growth factor, a cytokine, an anti-angiogenic agent, an anti-mitotic agent, an anthracycline, toxin, or an apoptotic agent.

9. An isolated nucleic acid encoding the amino acid sequence of a binding protein according to any one of claims 1-7.

10. A vector comprising the isolated nucleic acid according to claim 9, wherein the vector is optionally selected from pcDNA, pTT, pTT3, pEFBOS, pBV, pJV, pcDNA3.1 TOPO, pEF6 TOPO, pHybE, pBOS or pBJ.

11. A host cell comprising a vector according to claim 10, optionally wherein the host cell is a prokaryotic cell; an Escherichia coli; a eukaryotic cell, from a protist cell, an animal cell, a plant cell, a fungal cell, a yeast cell, an insect cell, an Sf9 cell, a mammalian cell, an avian cell, a CHO cell, or a COS cell.

12. A method of producing a binding protein, comprising culturing a host cell according to claim 11 in culture medium under conditions sufficient to produce the binding protein.

13. A protein produced by the method according to claim 12.

14. A pharmaceutical composition comprising the binding protein according to any one of claims 1 to 7, and a pharmaceutically acceptable carrier, wherein said pharmaceutical composition optionally further comprises at least one additional therapeutic agent, wherein the additional therapeutic agent is optionally selected from an imaging agent, a cytotoxic agent, an angiogenesis inhibitor, a kinase inhibitor, a co-stimulation molecule blocker, an adhesion molecule blocker, an anti-cytokine antibody or functional fragment thereof, methotrexate, cyclosporin, rapamycin, FK506, a detectable label or reporter, a TNF antagonist, an antirheumatic, a muscle relaxant, a narcotic, a non-steroid anti-inflammatory drug (NSAID), an analgesic, an anesthetic, a sedative, a local anesthetic, a neuromuscular blocker, an antimicrobial, an antipsoriatic, a corticosteriod, an anabolic steroid, an erythropoietin, an immunization, an immunoglobulin, an immunosuppressive, a growth hormone, a hormone replacement drug, a radiopharmaceutical, an antidepressant, an antipsychotic, a stimulant, an asthma medication, a beta agonist, an inhaled steroid, an epinephrine or analog, a cytokine, or a cytokine antagonist.

15. A binding protein according to any one of claims 1 to 7, a binding protein conjugate according to claim 8 or a composition according to claim 14 for use in therapy,
optionally wherein said therapy comprises treatment of rheumatoid arthritis, osteoarthritis, juvenile chronic arthritis, septic arthritis, Lyme arthritis, psoriatic arthritis, reactive arthritis, spondyloarthropathy, systemic lupus erythematosus, Crohn's disease, ulcerative colitis, inflammatory bowel disease, insulin dependent diabetes mellitus, thyroiditis, asthma, allergic diseases, psoriasis, dermatitis scleroderma, graft versus host disease, organ transplant rejection, acute or chronic immune disease associated with organ transplantation, sarcoidosis, atherosclerosis, disseminated intravascular coagulation, Kawasaki's disease, Grave's disease, nephrotic syndrome, chronic fatigue syndrome, Wegener's granulomatosis, Henoch-Schoenlein purpurea, microscopic vasculitis of the kidneys, chronic active hepatitis, uveitis, septic shock, toxic shock syndrome, sepsis syndrome, cachexia, infectious diseases, parasitic diseases, acquired immunodeficiency syndrome, acute transverse myelitis, Huntington's chorea, Parkinson's disease, Alzheimer's disease, stroke, primary biliary cirrhosis, hemolytic anemia, malignancies, heart failure, Addison's disease, sporadic, polyglandular deficiency type I, polyglandular deficiency type II, Schmidt's syndrome, adult (acute) respiratory distress syndrome, alopecia, alopecia areata, arthopathy, arthropathy, Reiter's disease, psoriatic arthropathy, ulcerative colitic arthropathy, enteropathic synovitis, chlamydia, yersinia associated arthropathy, salmonella associated arthropathy, atheromatous disease, arteriosclerosis, atopic allergy, autoimmune bullous disease, pemphigus vulgaris, pemphigus foliaceus, pemphigoid, linear IgA disease, autoimmune haemolytic anaemia, Coombs positive haemolytic anaemia, acquired pernicious anaemia, juvenile pernicious anaemia, myalgic encephalitis/Royal Free Disease, chronic mucocutaneous candidiasis, giant cell arteritis, primary sclerosing hepatitis, cryptogenic autoimmune hepatitis, acquired immunodeficiency related diseases, hepatitis B, hepatitis C, common varied immunodeficiency (common variable hypogammaglobulinaemia), dilated cardiomyopathy, female infertility, ovarian failure, premature ovarian failure, fibrotic lung disease, cryptogenic fibrosing alveolitis, post-inflammatory interstitial lung disease, interstitial pneumonitis, connective tissue disease associated interstitial lung disease, mixed connective tissue disease associated lung disease, systemic sclerosis associated interstitial lung disease, rheumatoid arthritis associated interstitial lung disease, systemic lupus erythematosus associated lung disease, dermatomyositis associated lung disease, polymyositis associated lung disease, Sjögren's disease associated lung disease, ankylosing spondylitis associated lung disease, vasculitic diffuse lung disease, haemosiderosis associated lung disease, drug-induced interstitial lung disease, fibrosis, radiation fibrosis, bronchiolitis obliterans, chronic eosinophilic pneumonia, lymphocytic infiltrative lung disease, postinfectious interstitial lung disease, gouty arthritis, autoimmune hepatitis, type-1 autoimmune hepatitis (classical autoimmune or lupoid hepatitis), type-2 autoimmune hepatitis (anti-LKM antibody hepatitis), autoimmune mediated hypoglycaemia, type B insulin resistance with acanthosis nigricans, hypoparathyroidism, acute immune disease associated with organ transplantation, chronic immune disease associated with organ transplantation, osteoarthrosis, primary sclerosing cholangitis, psoriasis type 1, psoriasis type 2, idiopathic leucopaenia, autoimmune neutropaenia, renal disease NOS, glomerulonephritides, microscopic vasulitis of the kidneys, lyme disease, discoid lupus erythematosus, male infertility idiopathic, male infertility NOS, sperm autoimmunity, multiple sclerosis (all subtypes), sympathetic ophthalmia, pulmonary hypertension secondary to connective tissue disease, Goodpasture's syndrome, pulmonary manifestation of polyarteritis nodosa, acute rheumatic fever, rheumatoid spondylitis, Still's disease, systemic sclerosis, Sjörgren's syndrome, Takayasu's disease/arteritis, autoimmune thrombocytopaenia, idiopathic thrombocytopaenia, autoimmune thyroid disease, hyperthyroidism, goitrous autoimmune hypothyroidism (Hashimoto's disease), atrophic autoimmune hypothyroidism, primary myxoedema, phacogenic uveitis, primary vasculitis, vitiligo acute liver disease, chronic liver diseases, alcoholic cirrhosis, alcohol-induced liver injury, choleosatatis, idiosyncratic liver disease, drug-induced hepatitis, non-alcoholic steatohepatitis, allergy and asthma, group B streptococci (GBS) infection, mental disorders, depression, schizophrenia, Th2 Type and Th1 Type mediated diseases, acute and chronic pain, different forms of pain, cancers, lung cancer, breast cancer, stomach cancer, bladder cancer, colon cancer, pancreatic cancer , ovarian cancer, prostate cancer, rectal cancer, hematopoietic malignancies, leukemia, lymphoma, Abetalipoprotemia, acrocyanosis, acute and chronic parasitic or infectious processes, acute leukemia, acute lymphoblastic leukemia (ALL), acute myeloid leukemia (AML), acute bacterial infection, chronic bacterial infection, acute pancreatitis, acute renal failure, adenocarcinomas, aerial ectopic beats, AIDS dementia complex, alcohol-induced hepatitis, allergic conjunctivitis, allergic contact dermatitis, allergic rhinitis, allograft rejection, alpha-1-antitrypsin deficiency, amyotrophic lateral sclerosis, anemia, angina pectoris, anterior horn cell degeneration, anti cd3 therapy, antiphospholipid syndrome, anti-receptor hypersensitivity reactions, aortic aneuryism, peripheralaneuryism, aortic dissection, arterial hypertension, arteriosclerosis, arteriovenous fistula, ataxia, sustained atrial fibrillation, paroxysmal atrial fibrillation, atrial flutter, atrioventricular block, B cell lymphoma, bone graft rejection, bone marrow transplant (BMT) rejection, bundle branch block, Burkitt's lymphoma, burns, cardiac arrhythmias, cardiac stun syndrome, cardiac tumors, cardiomyopathy, cardiopulmonary bypass inflammation response, cartilage transplant rejection, cerebellar cortical degenerations, cerebellar disorders, chaotic or multifocal atrial tachycardia, chemotherapy associated disorders, chronicmyelocytic leukemia (CML), chronic alcoholism, chronic inflammatory pathologies, chronic lymphocytic leukemia (CLL), chronic obstructive pulmonary disease (COPD), chronic salicylate intoxication, colorectal carcinoma, congestive heart failure, conjunctivitis, contact dermatitis, cor pulmonale, coronary artery disease, Creutzfeldt-Jakob disease, culture negative sepsis, cystic fibrosis, cytokine therapy associated disorders, dementia pugilistica, demyelinating diseases, dengue hemorrhagic fever, dermatitis, dermatologic conditions, diabetes, diabetes mellitus, diabetic ateriosclerotic disease, diffuse Lewy body disease, dilated congestive cardiomyopathy, disorders of the basal ganglia, Down's syndrome in middle age, drug-induced movement disorders induced by drugs which block CNS dopamine receptors, drug sensitivity, eczema, encephalomyelitis, endocarditis, endocrinopathy, epiglottitis, epstein-barr virus infection, erythromelalgia, extrapyramidal and cerebellar disorders, familial hematophagocytic lymphohistiocytosis, fetal thymus implant rejection, Friedreich's ataxia, functional peripheral arterial disorders, fungal sepsis, gas gangrene, gastric ulcer, glomerular nephritis, graft rejection of any organ, graft rejection of any tissue, gram negative sepsis, gram positive sepsis, granulomas due to intracellular organisms, hairy cell leukemia, Hallervorden-Spatz disease, Hashimoto'sthyroiditis, hay fever, heart transplant rejection, hemachromatosis, hemodialysis, hemolytic uremic syndrome/thrombolytic thrombocytopenic purpura, hemorrhage, hepatitis A, His bundle arrythmias, HIV infection/HIV neuropathy, Hodgkin's disease, hyperkinetic movement disorders, hypersensitity reactions, hypersensitivity pneumonitis, hypertension, hypokinetic movement disorders, hypothalamic-pituitary-adrenal axis evaluation, idiopathic Addison's disease, idiopathic pulmonary fibrosis, antibody mediated cytotoxicity, Asthenia, infantile spinal muscular atrophy, inflammation of the aorta, influenza A, ionizing radiation exposure, iridocyclitis, uveitis, optic neuritis, ischemia- reperfusion injury, ischemic stroke, juvenile rheumatoid arthritis, juvenile spinal muscular atrophy, Kaposi's sarcoma, kidney transplant rejection, legionella, leishmaniasis, leprosy, lesions of the corticospinal system, lipedema, liver transplant rejection, lymphederma, malaria, malignamt lymphoma, malignant histiocytosis, malignant melanoma, meningitis, meningococcemia, metabolic/idiopathic, migraine headache, mitochondrial multi.system disorder, mixed connective tissue disease, monoclonal gammopathy, multiple myeloma, multiple systems degeneration, Mencel degeneration, Dejerine-Thomas degeneration, Shi-Drager degeneration, Machado-Joseph degeneration, myasthenia gravis, mycobacterium avium intracellulare, mycobacterium tuberculosis, myelodyplastic syndrome, myocardial infarction, myocardial ischemic disorders, nasopharyngeal carcinoma, neonatal chronic lung disease, nephritis, nephrosis, neurodegenerative diseases, neurogenic muscular atrophies, neutropenic fever, non-hodgkins lymphoma, occlusion of the abdominal aorta and its branches, occulsive arterial disorders, okt3 therapy, orchitis/epidydimitis, orchitis/vasectomy reversal procedures, organomegaly, osteoporosis, pancreas transplant rejection, pancreatic carcinoma, paraneoplastic syndrome/hypercalcemia of malignancy, parathyroid transplant rejection, pelvic inflammatory disease, perennial rhinitis, pericardial disease, peripheral atherlosclerotic disease, peripheral vascular disorders, peritonitis, pernicious anemia, pneumocystis carinii pneumonia, pneumonia, POEMS syndrome (polyneuropathy, organomegaly, endocrinopathy, monoclonal gammopathy, and skin changes syndrome), post perfusion syndrome, post pump syndrome, post-MI cardiotomy syndrome, preeclampsia, progressive supranucleo palsy, primary pulmonary hypertension, radiation therapy, Raynaud's phenomenon and disease, Raynoud's disease, Refsum's disease, regular narrow QRS tachycardia, renovascular hypertension, reperfusion injury, restrictive cardiomyopathy, sarcomas, scleroderma, senile chorea, senile dementia of Lewy body type, seronegative arthropathies, shock, sickle cell anemia, skin allograft rejection, skin changes syndrome, small bowel transplant rejection, solid tumors, specific arrythmias, spinal ataxia, spinocerebellar degenerations, streptococcal myositis, structural lesions of the cerebellum, subacute sclerosing panencephalitis, syncope, syphilis of the cardiovascular system, systemic anaphalaxis, systemic inflammatory response syndrome, systemic onset juvenile rheumatoid arthritis, T-cell or FAB ALL, telangiectasia, thromboangitis obliterans, thrombocytopenia, toxicity, transplants, trauma, hemorrhage, type III hypersensitivity reactions, type IV hypersensitivity, unstable angina, uremia, urosepsis, urticaria, valvular heart diseases, varicose veins, vasculitis, venous diseases, venous thrombosis, ventricular fibrillation, viral infections, fungal infections, vital encephalitis, aseptic meningitis, vital-associated hemaphagocytic syndrome, Wernicke-Korsakoff syndrome, Wilson's disease, xenograft rejection of any organ, xenograft rejection of any tissue, acute coronary syndromes, acute idiopathic polyneuritis, acute inflammatory demyelinating polyradiculoneuropathy, acute ischemia, adult Still's disease, anaphylaxis, anti-phospholipid antibody syndrome, aplastic anemia, atopic eczema, atopic dermatitis, autoimmune dermatitis, autoimmune disorder associated with streptococcus infection, autoimmune enteropathy, autoimmune hearing loss, autoimmune lymphoproliferative syndrome (ALPS), autoimmune myocarditis, autoimmune premature ovarian failure, blepharitis, bronchiectasis, bullous pemphigoid, cardiovascular disease, catastrophic antiphospholipid syndrome, celiac disease, cervical spondylosis, chronic ischemia, cicatricial pemphigoid, clinically isolated syndrome (cis) with risk for multiple sclerosis, childhood onset psychiatric disorder, dacryocystitis, dermatomyositis, diabetic retinopathy, disk herniation, disk prolapse, drug induced immune hemolytic anemia, endometriosis, endophthalmitis, episcleritis, erythema multiforme, erythema multiforme major, gestational pemphigoid, Guillain-Barré syndrome (GBS), Hughes syndrome, idiopathic Parkinson's disease, idiopathic interstitial pneumonia, IgE-mediated allergy, immune hemolytic anemia, inclusion body myositis, infectious ocular inflammatory disease, inflammatory demyelinating disease, inflammatory heart disease, inflammatory kidney disease, IPF/UIP, iritis, keratitis, keratojuntivitis sicca, Kussmaul disease or Kussmaul-Meier disease, Landry's paralysis, Langerhan's cell histiocytosis, livedo reticularis, macular degeneration, microscopic polyangiitis, morbus bechterev, motor neuron disorders, mucous membrane pemphigoid, multiple organ failure, myelodysplastic syndrome, myocarditis, nerve root disorders, neuropathy, non-A non-B hepatitis, optic neuritis, osteolysis, pauciarticular JRA, peripheral artery occlusive disease (PAOD), peripheral vascular disease (PVD), peripheral artery, disease (PAD), phlebitis, polyarteritis nodosa, periarteritis nodosa, polychondritis, polymyalgia rheumatica, poliosis, polyarticular JRA, polyendocrine deficiency syndrome, polymyositis, polymyalgia rheumatica (PMR), primary Parkinsonism, prostatitis, pure red cell aplasia, primary adrenal insufficiency, recurrent neuromyelitis optica, restenosis, rheumatic heart disease, sapho (synovitis, acne, pustulosis, hyperostosis, and osteitis), secondary amyloidosis, shock lung, scleritis, sciatica, secondary adrenal insufficiency, silicone associated connective tissue disease, sneddon-wilkinson dermatosis, spondilitis ankylosans, Stevens-Johnson syndrome (SJS), temporal arteritis, toxoplasmic retinitis, toxic epidermal necrolysis, transverse myelitis, TRAPS (tumor necrosis factor receptor, type 1 allergic reaction, type II diabetes, usual interstitial pneumonia (UIP), vasculitis, vernal conjunctivitis, viral retinitis, Vogt-Koyanagi-Harada syndrome (VKH syndrome), wet macular degeneration, or wound healing.

16. The binding protein, conjugate or composition for use according to claim 15, wherein the binding protein, conjugate or composition is administered by parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, bolus, vaginal, rectal, buccal, sublingual, intranasal, or transdermal administration.

17. A method of determining the presence, amount or concentration of at least one target or fragment thereof in a test sample by an immunoassay,
wherein the immunoassay comprises contacting the test sample with at least one binding protein and at least one detectable label,
wherein the at least one binding protein comprises a binding protein according to any one of claims 1 to 7,
optionally wherein said method further comprises:
(a)
(i) contacting the test sample with the at least one binding protein, wherein the binding protein binds to an epitope on the target or fragment thereof so as to form a first complex,
(ii) contacting the complex with the at least one detectable label, wherein the detectable label binds to the binding protein or an epitope on the target or fragment thereof that is not bound by the binding protein to form a second complex, and
(iii) detecting the presence, amount or concentration of the target or fragment thereof in the test sample based on the signal generated by the detectable label in the second complex, wherein the presence, amount or concentration of the target or fragment thereof is directly correlated with the signal generated by the detectable label;
(b)
(i) contacting the test sample with the at least one binding protein, wherein the binding protein binds to an epitope on the target or fragment thereof so as to form a first complex,
(ii) contacting the complex with the at least one detectable label, wherein the detectable label competes with the target or fragment thereof for binding to the binding protein so as to form a second complex, and (iii) detecting the presence, amount or concentration of the target or fragment thereof in the test sample based on the signal generated by the detectable label in the second complex, wherein the presence, amount or concentration of the target or fragment thereof is indirectly correlated with the signal generated by the detectable label.

18. The method according to claim 17, wherein the test sample is from a patient and the method
(i) further comprises diagnosing, prognosticating, or assessing the efficiency of therapeutic/prophylactic treatment of the patient,
(ii) further comprises modifying the therapeutic/prophylactic treatment of the patient as needed to improve efficacy;
(iii) is adapted for use in an automated system or a semi-automated system; and/or
(iv) determines the presence, amount, or concentration of more than one target in the sample.

19. A kit for assaying a test sample for the presence, amount, or concentration of a target or fragment thereof, the kit comprising
(i) instructions for assaying the test sample for the target or fragment thereof; and
(ii) at least one binding protein comprising the binding protein according to any one of claims 1-7.
